# EUROPEAN PATENT APPLICATION

(11) **EP 4 843 575 A1**
(43) Date of publication of application: **25.11.2026**
(21) Application number: 25741619.8
(22) Date of filing: 17.01.2025
(51) Int. Cl.: A61K 31/513, A61K 31/517, A61K 31/519, A61P 35/00

(54) **PHARMACEUTICAL COMPOSITION COMPRISING BRAF INHIBITOR AND USE OF PHARMACEUTICAL COMPOSITION IN MEDICINE**

(30) Priority: 17.01.2024 CN 202410068349
(71) Applicant: Haisco Pharmaceutical Group Co., Ltd., Shannan, Xizang 856099 (CN)
(72) Inventor: JIANG, Wei, Chengdu, Sichuan 611130 (CN); WANG, Li, Chengdu, Sichuan 611130 (CN)
(74) Representative: AWA Denmark A/S
(86) International application number: PCT/CN2025/072990
(87) International publication number: WO 2025/153057

(57) **Abstract**

The present invention relates to a pharmaceutical composition or a pharmaceutical preparation. The pharmaceutical composition or the pharmaceutical preparation comprises a therapeutically effective amount of an active ingredient M and a pharmaceutically acceptable excipient, wherein the active ingredient M is selected from a compound as shown in general formula I or a stereoisomer, tautomer, deuterated product, solvate, prodrug, metabolite, pharmaceutically acceptable salt or eutectic crystal thereof; and the pharmaceutical composition or the pharmaceutical preparation comprises 1-1000 mg of the active ingredient M. The present invention also relates to a use of the pharmaceutical composition or the pharmaceutical preparation in preparation of a related drug for treating cancers.

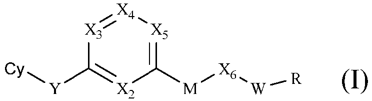

## Description

### Technical Field

The present invention belongs to the field of pharmaceutical preparations, and in particular relates to a pharmaceutical composition or a pharmaceutical preparation, wherein the pharmaceutical composition or the pharmaceutical preparation comprises a therapeutically effective amount of an active ingredient M and a pharmaceutically acceptable excipient; the active ingredient M is selected from a compound as shown in general formula (I) or a stereoisomer, tautomer, deuterated product, solvate, prodrug, metabolite, pharmaceutically acceptable salt or eutectic crystal thereof; and the pharmaceutical composition or the pharmaceutical preparation comprises 1-1000 mg of the active ingredient M. The present invention also relates to a use of the pharmaceutical composition or the pharmaceutical preparation in preparation of a related drug for treating cancers.

### Background Art

Kinases are enzymes that catalyse the transfer of phosphate groups from high-energy, phosphate-donating molecules to specific substrates. This process is called phosphorylation, in which the substrate gains a phosphate group and the high-energy ATP molecule donates a phosphate group. Kinases are divided into the following broad categories based on the substrates on which they act: protein kinases, lipid kinases, and carbohydrate kinases. Kinases are found in a variety of species, from bacteria to molds, to worms, and to mammals. More than five hundred different kinases have been identified in humans.

MAP kinases (MAPKs) are a family of serine/threonine kinases that respond to a variety of extracellular growth signals. For example, growth hormone, epidermal growth factor, platelet-derived growth factor, and insulin are all thought to participate in mitogenic stimulation of the MAPK pathway. Activation of this pathway at the receptor level initiates a signalling cascade whereby Ras GTPase exchanges GDP for GTP. Next, Ras activates Raf kinase (also known as MAPKKK), which activates MEK (MAPKK).

BRAF protein is a member of the RAF family of serine/threonine kinases that participates in cascades of the Ras Raf MEK extracellular signal-regulated kinase (ERK) pathway or the mitogen-activated protein kinase (MAPK)/ERK signalling pathway that affect cell division and differentiation. Mutations in the BRAF gene can lead to uncontrolled growth and subsequent tumour formation. BRAF is mutated and/or overactivated in common human cancers, such as melanoma, colorectal cancer, thyroid cancer, non-small cell lung cancer, and ovarian cancer and their metastatic cancers, and primary brain tumours. Although some BRAF inhibitors produce excellent extracranial responses, cancers may still develop brain metastases during or subsequent to BRAF inhibitor therapy. An estimated 20% of subjects with cancer will develop brain metastases, with the majority of brain metastases occurring in those with melanoma, colorectal cancer, lung cancer, and renal cell carcinoma. Brain metastases remain a substantial contributor to overall cancer mortality in subjects with advanced cancers, and despite multimodality treatments and advances in systemic therapies, including combinations of surgery, radiotherapy, chemotherapy, immunotherapy, and/or targeted therapies, the prognosis remains poor.

Additionally, BRAF has been identified as a potential target for the treatment of primary brain tumours. The prevalence of BRAF V600E mutations in primary brain tumours has been reported. Schindler et al analysed 1,320 central nervous system (CNS) tumours and Behling et al analysed 969 CNS tumours in paediatric and adult populations. These studies combined with others have reported the presence of BRAF V600E mutations in various cancers, including papillary craniopharyngioma, pleomorphic xanthoastrocytoma (PXA), ganglioglioma, astroblastoma, etc.

The blood-brain barrier (BBB) is a highly selective physical transport and metabolic barrier that separates the CNS from the blood. The BBB prevents certain drugs from entering brain tissue and is a limiting factor in the delivery of many peripherally administered agents to the CNS. Many drugs commonly used to treat cancer cannot cross the blood-brain barrier. This means that these drugs cannot penetrate the brain and therefore cannot effectively kill cancer cells in the brain. Current treatments for subjects with brain tumours include surgical resection, radiotherapy, and/or chemotherapy with agents such as temozolomide and/or bevacizumab. However, surgical treatment of brain cancer is not always possible; for example, the tumour may not be accessible or the subject may not be able to withstand the trauma of neurosurgery. Additionally, radiotherapy and treatments with cytotoxic agents are known to have undesirable side effects. For example, there is growing evidence that the use of temozolomide itself can induce mutations and worsen prognosis in a significant proportion of subjects, and the bevacizumab label has a boxed warning for gastrointestinal perforation, surgical and wound healing complications, and bleeding. Kinase inhibitors are used to treat many peripheral cancers. However, many kinase inhibitors such as BRAF inhibitors (e.g., vemurafenib and dabrafenib), due to their structural properties, are substrates of active transporters such as P-glycoprotein (P gp) or breast cancer resistance protein (BCRP). For example, dabrafenib was reported to have an MDR1 efflux ratio of 11.4, a BCRP efflux ratio of 21.0, and a total brain-to-plasma ratio of 0.023; Vemurafenib was reported to have an MDR1 efflux ratio of 83, a BCRP efflux ratio of 495, and a total brain-to-plasma ratio of 0.004.

Given that both P gp and BCRP are expressed in the endothelial cells lining blood-brain capillaries, the activity of both P gp and BCRP in the BBB plays a critical role in preventing the distribution of most kinase inhibitors in the brain parenchyma. Therefore, kinase inhibitors are generally not suitable for the treatment of tumours or cancers in the brain (which is protected by the BBB). Therefore, treatments against tumours harboring BRAF mutations are still needed. Additionally, there remains an unmet need for the treatment of CNS tumours, including CNS tumours harboring BRAF mutations.

### Summary of the Invention

An object of the present invention is to provide a pharmaceutical composition or a pharmaceutical preparation, wherein the pharmaceutical composition or the pharmaceutical preparation comprises a therapeutically effective amount of an active ingredient M and a pharmaceutically acceptable excipient, the active ingredient M is selected from a compound as shown in general formula (I) or a stereoisomer, tautomer, deuterated product, solvate, prodrug, metabolite, pharmaceutically acceptable salt or eutectic crystal thereof, and the formulation specification of the pharmaceutical composition or the pharmaceutical preparation is 1-1000 mg. The present invention also relates to a use of the pharmaceutical composition or the pharmaceutical preparation in preparation of a related drug for treating cancers.

The pharmaceutical composition or the pharmaceutical preparation of the present invention has advantages of good oral properties, good efficacy, low toxic side effects, good safety, high selectivity, good pharmacokinetics, high bioavailability, and no inhibition of CYP enzyme.

The present invention relates to a pharmaceutical composition or a pharmaceutical preparation, comprising a therapeutically effective amount of an active ingredient M and a pharmaceutically acceptable excipient. The pharmaceutical composition can be in a unit formulation form.

The present invention relates to a pharmaceutical composition or a pharmaceutical preparation, wherein the pharmaceutical composition or the pharmaceutical preparation comprises an active ingredient M and a pharmaceutically acceptable excipient, and the active ingredient M is selected from a compound as shown in general formula I or II, or a stereoisomer, tautomer, deuterated product, solvate, prodrug, metabolite, pharmaceutically acceptable salt or eutectic crystal thereof, 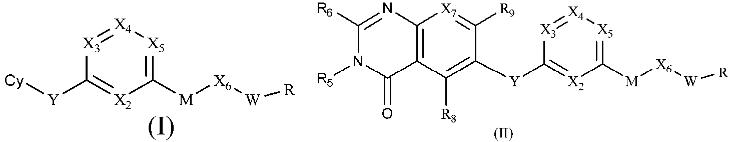 wherein, Cy is selected from P1, P2, P3, P4, P5, P6, P7 or P8; 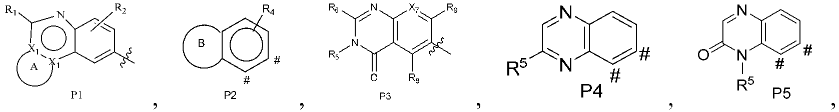 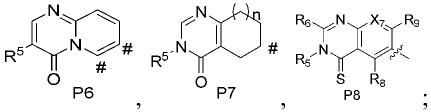
ring A is 5-membered or 6-membered heteroaryl containing 1-3 heteroatoms selected from N, S, and O, and the heteroaryl is optionally substituted with 1 or 2 groups selected from halogen, C₁₋₄ alkoxy, -NHC₁₋₄ alkyl, -N(C₁₋₄ alkyl)₂, CN, C₁₋₄ alkyl, haloC₁₋₄ alkoxy and haloC₁₋₄ alkyl; in some embodiments, the heteroaryl is 5-membered heteroaryl; in some embodiments, the heteroaryl is selected from pyrazolyl, oxazolyl, imidazolyl, triazole, thiazolyl, isoxazolyl, isothiazolyl, pyrrolyl, pyridyl, pyrimidinyl, etc., and the heteroaryl is optionally substituted with 1 or 2 groups selected from halogen, C₁₋₄ alkoxy, -NHC₁₋₄ alkyl, -N(C₁₋₄ alkyl)₂, CN, C₁₋₄ alkyl, haloC₁₋₄ alkoxy and haloC₁₋₄ alkyl; ring B is 5-membered heterocycle containing 1 to 3 heteroatoms selected from N, S, and O, and the heterocycle is optionally substituted with 1 to 3 groups selected from =O, halogen, C₁₋₄ alkyl, haloC₁₋₄ alkyl, C₁₋₄ alkoxy, OH and haloC₁₋₄ alkoxy;
# means that one point is selected to connect to Y;
n is selected from 0 or 1;
each X₁ is independently N or C; in some embodiments, each X₁ is independently C;
X₂ is N or CR₃; in some embodiments, X₂ is N; in some embodiments, X₂ is CR₃;
X₃ is N or CR₃₁; in some embodiments, X₃ is N or CH;
X₄ is N or CR₃₂; ; in some embodiments, X₄ is N or CH;
X₅ is N or CR₃₃; in some embodiments, X₅ is N or CH;
X₆ is C(O), S(O) or S(O)₂; in some embodiments, X₆ is C(O) or S(O)₂; in some embodiments, X₆ is S(O)₂;
X₇ is CR₇ or N; in some embodiments, X₇ is N; in some embodiments, X₇ is CH;
R₁, R₂ and R₄ are independently H, halogen, OH, -NHC₁₋₄ alkyl, -N(C₁₋₄ alkyl)₂, CN, C₁₋₄ alkyl, C₁₋₄ alkoxy, haloC₁₋₄ alkoxy, haloC₁₋₄ alkyl, C₂₋₄ alkenyl or C₂₋₄ alkynyl; the -NHC₁₋₄ alkyl includes -NH methyl, -NH ethyl, -NH isopropyl, - NH propyl, -NH butyl, and the -N(C₁₋₄ alkyl)₂ includes dimethylamino, diethylamino, etc;
R₃, R₃₁, R₃₂ and R₃₃ are independently H, halogen, C₁₋₄ alkyl, haloC₁₋₄ alkyl, C₂₋₄ alkenyl, C₂₋₄ alkynyl, C₁₋₄ alkoxy, haloC₁₋₄ alkoxy, -NHC₁₋₄ alkyl, -N(C₁₋₄ alkyl)₂, CN or C₃₋₆ cycloalkyl; in some embodiments, R₃, R₃₁, R₃₂ and R₃₃ are independently H, halogen, C₁₋₄ alkyl, haloC₁₋₄ alkyl, C₂₋₄ alkenyl, C₂₋₄ alkynyl, C₁₋₄ alkoxy, haloC₁₋₄ alkoxy, -NHC₁₋₄ alkyl, -N(C₁₋₄ alkyl)₂ or CN; in some embodiments, R₃ is C₁₋₄ alkyl, haloC₁₋₄ alkyl, C₁₋₄ alkoxy, haloC₁₋₄ alkoxy, C₂₋₄ alkenyl, C₂₋₄ alkynyl, -NHC₁₋₄ alkyl, -N(C₁₋₄ alkyl)₂, or CN; in some embodiments, R₃ is C₁₋₄ alkyl, haloC₁₋₄ alkyl, C₁₋₄ alkoxy, haloC₁₋₄ alkoxy, OH, NH₂, -NHC₁₋₄ alkyl, or -N(C₁₋₄alkyl)₂; in some embodiments, R₃ is H, C₁₋₄ alkyl, haloC₁₋₄ alkyl, C₁₋₄ alkoxy, haloC₁₋₄ alkoxy, C₂₋₄ alkenyl, C₂₋₄ alkynyl, -NHC₁₋₄ alkyl, -N(C₁₋₄ alkyl)₂ or CN;
alternatively, R₃₁ and R₃₂, or R₃₂ and R₃₃, together with the atoms to which they are attached form C₃₋₆ carbocycle or 5-membered or 6-membered heterocycle containing 1-3 heteroatoms selected from N, S, and O, wherein the carbocycle or the heterocycle is optionally substituted with 1 to 3 groups selected from halogen, C₁₋₄ alkyl, haloC₁₋₄ alkyl, C₁₋₄ alkoxy, OH, NH₂ and CN; in some embodiments, the carbocycle or the heterocycle is optionally substituted with 1 to 3 groups selected from halogen, C₁₋₄ alkyl, haloC₁₋₄ alkyl, C₁₋₄ alkoxy, C₁₋₄ haloalkoxy and CN. In some embodiments, R₃₁ and R₃₂, or R₃₂ and R₃₃, together with the atoms to which they are attached form C₃₋₆ cycloalkyl, such as cyclopropenyl, cyclobutenyl, or cyclopentenyl, or form 5-membered or 6-membered heterocycloalkyl containing 1-3 heteroatoms selected from N, S, and O, such as azethenyl, oxethenyl, azacyclopentenyl, oxacyclopentenyl, azacyclohexenyl, or oxacyclohexenyl, wherein the cycloalkyl and the heterocycloalkyl are optionally substituted with 1 to 3 groups selected from halogen, C₁₋₄ alkyl, haloC₁₋₄ alkyl, C₁₋₄ alkoxy, C₁₋₄ haloalkoxy and CN.

R₅ is C₁₋₄ alkyl, haloC₁₋₄ alkyl, C₃₋₆ cycloalkyl, 4-membered to 7-membered heterocycloalkyl containing 1-3 heteroatoms selected from N, S, and O, C₁₋₄ alkoxy, haloC₁₋₄ alkoxy or C₃₋₆ cycloalkyloxy, and the cycloalkyl includes but is not limited to cyclopropyl, cyclobutyl, cyclopentyl and cyclohexyl, the heterocycloalkyl includes but is not limited to aziridinyl, azetidinyl, tetrahydropyrrolyl, piperidinyl, piperazinyl, oxacyclopropanyl, oxetanyl, tetrahydrofuranyl, tetrahydropyranyl, morpholinyl, etc., and is optionally substituted with 1-3 groups selected from halogen, -NHC₁₋₄ alkyl, -N(C₁₋₄ alkyl)₂, C₁₋₄ alkoxy, haloC₁₋₄ alkoxy, haloC₁₋₄ alkyl, CN, C₁₋₄ alkyl and =O. In some embodiments, R₅ is C₁₋₄ alkyl, haloC₁₋₄ alkyl, C₃₋₆ cycloalkyl, 4-membered to 7-membered heterocycloalkyl containing 1-3 heteroatoms selected from N, S, and O, C₁₋₄ alkoxy, haloC₁₋₄ alkoxy or C₃₋₆ cycloalkyloxy, and is optionally substituted with 1-3 groups selected from halogen, -NHC₁₋₄ alkyl, -N(C₁₋₄ alkyl)₂, C₁₋₄ alkoxy, haloC₁₋₄ alkoxy, haloC₁₋₄ alkyl, CN and C₁₋₄ alkyl. In some embodiments, R₅ is C₁₋₄ alkyl, haloC₁₋₄ alkyl, C₃₋₆ cycloalkyl, 4-membered to 7-membered heterocycloalkyl containing 1-3 heteroatoms selected from N, S, and O, C₁₋₄ alkoxy, haloC₁₋₄ alkoxy or C₃₋₆ cycloalkyloxy, and is optionally substituted with 1 to 3 groups selected from halogen, OH, NH₂, -NHC₁₋₄ alkyl, -N(C₁₋₄ alkyl)₂, CN and C₁₋₄ alkyl; in some embodiments, R₅ is optionally substituted with 1 to 3 groups selected from halogen, -NHC₁₋₄ alkyl, -N(C₁₋₄ alkyl)₂, C₁₋₄ alkoxy, haloC₁₋₄ alkoxy, haloC₁₋₄ alkyl, CN and C₁₋₄ alkyl. In some embodiments, R₅ is substituted or unsubstituted C₃₋₆ cycloalkyl, substituted or unsubstituted 4-membered to 7-membered heterocycloalkyl containing 1-3 heteroatoms selected from N, S and O, substituted or unsubstituted C₃₋₆ cycloalkyloxy, substituted or unsubstituted C₁₋₄ alkoxy or substituted or unsubstituted haloC₁₋₄ alkoxy, the substituent is as described above. In some embodiments, R₅ is C₁₋₂ alkyl, CN substituted C₁₋₂ alkyl, haloC₁₋₂ alkyl, C₃₋₄ cycloalkyl, 4-membered to 7-membered heterocycloalkyl containing 1-3 heteroatoms selected from N, S, and O, C₁₋₂ alkoxy, haloC₁₋₂ alkoxy or C₃₋₄ cycloalkyloxy. In some embodiments, R₅ is selected from C₁₋₂ alkyl, CN substituted C₁₋₂ alkyl, haloC₁₋₂ alkyl or C₁₋₂ alkoxy. In some embodiments, R₅ is selected from -CH₂-CN, -CH₂CH₂F, -CH₂CHF₂, -CH₂CF₃, -CH₂F, -CHF₂, or -CF₃.

R₆ is H, halogen, C₁₋₄ alkyl or haloC₁₋₄ alkyl; In some embodiments, R₆ is H.

Alternatively, R₅ and R₆ together with the atoms to which they are attached form 5-membered or 6-membered heterocycle containing 1 to 3 heteroatoms selected from N, S, and O, and the heterocycle is optionally substituted with 1 to 3 groups selected from halogen, OH, NH₂, -NHC₁₋₄ alkyl, -N(C₁₋₄ alkyl)₂, CN and C₁₋₄ alkyl; the heterocycle includes heteroaryl and heterocycloalkyl; in some embodiments, the ring is 5-membered heterocycle; in some embodiments, the ring is 5-membered heterocycloalkyl, and is optionally substituted with 1 to 3 groups selected from halogen, OH, NH₂, -NHC₁₋₄ alkyl, -N(C₁₋₄ alkyl)₂, CN and C₁₋₄ alkyl.

R₇, R₈, and R₉ are independently H, halogen, -NHC₁₋₄ alkyl, -N(C₁₋₄ alkyl)₂, CN, C₁₋₄ alkyl, C₂₋₄ alkenyl, C₂₋₄ alkynyl, haloC₁₋₄ alkyl, C₁₋₄ alkoxy or haloC₁₋₄ alkoxy. In some embodiments, R₇, R₈, and R₉ are independently H, halogen, - NHC₁₋₄ alkyl, -N(C₁₋₄ alkyl)₂, CN, C₁₋₄ alkyl, C₂₋₄ alkenyl, C₂₋₄ alkynyl, haloC₁₋₄ alkyl, C₁₋₄ alkoxy or haloC₁₋₄ alkoxy. In some embodiments, R₇ is H or halogen. In some embodiments, R₈ is halogen, OH, NH₂, -NHC₁₋₄ alkyl, -N(C₁₋₄ alkyl)₂, CN, C₁₋₄ alkoxy or haloC₁₋₄ alkoxy. In some embodiments, R₈ is halogen, -NHC₁₋₄ alkyl, -N(C₁₋₄ alkyl)₂, CN, C₁₋₄ alkoxy or haloC₁₋₄ alkoxy. In some embodiments, R₉ is H or halogen;
Y is C₁₋₂ alkylene, O or NR_{y}. In some embodiments, Y is O. In some embodiments, Y is NH. In some embodiments, Y is methylene or ethylene;
R_{y} is H or C₁₋₄ alkyl. In some embodiments, R_{y} is H. In some embodiments, R_{y} is methyl, ethyl, propyl, isopropyl, etc.;
M is C₁₋₂ alkylene, O or NRₘ; In some embodiments, M is NH. In some embodiments, M is methylimino, ethylimino, propylimino, or isopropylimino;
W is a bond, O or NR_{w}. In some embodiments, W is a bond, O, NH or NC₁₋₄ alkyl;
Rₘ and R_{w} are independently H or C₁₋₄ alkyl. In some embodiments, Rₘ and R_{w} are independently H, methyl, ethyl, propyl or isopropyl;
R is selected from C₁₋₄ alkyl, C₃₋₈ cycloalkyl, 4-membered to 10-membered heterocycle containing 1 to 3 heteroatoms selected from N, S, and O, or -O-C₃₋₆ cycloalkyl, and the alkyl, cycloalkyl or heterocycle is optionally substituted with 1 to 3 groups selected from halogen, C₁₋₄ alkyl, C₁₋₄ alkoxy, haloC₁₋₄ alkoxy, OH, NH₂, -NHC₁₋₄ alkyl, -N(C₁₋₄ alkyl)₂ and haloC₁₋₄ alkyl. In some embodiments, R is selected from C₁₋₄ alkyl, C₃₋₈ cycloalkyl or 4-membered to 10-membered heterocycle containing 1 to 3 heteroatoms selected from N, S, and O, and the alkyl, cycloalkyl or heterocycle is optionally substituted with 1 to 3 groups selected from halogen, C₁₋₄ alkyl, C₁₋₄ alkoxy, haloC₁₋₄ alkoxy, OH, NH₂, -NHC₁₋₄ alkyl, -N(C₁₋₄ alkyl)₂ and haloC₁₋₄ alkyl. In some embodiments, R is selected from C₃₋₈ cycloalkyl or 4-membered to 10-membered heterocycle containing 1 to 3 heteroatoms selected from N, S, and O, and is optionally substituted with 1 to 3 groups selected from halogen, C₁₋₄ alkyl, C₁₋₄ alkoxy, haloC₁₋₄ alkoxy, OH, NH₂, -NHC₁₋₄ alkyl, - N(C₁₋₄ alkyl)₂ and haloC₁₋₄ alkyl. In some embodiments, R is C₃₋₄ cycloalkyl or 4-membered monocyclic heterocycloalkyl containing 1 to 3 heteroatoms selected from N, S, and O, 6-membered to 8-membered monocyclic heterocycloalkyl, 5-membered or 6-membered monocyclic heteroaryl, 5-membered to 10-membered fused heterocycloalkyl, 5-membered to 10-membered bridged heterocycloalkyl or 6-membered to 10-membered spirocyclic heterocycloalkyl, and is optionally substituted with 1 to 3 groups selected from halogen, C₁₋₄ alkyl, C₁₋₄ alkoxy, OH, NH₂, -NHC₁₋₄ alkyl, -N(C₁₋₄ alkyl)₂ and haloC₁₋₄ alkyl. In some embodiments, R is selected from C₁₋₄ alkyl, C₃₋₈ cycloalkyl or 4-membered to 10-membered heterocycloalkyl containing 1 to 3 heteroatoms selected from N, S, and O, and the alkyl, cycloalkyl or heterocycloalkyl is optionally substituted with 1 to 3 groups selected from halogen, C₁₋₄ alkyl, C₁₋₄ alkoxy, haloC₁₋₄ alkoxy, OH, NH₂, -NHC₁₋₄ alkyl, -N(C₁₋₄ alkyl)₂ and haloC₁₋₄ alkyl. In some embodiments, R is selected from C₁₋₄ alkyl, C₃₋₈ cycloalkyl or 4-membered to 7-membered monocyclic heterocycloalkyl containing 1 to 3 heteroatoms selected from N, S, and O, 5-membered to 10-membered fused heterocycloalkyl, 5-membered to 10-membered bridged heterocycloalkyl or 6-membered to 10-membered spirocyclic heterocycloalkyl, and is optionally substituted with 1 to 3 groups selected from halogen, C₁₋₄ alkyl, C₁₋₄ alkoxy, haloC₁₋₄ alkoxy, OH, NH₂, -NHC₁₋₄ alkyl, -N(C₁₋₄ alkyl)₂ and haloC₁₋₄ alkyl. In some embodiments, R is saturated 4-membered to 7-membered monocyclic heterocycloalkyl containing 1 to 3 heteroatoms selected from N, S, and O, saturated 5-membered to 10-membered fused heterocycloalkyl, saturated 5-membered to 10-membered bridged heterocycloalkyl or saturated 6-membered to 10-membered spirocyclic heterocycloalkyl, and is optionally substituted with 1 to 3 groups selected from halogen, C₁₋₄ alkyl, C₁₋₄ alkoxy, haloC₁₋₄ alkoxy, OH, NH₂, -NHC₁₋₄ alkyl, -N(C₁₋₄ alkyl)₂ and haloC₁₋₄ alkyl. In some embodiments, R is selected from C₃₋₈ cycloalkyl, 4-membered to 7-membered monocyclic heterocycloalkyl containing 1 to 3 heteroatoms selected from N, S, and O, 5-membered to 10-membered fused heterocycloalkyl, 5-membered to 10-membered bridged heterocycloalkyl or 6-membered to 10-membered spirocyclic heterocycloalkyl, and the cycloalkyl is selected from cyclopropyl, cyclobutyl, cyclopentyl, cyclohexyl, cyclopentenyl, cyclohexenyl, etc., the monocyclic heterocycloalkyl is selected from aziridinyl, azetidinyl, tetrahydropyrrolyl, piperidinyl, piperazinyl, oxacyclopropanyl, oxetanyl, tetrahydrofuranyl, tetrahydropyranyl, morpholinyl, etc., the fused heterocycloalkyl is selected from 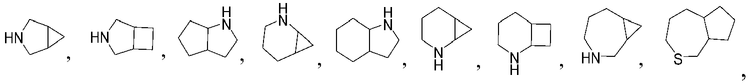 etc., the bridged heterocycloalkyl is selected from 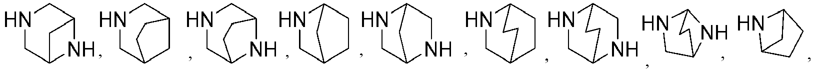 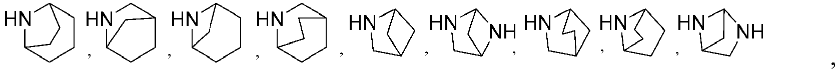 etc., and the spirocyclic heterocycloalkyl is selected from: 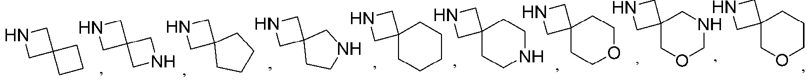 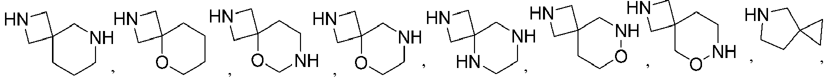 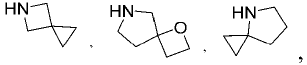 etc.; the above-mentioned groups are optionally substituted with 1 to 3 groups selected from halogen, C₁₋₄ alkyl, C₁₋₄ alkoxy, haloC₁₋₄ alkoxy, OH, NH₂, -NHC₁₋₄ alkyl, -N(C₁₋₄ alkyl)₂ and haloC₁₋₄ alkyl;
when Cy is P2, R is 4-membered to 10-membered saturated heterocycloalkyl containing 1 to 3 heteroatoms selected from N, S, and O, and is optionally substituted with 1 to 3 groups selected from halogen, C₁₋₄ alkyl, C₁₋₄ alkoxy, haloC₁₋₄ alkoxy, OH, NH₂, -NHC₁₋₄ alkyl, -N(C₁₋₄ alkyl)₂ and haloC₁₋₄ alkyl.

The pharmaceutical composition or the pharmaceutical preparation comprises 1-1000 mg of the active ingredient M and the excipient includes one or more of a filler, a binder, a glidant, a lubricant, and a disintegrant;
in some embodiments, the pharmaceutical composition or the pharmaceutical preparation of the present invention comprises 5-800 mg of the active ingredient M;
in some embodiments, the pharmaceutical composition or the pharmaceutical preparation of the present invention comprises 5-600 mg of the active ingredient M;
in some embodiments, the pharmaceutical composition or the pharmaceutical preparation of the present invention comprises 5-400 mg of the active ingredient M;
in some embodiments, the pharmaceutical composition or the pharmaceutical preparation of the present invention comprises 20-800 mg of the active ingredient M;
in some embodiments, the pharmaceutical composition or the pharmaceutical preparation of the present invention comprises 20-600 mg of the active ingredient M;
in some embodiments, the pharmaceutical composition or the pharmaceutical preparation of the present invention comprises 20-400 mg of the active ingredient M;
in some embodiments, the pharmaceutical composition or the pharmaceutical preparation of the present invention comprises 5 mg of the active ingredient M;
in some embodiments, the pharmaceutical composition or the pharmaceutical preparation of the present invention comprises 10 mg of the active ingredient M;
in some embodiments, the pharmaceutical composition or the pharmaceutical preparation of the present invention comprises 20 mg of the active ingredient M;
in some embodiments, the pharmaceutical composition or the pharmaceutical preparation of the present invention comprises 50 mg of the active ingredient M;
in some embodiments, the pharmaceutical composition or the pharmaceutical preparation of the present invention comprises 60 mg of the active ingredient M;
in some embodiments, the pharmaceutical composition or the pharmaceutical preparation of the present invention comprises 70 mg of the active ingredient M;
in some embodiments, the pharmaceutical composition or the pharmaceutical preparation of the present invention comprises 80 mg of the active ingredient M;
in some embodiments, the pharmaceutical composition or the pharmaceutical preparation of the present invention comprises 90 mg of the active ingredient M;
in some embodiments, the pharmaceutical composition or the pharmaceutical preparation of the present invention comprises 100 mg of the active ingredient M;
in some embodiments, the pharmaceutical composition or the pharmaceutical preparation of the present invention comprises 150 mg of the active ingredient M;
in some embodiments, the pharmaceutical composition or the pharmaceutical preparation of the present invention comprises 200 mg of the active ingredient M;
in some embodiments, the pharmaceutical composition or the pharmaceutical preparation of the present invention comprises 400 mg of the active ingredient M;
in some embodiments, the pharmaceutical composition or the pharmaceutical preparation of the present invention comprises 600 mg of the active ingredient M;
in some embodiments, the pharmaceutical composition or the pharmaceutical preparation of the present invention comprises 800 mg of the active ingredient M;
in some embodiments, the pharmaceutical composition or the pharmaceutical preparation of the present invention comprises 1000 mg of the active ingredient M.

In some embodiments, R₆ and R₉ are H; Y is O, M is NH, X₂ is CR₃, X₃ is CR₃, X₄ is CR₃₂, X₅ is CH, X₆ is SO₂, X₇ is CH;
R₃ is C₁₋₂ alkyl, haloC₁₋₂ alkyl, C₁₋₂ alkoxy, haloC₁₋₂ alkoxy, C₂₋₄ alkenyl, C₂₋₄ alkynyl, -NHC₁₋₂ alkyl, -N(C₁₋₂ alkyl)₂ or CN;
each of R₃₁ and R₃₂ is independently H, F, Cl, C₁₋₂ alkyl, haloC₁₋₂ alkyl, C₂₋₄ alkenyl, C₂₋₄ alkynyl, C₁₋₂ alkoxy, haloC₁₋₂ alkoxy, -NHC₁₋₂ alkyl, -N(C₁₋₂ alkyl)₂, CN or C₃₋₆ cycloalkyl;
R₅ is C₁₋₂ alkyl, CN or =O substituted C₁₋₂ alkyl, haloC₁₋₂ alkyl, C₃₋₄ cycloalkyl, 4-membered to 7-membered heterocycloalkyl containing 1-3 heteroatoms selected from N, S, and O, C₁₋₂ alkoxy, haloC₁₋₂ alkoxy or C₃₋₄ cycloalkyloxy;
R₈ is selected from H, F, Cl, CN, C₁₋₂ alkyl, C₂₋₄ alkenyl, C₂₋₄ alkynyl, haloC₁₋₂ alkyl, C₁₋₂ alkoxy or haloC₁₋₂ alkoxy;
R is 4-membered or 5-membered monocyclic heterocycloalkyl containing 1 to 3 heteroatoms selected from N, S, and O, 6-membered to 8-membered monocyclic heterocycloalkyl, 5-membered or 6-membered monocyclic heteroaryl, 5-membered to 10-membered fused heterocycloalkyl, 5-membered to 10-membered bridged heterocycloalkyl, 6-membered to 10-membered spirocyclic heterocycloalkyl or -O-C₃₋₆ cycloalkyl, and the 4-membered monocyclic heterocycloalkyl is substituted with 1 to 3 groups selected from F, Cl, C₁₋₂ alkyl, C₁₋₂ alkoxy, OH, NH₂, -NHC₁₋₂ alkyl, -N(C₁₋₂ alkyl)₂ and haloC₁₋₂ alkyl, the 6-membered to 8-membered monocyclic heterocycloalkyl, the 5-membered or 6-membered monocyclic heteroaryl, the 5-membered to 10-membered fused heterocycloalkyl, the 5-membered to 10-membered bridged heterocycloalkyl, the 6-membered to 10-membered spirocyclic heterocycloalkyl or the C₃₋₆ cycloalkyl is optionally substituted with 1 to 3 groups selected from F, Cl, C₁₋₂ alkyl, C₁₋₂ alkoxy, OH, NH₂, -NHC₁₋₂ alkyl, -N(C₁₋₂ alkyl)₂ and haloC₁₋₂ alkyl;
in some embodiments, R₃ is CN;
each of R₃₁ and R₃₂ is independently H, F, Cl or C₃₋₆ cycloalkyl;
R₅ is selected from C₁₋₂ alkyl, haloC₁₋₂ alkyl, C₁₋₂ alkoxy, or CN or =O substituted C₁₋₂ alkyl;
R₈ is H;
R is 6-membered to 10-membered spirocyclic heterocycloalkyl containing 1 to 3 heteroatoms selected from N, S, and O, 4-membered or 5-membered monocyclic heterocycloalkyl containing 1 to 3 heteroatoms selected from N, S, and O, or -O-C₃₋₆ cycloalkyl, and the 6-membered to 10-membered spirocyclic heterocycloalkyl, the 4-membered or 5-membered monocyclic heterocycloalkyl or the C₃₋₆ cycloalkyl is optionally substituted with 1 to 3 groups selected from F, Cl, C₁₋₂ alkyl or C₁₋₂ alkoxy.

In some embodiments, R₃ is CN;
each of R₃₁ and R₃₂ is independently H, F, Cl, cyclopropane, cyclobutane, cyclopentane or cyclohexane;
R₅ is selected from -CH₃, -CH₂CH₃, -CH₂F, -CHF₂, -CF₃, -CH₂CH₂F, - CH₂CHF₂, -CH₂CF₃, -CHFCH₂F, -CHFCHF₂, -CHFCF₃, -CF₂CH₂F, -CF₂CHF₂, - CF₂CF₃, -CH₂Cl, -CHCl₂, -CCl₃, -CH₂CH₂Cl, -CH₂CHCl₂, -CH₂CCl₃, - CHClCH₂Cl, -CHClCHCl₂, -CHClCCl₃, -CCl₂CH₂Cl, -CCl₂CHCl₂, -CCl₂CCl₃, - OCH₃, -OCH₂CH₃, -CH₂CN, -CH₂CH₂CN, -CH(CN)CH₃, -COCH₃, or -COCH₂CN;
R₈ is H;
R is 6-membered to 10-membered spirocyclic heterocycloalkyl containing 1 to 3 heteroatoms selected from N, S, and O, 4-membered or 5-membered monocyclic heterocycloalkyl containing 1 to 3 heteroatoms selected from N, S, and O, or -O-C₃₋₆ cycloalkyl, and the 6-membered to 10-membered spirocyclic heterocycloalkyl, 4-membered or 5-membered monocyclic heterocycloalkyl or C₃₋₆ cycloalkyl is optionally substituted with 1 to 3 groups selected from F, Cl, C₁₋₂ alkyl or C₁₋₂ alkoxy.

A specific technical solution of the present invention is the compound, and the stereoisomer, the deuterated product or the pharmaceutically acceptable salt thereof described in the present invention, the compound is selected from one of the structures in Table 1,

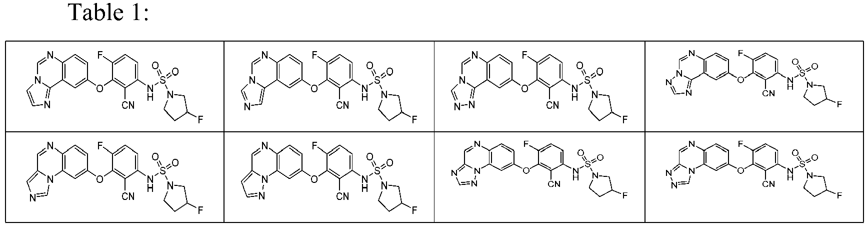

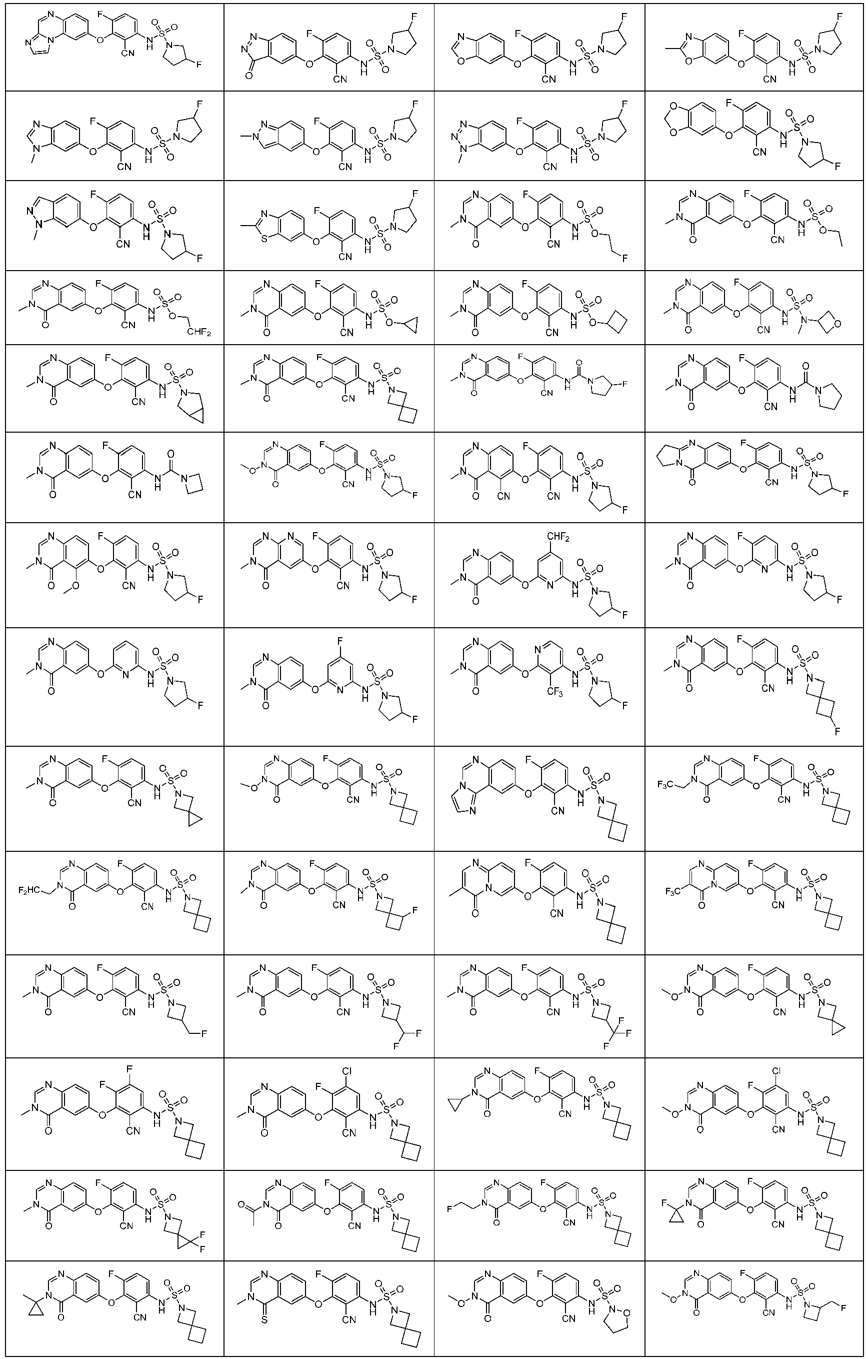

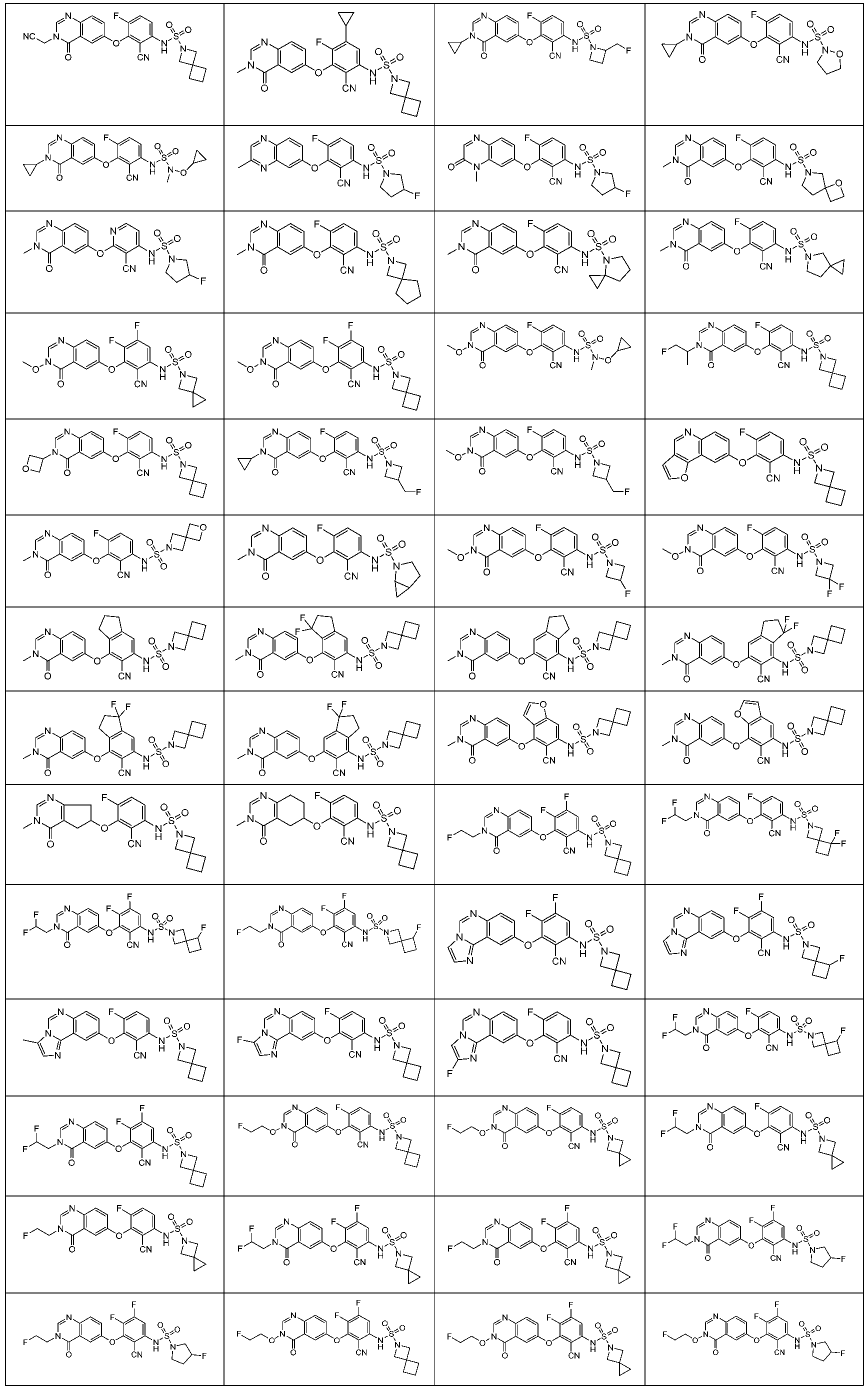

A specific technical solution of the present invention is the compound, and the stereoisomer, the deuterated product or the pharmaceutically acceptable salt thereof described in the present invention, the compound is selected from one of the structures in Table 2,

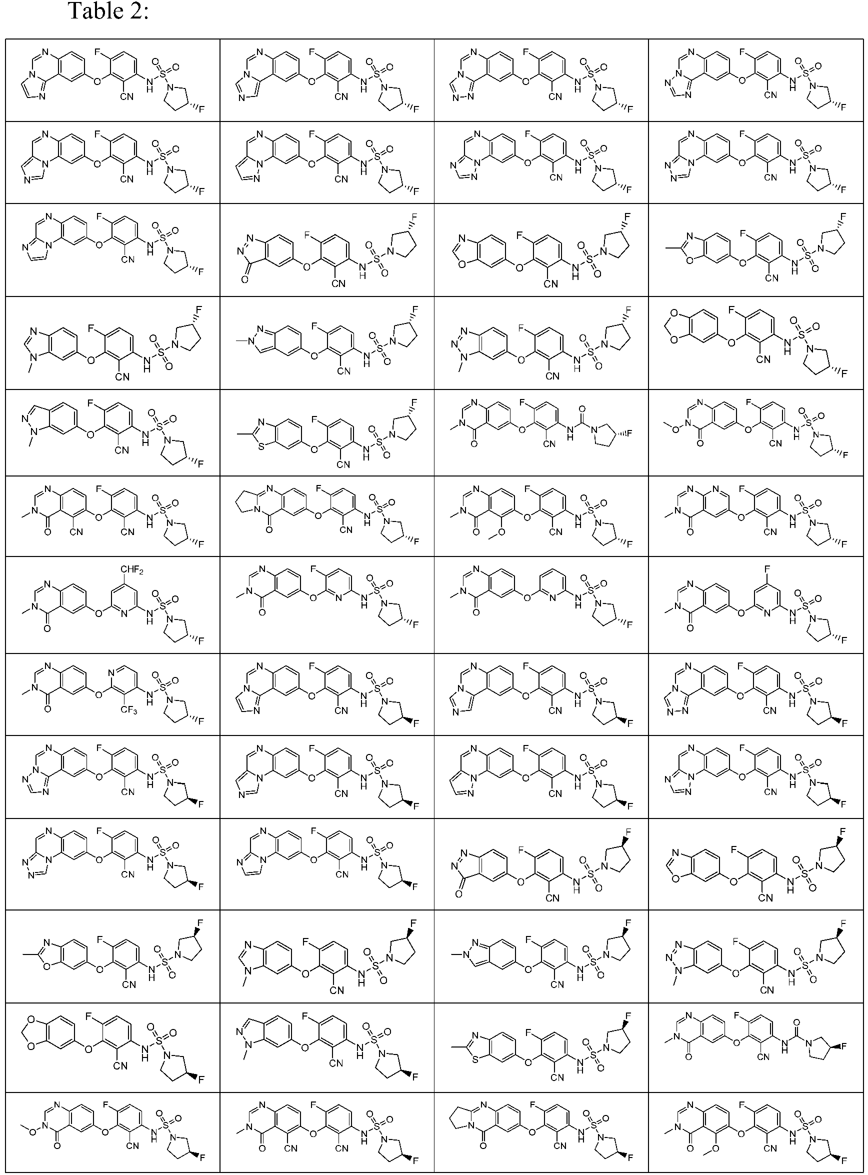

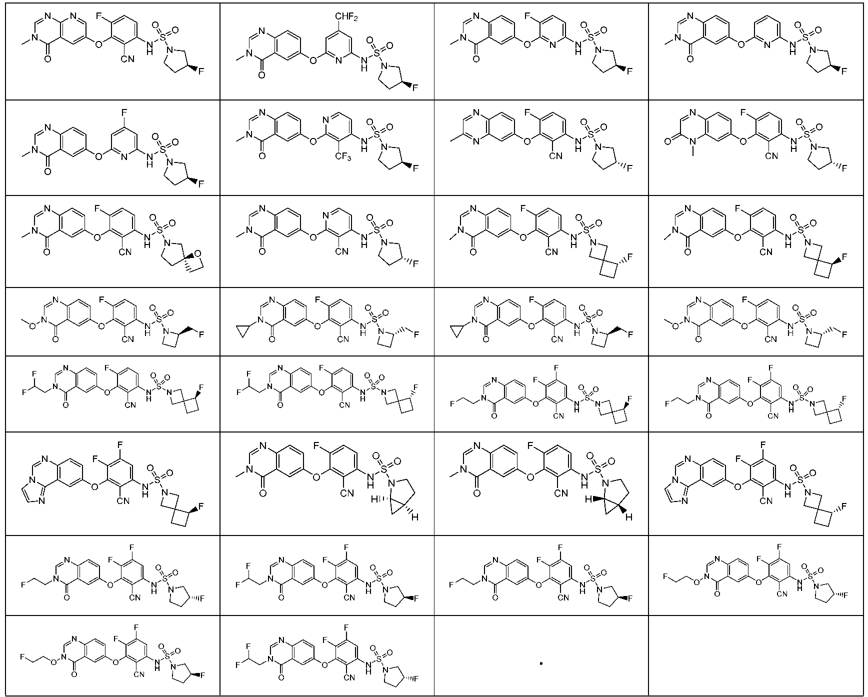

In some embodiments, the active ingredient M is selected from the following structure:

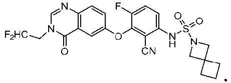

The pharmaceutical composition or the pharmaceutical preparation of the present invention, comprises the active ingredient M and the pharmaceutically acceptable excipient of any one of the preceding embodiments, wherein the active ingredient M is present with a content of 0.5%-99%; in some embodiments, the active ingredient M is present with a content of 1%-90%; in some embodiments, the active ingredient M is present with a content of 1%-80%; in some embodiments, the active ingredient M is present with a content of 1%-70%; in some embodiments, the active ingredient M is present with a content of 1%-60%; in some embodiments, the active ingredient M is present with a content of 1%-50%; in some embodiments, the active ingredient M is present with a content of 1%-40%; in some embodiments, the active ingredient M is present with a content of 1%-30%; in some embodiments, the active ingredient M is present with a content of 1%-20%; in some embodiments, the active ingredient M is present with a content of 1%-10%; in some embodiments, the active ingredient M is present with a content of 5%-90%; in some embodiments, the active ingredient M is present with a content of 5%-80%; in some embodiments, the active ingredient M is present with a content of 5%-70%; in some embodiments, the active ingredient M is present with a content of 5%-60%; in some embodiments, the active ingredient M is present with a content of 5%-45%; in some embodiments, the active ingredient M is present with a content of 5%-40%; in some embodiments, the active ingredient M is present with a content of 5%-30%; in some embodiments, the active ingredient M is present with a content of 5%-20%; in some embodiments, the active ingredient M is present with a content of 5%-10%; in some embodiments, the active ingredient M is present with a content of 10%-90%; in some embodiments, the active ingredient M is present with a content of 10%-80%; in some embodiments, the active ingredient M is present with a content of 10%-70%; in some embodiments, the active ingredient M is present with a content of 10%-60%; in some embodiments, the active ingredient M is present with a content of 10%-50%; in some embodiments, the active ingredient M is present with a content of 10%-45%; in some embodiments, the active ingredient M is present with a content of 10%-40%; in some embodiments, the active ingredient M is present with a content of 10%-30%; in some embodiments, the active ingredient M is present with a content of 10%-25%; in some embodiments, the active ingredient M is present with a content of 25%; in some embodiments, the active ingredient M is present with a content of 10%.

The pharmaceutical composition or the pharmaceutical preparation of the present invention, comprises the active ingredient M and the pharmaceutically acceptable excipient of any one of the preceding embodiments, wherein the pharmaceutically acceptable excipient comprises one or more of a filler, a binder, a glidant, a lubricant, and a disintegrant.

The pharmaceutical composition or the pharmaceutical preparation of the present invention, comprises the active ingredient M and the pharmaceutically acceptable excipient of any one of the preceding embodiments, wherein the pharmaceutically acceptable excipient comprises a filler, a binder, a glidant, a lubricant, and a disintegrant.

The pharmaceutical composition or the pharmaceutical preparation of the present invention, comprises the active ingredient M and the pharmaceutically acceptable excipient of any one of the preceding embodiments, wherein the pharmaceutically acceptable excipient comprises a filler, a binder, a glidant, a lubricant, and a disintegrant, and further contains one or more of a solubilizer, a pH regulator, and a surfactant.

The pharmaceutical composition or the pharmaceutical preparation of the present invention, comprises the active ingredient M and the pharmaceutically acceptable excipient of any one of the preceding embodiments, wherein the pharmaceutically acceptable excipient comprises a filler, a binder, a glidant, a lubricant, a disintegrant, and a solubilizer.

The pharmaceutical composition or the pharmaceutical preparation of the present invention, comprises the active ingredient M and the pharmaceutically acceptable excipient of any one of the preceding embodiments, wherein the pharmaceutically acceptable excipient comprises a filler, a binder, a glidant, a lubricant, a disintegrant, and a pH regulator.

The pharmaceutical composition or the pharmaceutical preparation of the present invention, comprises the active ingredient M and the pharmaceutically acceptable excipient of any one of the preceding embodiments, wherein the pharmaceutically acceptable excipient comprises a filler, a binder, a glidant, a lubricant, a disintegrant, and a surfactant.

The present invention relates to a pharmaceutical composition and a pharmaceutical preparation, wherein the filler is selected from one or more of microcrystalline cellulose, lactose, mannitol, pregelatineized starch, silicified microcrystalline cellulose, sucrose, sorbitol, dextran, calcium dihydrogen phosphate, or starch; in some embodiments, the filler is selected from one or more of microcrystalline cellulose, silicified microcrystalline cellulose, or sorbitol; in some embodiments, the filler is microcrystalline cellulose.

The present invention relates to a pharmaceutical composition and a pharmaceutical preparation, wherein the binder is selected from one or more of povidone, hydroxypropyl cellulose, hypromellose, methylcellulose, sodium carboxymethyl cellulose, or sodium carboxymethyl cellulose; in some embodiments, the binder is selected from one or more of hypromellose, methylcellulose, or sodium carboxymethyl cellulose; in some embodiments, the binder is hypromellose.

The present invention relates to a pharmaceutical composition and a pharmaceutical preparation, wherein the glidant is selected from one or more of talc, silicon dioxide, micronized silica gel, polyethylene glycol, or magnesium lauryl sulphate; in some embodiments, the glidant is selected from one or more of silicon dioxide or micronized silica gel; in some embodiments, the glidant is colloidal silicon dioxide.

The present invention relates to a pharmaceutical composition and a pharmaceutical preparation, wherein the lubricant is selected from one or more of magnesium stearate, calcium stearate, stearic acid, or sodium stearyl fumarate; in some embodiments, the lubricant is sodium stearyl fumarate.

The present invention relates to a pharmaceutical composition and a pharmaceutical preparation, wherein the disintegrant is selected from one or more of sodium carboxymethyl starch, low-substituted hydroxypropyl cellulose, crospovidone, croscarmellose sodium, or calcium carboxymethylcellulose. in some embodiments, the disintegrant is selected from one or more of crospovidone, croscarmellose sodium, or calcium carboxymethylcellulose; in some embodiments, the disintegrant is crospovidone.

The present invention relates to a pharmaceutical composition and a pharmaceutical preparation, wherein the solubilizer is selected from one or more of oxyethylene castor oil, polyoxyethylene hydrogenated castor oil, polysorbate, polyethylene glycol 15-hydroxystearate, poloxamer, cyclodextrin, hydroxypropyl betacyclodextrin, docusate sodium, or vitamin E polyethylene glycol succinate; in some embodiments, the solubilizer is selected from one or more of cyclodextrin or hydroxypropyl betacyclodextrin; in some embodiments, the solubilizer is cyclodextrin; in some embodiments, the solubilizer is hydroxypropyl betacyclodextrin.

The present invention relates to a pharmaceutical composition and a pharmaceutical preparation, wherein the surfactant is sodium dodecyl sulphate.

The present invention relates to a pharmaceutical composition and a pharmaceutical preparation, wherein the pH regulator is magnesium oxide.

The pharmaceutical composition or the pharmaceutical preparation of the present invention, comprises the active ingredient M and the pharmaceutically acceptable excipient of any one of the preceding embodiments, wherein the pharmaceutically acceptable excipient comprises a filler, a binder, a glidant, a lubricant, and a disintegrant, and further optionally contains one or more of a solubilizer, a pH regulator, and a surfactant, and the active ingredient M is present with a content of 0.5%-99%; in some embodiments, the active ingredient M is present with a content of 1%-90%; in some embodiments, the active ingredient M is present with a content of 1%-80%; in some embodiments, the active ingredient M is present with a content of 1%-70%; in some embodiments, the active ingredient M is present with a content of 1%-60%; in some embodiments, the active ingredient M is present with a content of 1%-50%; in some embodiments, the active ingredient M is present with a content of 1%-40%; in some embodiments, the active ingredient M is present with a content of 1%-30%; in some embodiments, the active ingredient M is present with a content of 1%-20%; in some embodiments, the active ingredient M is present with a content of 1%-10%; in some embodiments, the active ingredient M is present with a content of 5%-90%; in some embodiments, the active ingredient M is present with a content of 5%-80%; in some embodiments, the active ingredient M is present with a content of 5%-70%; in some embodiments, the active ingredient M is present with a content of 5%-60%; in some embodiments, the active ingredient M is present with a content of 5%-45%; in some embodiments, the active ingredient M is present with a content of 5%-40%; in some embodiments, the active ingredient M is present with a content of 5%-30%; in some embodiments, the active ingredient M is present with a content of 5%-20%; in some embodiments, the active ingredient M is present with a content of 5%-10%; in some embodiments, the active ingredient M is present with a content of 10%-90%; in some embodiments, the active ingredient M is present with a content of 10%-80%; in some embodiments, the active ingredient M is present with a content of 10%-70%; in some embodiments, the active ingredient M is present with a content of 10%-60%; in some embodiments, the active ingredient M is present with a content of 10%-50%; in some embodiments, the active ingredient M is present with a content of 10%-45%; in some embodiments, the active ingredient M is present with a content of 10%-40%; in some embodiments, the active ingredient M is present with a content of 10%-30%; in some embodiments, the active ingredient M is present with a content of 10%-25%; in some embodiments, the active ingredient M is present with a content of 25%; in some embodiments, the active ingredient M is present with a content of 10%.

The pharmaceutical composition or the pharmaceutical preparation of the present invention, comprises the active ingredient M and the pharmaceutically acceptable excipient of any one of the preceding embodiments, wherein the pharmaceutically acceptable excipient comprises a filler, a binder, a glidant, a lubricant, and a disintegrant, and further optionally contains one or more of a solubilizer, a pH regulator, and a surfactant, and the filler is present with a content of 10%-90%; in some embodiments, the filler is present with a content of 10%-80%; in some embodiments, the filler is present with a content of 10%-75%; in some embodiments, the filler is present with a content of 10%-70%; in some embodiments, the filler is present with a content of 10%-60%; in some embodiments, the filler is present with a content of 10%-50%; in some embodiments, the filler is present with a content of 10%-40%; in some embodiments, the filler is present with a content of 10%-30%; in some embodiments, the filler is present with a content of 10%-20%; in some embodiments, the filler is present with a content of 20%-80%; in some embodiments, the filler is present with a content of 20%-75%; in some embodiments, the filler is present with a content of 20%-70%; in some embodiments, the filler is present with a content of 20%-60%; in some embodiments, the filler is present with a content of 20%-50%; in some embodiments, the filler is present with a content of 30%-80%; in some embodiments, the filler is present with a content of 30%-75%; in some embodiments, the filler is present with a content of 30%-70%; in some embodiments, the filler is present with a content of 40%-80%; in some embodiments, the filler is present with a content of 40%-75%; in some embodiments, the filler is present with a content of 40%-70%; in some embodiments, the filler is present with a content of 37%; in some embodiments, the filler is present with a content of 40%; in some embodiments, the filler is present with a content of 72.5%; in some embodiments, the filler is present with a content of 80%.

The pharmaceutical composition or the pharmaceutical preparation of the present invention, comprises the active ingredient M and the pharmaceutically acceptable excipient of any one of the preceding embodiments, wherein the pharmaceutically acceptable excipient comprises a filler, a binder, a glidant, a lubricant, and a disintegrant, and further optionally contains one or more of a solubilizer, a pH regulator, and a surfactant, and the binder is present with a content of 1%-50%; in some embodiments, the binder is present with a content of 1%-40%; in some embodiments, the binder is present with a content of 1%-30%; in some embodiments, the binder is present with a content of 1%-10%; in some embodiments, in some embodiments, the binder is present with a content of 1%-6%; in some embodiments, the binder is present with a content of 1%-5%; in some embodiments, the binder is present with a content of 1%-4%; in some embodiments, the binder is present with a content of 1%-3%; in some embodiments, the binder is present with a content of 1%-2.5%; in some embodiments, the binder is present with a content of 1.5%-2.5%; in some embodiments, the binder is present with a content of 2%-2.5%; in some embodiments, the binder is present with a content of 2%; in some embodiments, the binder is present with a content of 2.5%; in some embodiments, the binder is present with a content of 3%.

The pharmaceutical composition or the pharmaceutical preparation of the present invention, comprises the active ingredient M and the pharmaceutically acceptable excipient of any one of the preceding embodiments, wherein the pharmaceutically acceptable excipient comprises a filler, a binder, a glidant, a lubricant, and a disintegrant, and further optionally contains one or more of a solubilizer, a pH regulator, and a surfactant, and the glidant is present with a content of 0.5%-10%; in some embodiments, the glidant is present with a content of 0.5%-8%; in some embodiments, the glidant is present with a content of 0.5%-6%; in some embodiments, the glidant is present with a content of 0.5%-5%; in some embodiments, the glidant is present with a content of 0.5%-4%; in some embodiments, the glidant is present with a content of 0.5%-3%; in some embodiments, the glidant is present with a content of 1%-10%; in some embodiments, the glidant is present with a content of 1%-8%; in some embodiments, the glidant is present with a content of 1%-6%; in some embodiments, the glidant is present with a content of 1%-5%; in some embodiments, the glidant is present with a content of 1%-4%; in some embodiments, the glidant is present with a content of 1%-3%; in some embodiments, the glidant is present with a content of 1%-2%; in some embodiments, the glidant is present with a content of 1%-2.5%; in some embodiments, the glidant is present with a content of 1%-2%; in some embodiments, the glidant is present with a content of 1%; in some embodiments, the glidant is present with a content of 2%; in some embodiments, the glidant is present with a content of 3%.

The pharmaceutical composition or the pharmaceutical preparation of the present invention, comprises the active ingredient M and the pharmaceutically acceptable excipient of any one of the preceding embodiments, wherein the pharmaceutically acceptable excipient comprises a filler, a binder, a glidant, a lubricant, and a disintegrant, and further optionally contains one or more of a solubilizer, a pH regulator, and a surfactant, and the lubricant is present with a content of 0.1%-10%; in some embodiments, the lubricant is present with a content of 0.1%-8%; in some embodiments, the lubricant is present with a content of 0.1%-6%; in some embodiments, the lubricant is present with a content of 0.1%-5%; in some embodiments, the lubricant is present with a content of 0.5%-8%; in some embodiments, the lubricant is present with a content of 0.5%-6%; in some embodiments, the lubricant is present with a content of 0.5%-5%; in some embodiments, the lubricant is present with a content of 0.5%-4%; in some embodiments, the lubricant is present with a content of 0.5%-3%; in some embodiments, the lubricant is present with a content of 0.5%-2.0%; in some embodiments, the lubricant is present with a content of 0.5%-1%; in some embodiments, the lubricant is present with a content of 1%-10%; in some embodiments, the lubricant is present with a content of 1%-8%; in some embodiments, the lubricant is present with a content of 1%-6%; in some embodiments, the lubricant is present with a content of 1%-5%; in some embodiments, the lubricant is present with a content of 1%-3%; in some embodiments, the lubricant is present with a content of 1%-2%; in some embodiments, the lubricant is present with a content of 0.5%; in some embodiments, the lubricant is present with a content of 0.6%; in some embodiments, the lubricant is present with a content of 0.7%; in some embodiments, the lubricant is present with a content of 0.8%; in some embodiments, the lubricant is present with a content of 0.9%; in some embodiments, the lubricant is present with a content of 1.0%; in some embodiments, the lubricant is present with a content of 2%.

The pharmaceutical composition or the pharmaceutical preparation of the present invention, comprises the active ingredient M and the pharmaceutically acceptable excipient of any one of the preceding embodiments, wherein the pharmaceutically acceptable excipient comprises a filler, a binder, a glidant, a lubricant, and a disintegrant, and further optionally contains one or more of a solubilizer, a pH regulator, and a surfactant, and the disintegrant is present with a content of 0.5%-10%; in some embodiments, the disintegrant is present with a content of 1%-10%; in some embodiments, the disintegrant is present with a content of 1%-9%; in some embodiments, the disintegrant is present with a content of 1%-8%; in some embodiments, the disintegrant is present with a content of 1%-7%; in some embodiments, the disintegrant is present with a content of 1%-6%; in some embodiments, the disintegrant is present with a content of 1%-5%; in some embodiments, the disintegrant is present with a content of 1%-4%; in some embodiments, the disintegrant is present with a content of 1%-3%; in some embodiments, the disintegrant is present with a content of 1%-2%; in some embodiments, the disintegrant is present with a content of 2%-10%; in some embodiments, the disintegrant is present with a content of 2%-8%; in some embodiments, the disintegrant is present with a content of 2%-7%; in some embodiments, the disintegrant is present with a content of 2%-6%; in some embodiments, the disintegrant is present with a content of 3%-7%; in some embodiments, the disintegrant is present with a content of 4%-7%; in some embodiments, the disintegrant is present with a content of 5%-7%; in some embodiments, the disintegrant is present with a content of 2%; in some embodiments, the disintegrant is present with a content of 3%; in some embodiments, the disintegrant is present with a content of 5%; in some embodiments, the disintegrant is present with a content of 6%; in some embodiments, the disintegrant is present with a content of 7%; in some embodiments, the disintegrant is present with a content of 10%.

The pharmaceutical composition or the pharmaceutical preparation of the present invention, comprises the active ingredient M and the pharmaceutically acceptable excipient of any one of the preceding embodiments, wherein the pharmaceutically acceptable excipient comprises a filler, a binder, a glidant, a lubricant, and a disintegrant, and further contains a solubilizer, and the solubilizer is present with a content of 5%-50%; in some embodiments, the solubilizer is present with a content of 10%-50%; in some embodiments, the solubilizer is present with a content of 10%-40%; in some embodiments, the solubilizer is present with a content of 30%-50%; in some embodiments, the solubilizer is present with a content of 30%-45%; in some embodiments, the solubilizer is present with a content of 10%; in some embodiments, the solubilizer is present with a content of 20%; in some embodiments, the solubilizer is present with a content of 30%; in some embodiments, the solubilizer is present with a content of 32%; in some embodiments, the solubilizer is present with a content of 40%; in some embodiments, the solubilizer is present with a content of 42.5%; in some embodiments, the solubilizer is present with a content of 45%.

The pharmaceutical composition or the pharmaceutical preparation of the present invention, comprises the active ingredient M and the pharmaceutically acceptable excipient of any one of the preceding embodiments, wherein the pharmaceutically acceptable excipient comprises a filler, a binder, a glidant, a lubricant, and a disintegrant, and further contains a surfactant, and the surfactant is present with a content of 1%-40%; in some embodiments, the surfactant is present with a content of 1%-30%; in some embodiments, the surfactant is present with a content of 1%-20%; in some embodiments, the surfactant is present with a content of 5%-20%; in some embodiments, the surfactant is present with a content of 10%-20%; in some embodiments, the surfactant is present with a content of 10%-15%; in some embodiments, the surfactant is present with a content of 5%; in some embodiments, the surfactant is present with a content of 10%; in some embodiments, the surfactant is present with a content of 15%; in some embodiments, the surfactant is present with a content of 20%.

The pharmaceutical composition or the pharmaceutical preparation of the present invention, comprises the active ingredient M and the pharmaceutically acceptable excipient of any one of the preceding embodiments, wherein the pharmaceutically acceptable excipient comprises a filler, a binder, a glidant, a lubricant, and a disintegrant, and further contains a pH regulator, and the pH regulator is present with a content of 1%-50%; in some embodiments, the pH regulator is present with a content of 1%-9%; in some embodiments, the pH regulator is present with a content of 1%-8%; in some embodiments, the pH regulator is present with a content of 1%-6%; in some embodiments, the pH regulator is present with a content of 1%-5%; in some embodiments, the pH regulator is present with a content of 1%; in some embodiments, the pH regulator is present with a content of 2%; in some embodiments, the pH regulator is present with a content of 2.5%; in some embodiments, the pH regulator is present with a content of 3%; in some embodiments, the pH regulator is present with a content of 4%; in some embodiments, the pH regulator is present with a content of 5%.

The present invention relates to a pharmaceutical composition and a pharmaceutical preparation, comprising
(i) the active ingredient M with a content of 0.5%-99%; in some embodiments, the active ingredient M is present with a content of 1%-90%; in some embodiments, the active ingredient M is present with a content of 1%-80%; in some embodiments, the active ingredient M is present with a content of 1%-70%; in some embodiments, the active ingredient M is present with a content of 1%-60%; in some embodiments, the active ingredient M is present with a content of 1%-50%; in some embodiments, the active ingredient M is present with a content of 1%-40%; in some embodiments, the active ingredient M is present with a content of 1%-30%; in some embodiments, the active ingredient M is present with a content of 1%-20%; in some embodiments, the active ingredient M is present with a content of 1%-10%; in some embodiments, the active ingredient M is present with a content of 5%-90%; in some embodiments, the active ingredient M is present with a content of 5%-80%; in some embodiments, the active ingredient M is present with a content of 5%-70%; in some embodiments, the active ingredient M is present with a content of 5%-60%; in some embodiments, the active ingredient M is present with a content of 5%-45%; in some embodiments, the active ingredient M is present with a content of 5%-40%; in some embodiments, the active ingredient M is present with a content of 5%-30%; in some embodiments, the active ingredient M is present with a content of 5%-20%; in some embodiments, the active ingredient M is present with a content of 5%-10%; in some embodiments, the active ingredient M is present with a content of 10%-90%; in some embodiments, the active ingredient M is present with a content of 10%-80%; in some embodiments, the active ingredient M is present with a content of 10%-70%; in some embodiments, the active ingredient M is present with a content of 10%-60%; in some embodiments, the active ingredient M is present with a content of 10%-50%; in some embodiments, the active ingredient M is present with a content of 10%-40%; in some embodiments, the active ingredient M is present with a content of 10%-30%; in some embodiments, the active ingredient M is present with a content of 10%-25%; in some embodiments, the active ingredient M is present with a content of 25%-40%; in some embodiments, the active ingredient M is present with a content of 25%-50%; in some embodiments, the active ingredient M is present with a content of 42%; in some embodiments, the active ingredient M is present with a content of 25%; in some embodiments, the active ingredient M is present with a content of 10%;
(ii) a filler, which is one or more of microcrystalline cellulose, silicified microcrystalline cellulose or sorbitol with a content of 10%-90%; in some embodiments, the filler is present with a content of 10%-80%; in some embodiments, the filler is present with a content of 10%-70%; in some embodiments, the filler is present with a content of 10%-60%; in some embodiments, the filler is present with a content of 10%-50%; in some embodiments, the filler is present with a content of 10%-40%; in some embodiments, the filler is present with a content of 10%-30%; in some embodiments, the filler is present with a content of 10%-20%; in some embodiments, the filler is present with a content of 30%-80%; in some embodiments, the filler is present with a content of 40%-80%; in some embodiments, the filler is present with a content of 37%; in some embodiments, the filler is present with a content of 40%; in some embodiments, the filler is present with a content of 72.5%; in some embodiments, the filler is present with a content of 80%;
(iii) a binder, which is one or more of hypromellose, methyl cellulose or sodium carboxymethyl cellulose with a content of 1%-50%; in some embodiments, the binder is present with a content of 1%-40%; in some embodiments, the binder is present with a content of 1%-30%; in some embodiments, the binder is present with a content of 1%-20%; in some embodiments, the binder is present with a content of 1%-15%; in some embodiments, the binder is present with a content of 1%-10%; in some embodiments, the binder is present with a content of 1%-6%; in some embodiments, the binder is present with a content of 1%-5%; in some embodiments, the binder is present with a content of 1%-4%; in some embodiments, the binder is present with a content of 1%-3%; in some embodiments, the binder is present with a content of 1%-2.5%; in some embodiments, the binder is present with a content of 1.5%-2.5%; in some embodiments, the binder is present with a content of 2%-2.5%; in some embodiments, the binder is present with a content of 2%; in some embodiments, the binder is present with a content of 2.5%; in some embodiments, the binder is present with a content of 3%;
(iv) a glidant, which is one or more of silicon dioxide or micronized silica gel with a content of 0.5%-10%; in some embodiments, the glidant is present with a content of 0.5%-5%; in some embodiments, the glidant is present with a content of 0.5%-4%; in some embodiments, the glidant is present with a content of 0.5%-3%; in some embodiments, the glidant is present with a content of 0.5%-2%; in some embodiments, the glidant is present with a content of 1%-2%; in some embodiments, the glidant is present with a content of 0.5%; in some embodiments, the glidant is present with a content of 1%; in some embodiments, the glidant is present with a content of 2%; in some embodiments, the glidant is present with a content of 3%;
(v) a lubricant, which is sodium stearyl fumarate with a content of 0.1%-10%; in some embodiments, the lubricant is present with a content of 0.1%-8%; in some embodiments, the lubricant is present with a content of 0.1%-6%; in some embodiments, the lubricant is present with a content of 0.1%-5%; in some embodiments, the lubricant is present with a content of 0.5%-2.0%; in some embodiments, the lubricant is present with a content of 0.5%-1%; in some embodiments, the lubricant is present with a content of 0.5%; in some embodiments, the lubricant is present with a content of 0.6%; in some embodiments, the lubricant is present with a content of 0.7%; in some embodiments, the lubricant is present with a content of 0.8%; in some embodiments, the lubricant is present with a content of 0.9%; in some embodiments, the lubricant is present with a content of 1.0%.
(vi) a disintegrant, which is one or more of copovidone, croscarmellose sodium, or calcium carboxymethylcellulose with a content of 0.5%-10%; in some embodiments, the disintegrant is present with a content of 1%-10%; in some embodiments, the disintegrant is present with a content of 1%-9%; in some embodiments, the disintegrant is present with a content of 1%-8%; in some embodiments, the disintegrant is present with a content of 1%-7%; in some embodiments, the disintegrant is present with a content of 1%-6%; in some embodiments, the disintegrant is present with a content of 1%-5%; in some embodiments, the disintegrant is present with a content of 1%-4%; in some embodiments, the disintegrant is present with a content of 1%-3%; in some embodiments, the disintegrant is present with a content of 1%-2%; in some embodiments, the disintegrant is present with a content of 1.5%-10%; in some embodiments, the disintegrant is present with a content of 2%-10%; in some embodiments, the disintegrant is present with a content of 2%-8%; in some embodiments, the disintegrant is present with a content of 2%-7%; in some embodiments, the disintegrant is present with a content of 2%-6%; in some embodiments, the disintegrant is present with a content of 3%-7%; in some embodiments, the disintegrant is present with a content of 4%-7%; in some embodiments, the disintegrant is present with a content of 5%-7%; in some embodiments, the disintegrant is present with a content of 2%; in some embodiments, the disintegrant is present with a content of 3%; in some embodiments, the disintegrant is present with a content of 4%; in some embodiments, the disintegrant is present with a content of 6%; in some embodiments, the disintegrant is present with a content of 8%; in some embodiments, the disintegrant is present with a content of 10%;
(vii) in some embodiments, the pharmaceutical composition and the pharmaceutical preparation further comprise one or more of a solubilizer, a surfactant and a pH regulator, and preferably further comprise one of a solubilizer, a surfactant and a pH regulator;
   the solubilizer is one or more of cyclodextrin or hydroxypropyl betacyclodextrin with a content of 5%-50%; in some embodiments, the solubilizer is present with a content of 10%-50%; in some embodiments, the solubilizer is present with a content of 10%-45%; in some embodiments, the solubilizer is present with a content of 10%-40%; in some embodiments, the solubilizer is present with a content of 30%-50%; in some embodiments, the solubilizer is present with a content of 30%-45%; in some embodiments, the solubilizer is present with a content of 10%; in some embodiments, the solubilizer is present with a content of 20%; in some embodiments, the solubilizer is present with a content of 30%; in some embodiments, the solubilizer is present with a content of 32%; in some embodiments, the solubilizer is present with a content of 40%; in some embodiments, the solubilizer is present with a content of 42.5%; in some embodiments, the solubilizer is present with a content of 45%;
   the surfactant is sodium dodecyl sulphate with a content of 1%-40%; in some embodiments, the surfactant is present with a content of 1%-30%; in some embodiments, the surfactant is present with a content of 1%-20%; in some embodiments, the surfactant is present with a content of 5%-20%; in some embodiments, the surfactant is present with a content of 10%-20%; in some embodiments, the surfactant is present with a content of 10%-15%; in some embodiments, the surfactant is present with a content of 5%; in some embodiments, the surfactant is present with a content of 10%; in some embodiments, the surfactant is present with a content of 15%; in some embodiments, the surfactant is present with a content of 20%;
   the pH regulator is magnesium oxide with a content of 1%-50%; in some embodiments, the pH regulator is present with a content of 10%-50%; in some embodiments, the pH regulator is present with a content of 1%-10%; in some embodiments, the pH regulator is present with a content of 1%-9%; in some embodiments, the pH regulator is present with a content of 1%-8%; in some embodiments, the pH regulator is present with a content of 1%-6%; in some embodiments, the pH regulator is present with a content of 1%-5%; in some embodiments, the pH regulator is present with a content of 1%; in some embodiments, the pH regulator is present with a content of 2%; in some embodiments, the pH regulator is present with a content of 2.5%; in some embodiments, the pH regulator is present with a content of 3%; in some embodiments, the pH regulator is present with a content of 4%; in some embodiments, the pH regulator is present with a content of 5%.

Optionally, the pharmaceutical preparation as described above is characterised in that the binder can be added in a solution state or in a powder state; and the disintegrant can be added internally, externally, or both.

The present invention also provides a use of the pharmaceutical composition or the pharmaceutical preparation in preparation of a related drug for treating cancers.

The present invention also provides a method for treating a disease in a mammal, comprising administering to the mammal a therapeutically effective amount of the compound or the pharmaceutical composition disclosed in the present application, wherein the therapeutically effective amount is preferably 1-1000 mg, and the disease is preferably cancer, for example, BRAF-mediated cancers, such as solid tumours.

The term "effective amount" or "therapeutically effective amount" according to the present application refers to a sufficient amount of the compound disclosed in the present application that is administered to ameliorate, to some extent, one or more symptoms of a disease or condition (such as cancer) being treated. In some embodiments, the outcome is the reduction and/or remission of signs, symptoms or causes of the disease, or any other desired change in the biological system. For example, an "effective amount" in terms of the therapeutic use is an amount of the composition comprising the compound disclosed in the present application that is required to provide clinically significant reduction of the symptoms of the disease. Examples of therapeutically effective amounts include, but are not limited to, 1-1000 mg, 2-1000 mg, 3-1000 mg, 4-1000 mg, 5-1000 mg, 6-1000 mg, 10-1000 mg, 20-1000 mg, 25-1000 mg, 30-1000 mg, 40-1000 mg, 50-1000 mg, 60-1000 mg, 70-1000 mg, 75-1000 mg, 80-1000 mg, 90-1000 mg, 100-1000 mg, 200-1000 mg, 300-1000 mg, 400-1000 mg, 1-900 mg, 2-900 mg, 3-900 mg, 4-900 mg, 5-900 mg, 6-900 mg, 10-900 mg, 20-900 mg, 25-900 mg, 30-900 mg, 40-900 mg, 50-900 mg, 60-900 mg, 70-900 mg, 75-900 mg, 80-900 mg, 90-900 mg, 100-900 mg, 200-900 mg, 300-900 mg, 400-900 mg 1-800 mg, 2-800 mg, 3-800 mg, 4-800 mg, 5-800 mg, 6-800 mg, 10-800 mg, 20-800 mg, 25-800 mg, 30-800 mg, 40-800 mg, 50-800 mg, 60-800 mg, 70-800 mg, 75-800 mg, 80-800 mg, 90-800 mg, 100-800 mg, 200-800 mg, 300-800 mg, 400-800 mg 1-700 mg, 2-700 mg, 3-700 mg, 4-700 mg, 5-700 mg, 6-700 mg, 10-700 mg, 20-700 mg, 25-700 mg, 30-700 mg, 40-700 mg, 50-700 mg, 60-700 mg, 70-700 mg, 75-700 mg, 80-700 mg, 90-700 mg, 100-700 mg, 200-700 mg, 300-700 mg, 400-700 mg, 1-600 mg, 2-600 mg, 3-600 mg, 4-600 mg, 5-600 mg, 6-500 mg, 10-500 mg, 20-500 mg, 25-500 mg, 30-500 mg, 40-500 mg, 50-500 mg, 60-500 mg, 70-500 mg, 75-500 mg, 80-500 mg, 90-500 mg, 100-500 mg, 125-500 mg, 150-500 mg, 200-500 mg, 250-500 mg, 300-500 mg, 400-500 mg, 5-400 mg, 10-400 mg, 20-400 mg, 25-400 mg, 30-400 mg, 40-400 mg, 50-400 mg, 60-400 mg, 70-400 mg, 75-400 mg, 80-400 mg, 90-400 mg, 100-400 mg, 125-400 mg, 150-400 mg, 200-400 mg, 250-400 mg, 300-400 mg, 1-300 mg, 2-300 mg, 5-300 mg, 10-300 mg, 20-300 mg, 25-300 mg, 30-300 mg, 40-300 mg, 50-300 mg, 60-300 mg, 70-300 mg, 75-300 mg, 80-300 mg, 90-300 mg, 100-300 mg, 125-300 mg, 150-300 mg, 200-300 mg, 250-300 mg, 1-200 mg, 2-200 mg, 5-200 mg, 10-200 mg, 20-200 mg, 25-200 mg, 30-200 mg, 40-200 mg, 50-200 mg, 60-200 mg, 70-200 mg, 75-200 mg, 80-200 mg, 90-200 mg, 100-200 mg, 125-200 mg, 150-200 mg, 1-100 mg, 2-100 mg, 5-100 mg, 10-100 mg, 15-100 mg, 20-100 mg, 25-100 mg, 30-100 mg, 40-100 mg, 50-100 mg, 60-100 mg, 70-100 mg, 75-100 mg, 80-100 mg, and 90-100 mg; in some embodiments, examples of therapeutically effective amounts include, but are not limited to, 1mg, 5mg, 10 mg, 15mg, 20 mg, 25mg, 30 mg, 35mg, 40 mg, 45mg, 50 mg, 55mg, 65mg, 70 mg, 75mg, 80 mg, 85mg, 90 mg, 95mg, 100 mg, 110 mg, 120 mg, 125mg, 130 mg, 140 mg, 150 mg, 160 mg, 170 mg, 180 mg, 190 mg, 200 mg, 210 mg, 220 mg, 230 mg, 240 mg, 250 mg, 300 mg, 350 mg, 400 mg, 600 mg, and 800 mg.

In some embodiments, the specification of the preparation of the pharmaceutical composition or pharmaceutical preparation of the present invention includes, but is not limited to, 1-1000 mg, 2-1000 mg, 3-1000 mg, 4-1000 mg, 5-1000 mg, 6-1000 mg, 10-1000 mg, 20-1000 mg, 25-1000 mg, 30-1000 mg, 40-1000 mg, 50-1000 mg, 60-1000 mg, 70-1000 mg, 75-1000 mg, 80-1000 mg, 90-1000 mg, 100-1000 mg, 200-1000 mg, 300-1000 mg, 400-1000 mg, 1-900 mg, 2-900 mg, 3-900 mg, 4-900 mg, 5-900 mg, 6-900 mg, 10-900 mg, 20-900 mg, 25-900 mg, 30-900 mg, 40-900 mg, 50-900 mg, 60-900 mg, 70-900 mg, 75-900 mg, 80-900 mg, 90-900 mg, 100-900 mg, 200-900 mg, 300-900 mg, 400-900 mg, 1-800 mg, 2-800 mg, 3-800 mg, 4-800 mg, 5-800 mg, 6-800 mg, 10-800 mg, 20-800 mg, 25-800 mg, 30-800 mg, 40-800 mg, 50-800 mg, 60-800 mg, 70-800 mg, 75-800 mg, 80-800 mg, 90-800 mg, 100-800 mg, 200-800 mg, 300-800 mg, 400-800 mg, 1-700 mg, 2-700 mg, 3-700 mg, 4-700 mg, 5-700 mg, 6-700 mg, 10-700 mg, 20-700 mg, 25-700 mg, 30-700 mg, 40-700 mg, 50-700 mg, 60-700 mg, 70-700 mg, 75-700 mg, 80-700 mg, 90-700 mg, 100-700 mg, 200-700 mg, 300-700 mg, 400-700 mg, 1-600 mg, 2-600 mg, 3-600 mg, 4-600 mg, 5-600 mg, 6-500 mg, 10-500 mg, 20-500 mg, 25-500 mg, 30-500 mg, 40-500 mg, 50-500 mg, 60-500 mg, 70-500 mg, 75-500 mg, 80-500 mg, 90-500 mg, 100-500 mg, 125-500 mg, 150-500 mg, 200-500 mg, 250-500 mg, 300-500 mg, 400-500 mg, 5-400 mg, 10-400 mg, 20-400 mg, 25-400 mg, 30-400 mg, 40-400 mg, 50-400 mg, 60-400 mg, 70-400 mg, 75-400 mg, 80-400 mg, 90-400 mg, 100-400 mg, 125-400 mg, 150-400 mg, 200-400 mg, 250-400 mg, 300-400 mg, 1-300 mg, 2-300 mg, 5-300 mg, 10-300 mg, 20-300 mg, 25-300 mg, 30-300 mg, 40-300 mg, 50-300 mg, 60-300 mg, 70-300 mg, 75-300 mg, 80-300 mg, 90-300 mg, 100-300 mg, 125-300 mg, 150-300 mg, 200-300 mg, 250-300 mg, 1-200 mg, 2-200 mg, 5-200 mg, 10-200 mg, 20-200 mg, 25-200 mg, 30-200 mg, 40-200 mg, 50-200 mg, 60-200 mg, 70-200 mg, 75-200 mg, 80-200 mg, 90-200 mg, 100-200 mg, 125-200 mg, 150-200 mg, 1-100 mg, 2-100 mg, 5-100 mg, 10-100 mg, 15-100 mg, 20-100 mg, 25-100 mg, 30-100 mg, 40-100 mg, 50-100 mg, 60-100 mg, 70-100 mg, 75-100 mg, 80-100 mg, and 90-100 mg; in some embodiments, examples of therapeutically effective amounts include, but are not limited to, 1mg, 5mg, 10 mg, 15mg, 20 mg, 25mg, 30 mg, 35mg, 40 mg, 45mg, 50 mg, 55mg, 65mg, 70 mg, 75mg, 80 mg, 85mg, 90 mg, 95mg, 100 mg, 110 mg, 120 mg, 125mg, 130 mg, 140 mg, 150 mg, 160 mg, 170 mg, 180 mg, 190 mg, 200 mg, 210 mg, 220 mg, 230 mg, 240 mg, 250 mg, 300 mg, 350 mg, 400 mg, 600 mg, and 800 mg.

In some embodiments, the amount of active ingredient M in the unit preparation of the pharmaceutical composition or pharmaceutical preparation of the present invention includes, but is not limited to, 1-1000 mg, 2-1000 mg, 3-1000 mg, 4-1000 mg, 5-1000 mg, 6-1000 mg, 10-1000 mg, 20-1000 mg, 25-1000 mg, 30-1000 mg, 40-1000 mg, 50-1000 mg, 60-1000 mg, 70-1000 mg, 75-1000 mg, 80-1000 mg, 90-1000 mg, 100-1000 mg, 200-1000 mg, 300-1000 mg, 400-1000 mg, 1-900 mg, 2-900 mg, 3-900 mg, 4-900 mg, 5-900 mg, 6-900 mg, 10-900 mg, 20-900 mg, 25-900 mg, 30-900 mg, 40-900 mg, 50-900 mg, 60-900 mg, 70-900 mg, 75-900 mg, 80-900 mg, 90-900 mg, 100-900 mg, 200-900 mg, 300-900 mg, 400-900m, 1-800 mg, 2-800 mg, 3-800 mg, 4-800 mg, 5-800 mg, 6-800 mg, 10-800 mg, 20-800 mg, 25-800 mg, 30-800 mg, 40-800 mg, 50-800 mg, 60-800 mg, 70-800 mg, 75-800 mg, 80-800 mg, 90-800 mg, 100-800 mg, 200-800 mg, 300-800 mg, 400-800 mg, 1-700 mg, 2-700 mg, 3-700 mg, 4-700 mg, 5-700 mg, 6-700 mg, 10-700 mg, 20-700 mg, 25-700 mg, 30-700 mg, 40-700 mg, 50-700 mg, 60-700 mg, 70-700 mg, 75-700 mg, 80-700 mg, 90-700 mg, 100-700 mg, 200-700 mg, 300-700 mg, 400-700 mg, 1-600 mg, 2-600 mg, 3-600 mg, 4-600 mg, 5-600 mg, 6-600 mg, 10-600 mg, 20-600 mg, 25-600 mg, 30-600 mg, 40-600 mg, 50-600 mg, 60-600 mg, 70-600 mg, 75-600 mg, 80-600 mg, 90-600 mg, 100-600 mg, 200-600 mg, 300-600 mg, 400-600 mg, 1-500 mg, 2-500 mg, 3-500 mg, 4-500 mg, 5-500 mg, 6-500 mg, 10-500 mg, 20-500 mg, 25-500 mg, 30-500 mg, 40-500 mg, 50-500 mg, 60-500 mg, 70-500 mg, 75-500 mg, 80-500 mg, 90-500 mg, 100-500 mg, 125-500 mg, 150-500 mg, 200-500 mg, 250-500 mg, 300-500 mg, 400-500 mg, 5-400 mg, 10-400 mg, 20-400 mg, 25-400 mg, 30-400 mg, 40-400 mg, 50-400 mg, 60-400 mg, 70-400 mg, 75-400 mg, 80-400 mg, 90-400 mg, 100-400 mg, 125-400 mg, 150-400 mg, 200-400 mg, 250-400 mg, 300-400 mg, 1-300 mg, 2-300 mg, 5-300 mg, 10-300 mg, 20-300 mg, 25-300 mg, 30-300 mg, 40-300 mg, 50-300 mg, 60-300 mg, 70-300 mg, 75-300 mg, 80-300 mg, 90-300 mg, 100-300 mg, 125-300 mg, 150-300 mg, 200-300 mg, 250-300 mg, 1-200 mg, 2-200 mg, 5-200 mg, 10-200 mg, 20-200 mg, 25-200 mg, 30-200 mg, 40-200 mg, 50-200 mg, 60-200 mg, 70-200 mg, 75-200 mg, 80-200 mg, 90-200 mg, 100-200 mg, 125-200 mg, 150-200 mg, 1-100 mg, 2-100 mg, 5-100 mg, 10-100 mg, 15-100 mg, 20-100 mg, 25-100 mg, 30-100 mg, 40-100 mg, 50-100 mg, 60-100 mg, 70-100 mg, 75-100 mg, 80-100 mg, and 90-100 mg; in some embodiments, examples of therapeutically effective amounts include, but are not limited to, 1mg, 5mg, 10 mg, 15mg, 20 mg, 25mg, 30 mg, 35mg, 40 mg, 45mg, 50 mg, 55mg, 65mg, 70 mg, 75mg, 80 mg, 85mg, 90 mg, 95mg, 100 mg, 110 mg, 120 mg, 125mg, 130 mg, 140 mg, 150 mg, 160 mg, 170 mg, 180 mg, 190 mg, 200 mg, 210 mg, 220 mg, 230 mg, 240 mg, 250 mg, 300 mg, 350 mg, 400 mg, 600 mg, and 800 mg.

A method for treating a disease in a mammal is provided, the method comprising administering to a subject an active ingredient M at a daily dose of 1-1200 mg/day, wherein the daily dose can be a single dose or divided doses. In some embodiments, the daily dose includes, but is not limited to, 10-3600 mg/day, 25-3600 mg/day, 50-3600 mg/day, 100-3600 mg/day, 200-3600 mg/day, 10-2400 mg/day, 25-2400 mg/day, 50-2400 mg/day, 100-2400 mg/day, 200-2400 mg/day, 10-1200 mg/day, 25-1200 mg/day, 50-1200 mg/day, 100-1200 mg/day, 200-1200 mg/day, 25-600 mg/day, 50-600 mg/day, 100-600 mg/day, or 200-600 mg/day. In some embodiments, the daily dose includes, but is not limited to, 10 mg/day, 20 mg/day, 25 mg/day, 50 mg/day, 100 mg/day, 120 mg/day, 125 mg/day, 150 mg/day, 200 mg/day, 300 mg/day, 240 mg/day, 400 mg/day, 600 mg/day, 800 mg/day, 1000 mg/day, 1200 mg/day, 2400 mg/day, or 3600 mg/day.

The present invention relates to a kit, which may comprise a pharmaceutical composition or pharmaceutical preparation in single-dose or multi-dose form, wherein the kit comprises the active ingredient M in an amount as described in the pharmaceutical composition of the present invention, including but not limited to, 1 mg, 5 mg, 10 mg, 15 mg, 20 mg, 25 mg, 30 mg, 35 mg, 40 mg, 45 mg, 50 mg, 55 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 300 mg, 350 mg, 400 mg, 600 mg, 800 mg, or 1200 mg.

Unless stated to the contrary, the terms used in the description and claims of the present application have the following meanings.

The term "preparation specification" refers to the weight of the active drug (the active ingredient M) contained in each vial, tablet or other unit preparation.

### Synthetic route

The compounds used in the reactions described herein are prepared according to organic synthesis techniques known to those skilled in the art, starting from commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

### Term

Unless otherwise specified, the terms of the present invention have the following meanings.

The carbon, hydrogen, oxygen, sulphur, nitrogen and halogen involved in the groups and compounds of the present invention all include isotopes thereof, and are optionally further replaced by one or more of the corresponding isotopes thereof, wherein the isotopes of carbon include ¹²C, ¹³C and ¹⁴C; the isotopes of hydrogen include protium (H), deuterium (D, also known as heavy hydrogen) and tritium (T, also known as superheavy hydrogen); the isotopes of oxygen include ¹⁶O, ¹⁷O and ¹⁸O; the isotopes of sulphur include ³²S, ³³S, ³⁴S and ³⁶S; the isotopes of nitrogen include ¹⁴N and ¹⁵N; the isotope of fluorine includes ¹⁹F; the isotopes of chlorine include ³⁵Cl and ³⁷Cl; and the isotopes of bromine include ⁷⁹Br and ⁸¹Br.

The term "halogen" herein refers to F, Cl, Br, I, or isotopes thereof.

The term "halo" or "substituted with halogen" refers to being substituted with one or more groups selected from F, Cl, Br, I, or isotopes thereof, wherein the upper limit of the number of halogen substituents is equal to the sum of the number of hydrogens that can be substituted in the group to be substituted. Without particular limitation, the number of halogen substituents is any integer between 1 and the upper limit, and when the number of halogen substituents is greater than 1, the group to be substituted can be substituted with the same or different halogen. Generally, the circumstances of being substituted with 1-5 halogen, 1-3 halogen, 1-2 halogen, and 1 halogen are included.

The term "deuterium" refers to the isotope deuterium of hydrogen (H).

The term "deuterated" or "deuterated product" refers to the case where a hydrogen atom on a group, such as alkyl, cycloalkyl, alkylene, aryl, heteroaryl, mercapto, heterocycloalkyl, alkenyl and alkynyl is substituted with at least one deuterium atom, wherein the upper limit of the number of deuterium substituents is equal to the sum of the number of hydrogens that can be substituted in the group to be substituted. Without particular limitation, the number of deuterium substituents is any integer between 1 and the upper limit, for example, 1-20 deuterium atoms, 1-10 deuterium atoms, 1-6 deuterium atoms, 1-3 deuterium atoms, 1-2 deuterium atoms or 1 deuterium atom.

Group "C_{x-y}" refers to a group comprising x to y carbon atoms, for example, "C₁₋₆ alkyl" refers to alkyl comprising 1-6 carbon atoms.

The term "alkyl" refers to a monovalent straight or branched saturated aliphatic hydrocarbon group, usually an alkyl group with 1 to 20 carbon atoms, or an alkyl group with 1 to 8 carbon atoms, or an alkyl group with 1 to 6 carbon atoms, or an alkyl group with 1 to 4 carbon atoms. Non-limiting examples of alkyl include methyl, ethyl, n-propyl, isopropyl, n-butyl, sec-butyl, neobutyl, tert-butyl, n-pentyl, isopentyl, neopentyl, n-hexyl, etc., and alkyl may be further substituted with a substituent.

The term "alkylene" refers to a divalent straight or branched saturated alkyl group. Examples of alkylene include, but are not limited to methylene, ethylene, *etc.*

The term "haloalkyl" refers to an alkyl group in which one or more hydrogens are replaced by one or more halogen atoms (e.g., fluorine, chlorine, bromine, iodine, or isotopes thereof), wherein the upper limit of the number of halogen substituents is equal to the sum of the number of hydrogens that can be substituted in the alkyl group. Without particular limitation, the number of halogen substituents is any integer between 1 and the upper limit. Generally, the alkyl group is substituted with 1-5 halogen, 1-3 halogen, 1-2 halogen or 1 halogen; and when the number of halogen substituents is greater than 1, the group to be substituted can be substituted with the same or different halogen. Specific examples include, but are not limited to -CF₃, -CH₂Cl, -CH₂CF₃, -CCl₂, CF₃, etc.

The term "alkoxy" or "alkyloxy" refers to -O-alkyl, such as -O-C₁₋₈ alkyl, -O-C₁₋₆ alkyl, -O-C₁₋₄ alkyl or -O-C₁₋₂ alkyl. Non-limiting and specific examples of alkoxy or alkyloxy include methoxy, ethoxy, n-propoxy, isopropoxy, n-butoxy, sec-butoxy, tert-butoxy, n-pentoxy, n-hexyloxy, cyclopropoxy, cyclobutoxy, etc. The alkoxy may be optionally substituted with a substituent.

The term "haloalkoxy" refers to -O-haloalkyl, such as -O-halo C₁₋₈ alkyl, -O-halo C₁₋₆ alkyl, -O-halo C₁₋₄ alkyl or -O-halo C₁₋₂ alkyl; the upper limit of the number of halogen substituents is equal to the sum of the number of hydrogens that can be substituted in the group to be substituted. Without particular limitation, the number of halogen substituents is any integer between 1 and the upper limit, preferably 1-5 halogen, 1-3 halogen, 1-2 halogen, and 1 halogen; and when the number of halogen substituents is greater than 1, the group to be substituted can be substituted with the same or different halogen. Non-limiting examples of haloalkoxy include monofluoromethoxy, difluoromethoxy, trifluoromethoxy, difluoroethyloxy, *etc.*

The term "alkenyl" refers to a straight or branched hydrocarbon group comprising at least one carbon-carbon double bond (C=C) and generally comprises 2 to 18 carbon atoms, such as 2 to 8 carbon atoms, further such as 2 to 6 carbon atoms, and still further such as 2 to 4 carbon atoms. Examples of alkenyl include, but are not limited to ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc.; and the alkenyl may further be optionally substituted with a substituent.

The term "alkenylene" refers to a straight or branched divalent unsaturated hydrocarbon group comprising at least one carbon-carbon double bond (C=C). Unless otherwise specified, the alkynylene contains 2-6 carbon atoms, preferably 2-4 carbon atoms. Non-limiting examples of alkynylene include ethynylene, and the alkenylene may be optionally substituted with a substituent.

The term "alkynyl" refers to a straight or branched hydrocarbon group comprising at least one carbon-carbon triple bond (C≡C) and generally comprises 2 to 18 carbon atoms, further comprises 2 to 8 carbon atoms, further comprises 2 to 6 carbon atoms, and still further comprises 2 to 4 carbon atoms. Examples of alkynyl include, but are not limited to ethynyl, 1-propynyl, 2-propynyl, butynyl, 2-butynyl, 3-butynyl, 1-methyl-2-propynyl, 4-pentynyl, 3-pentynyl, 1-methyl-2-butynyl, 2-hexynyl, 3-hexynyl, 2-heptynyl, 3-heptynyl, 4-heptynyl, 3-octynyl, 3-nonynyl, 4-decynyl, etc.; and the alkynyl may be optionally substituted with a substituent.

The term "alkynylene" refers to a straight or branched divalent unsaturated hydrocarbon group comprising a carbon-carbon triple bond (C≡C) and generally comprises 2-6 carbon atoms, and further comprises 2-4 carbon atoms. Non-limiting examples of alkynylene include ethynylene, propynylene and butynylene; and the alkynylene may be optionally substituted with a substituent.

The term "cycloalkyl" refers to a saturated or partially unsaturated, non-aromatic carbocyclic hydrocarbon group containing no ring heteroatoms. The cycloalkyl may be monocyclic, bicyclic or polycyclic, the bicyclic or polycyclic cycloalkyl may be in the form of a fused ring, a spiro ring, a bridged ring or a combination thereof, and may comprise one or more aromatic rings, but the ring system is non-aromatic as a whole, and the attachment site may be on an aromatic ring or a non-aromatic ring. Generally, the cycloalkyl contains 3 to 20 carbon atoms, further contains 3-8 carbon atoms, and still further contains 3-6 carbon atoms; when the cycloalkyl is monocyclic cycloalkyl, the cycloalkyl contains 3-15 carbon atoms, or 3-10 carbon atoms, or 3-8 carbon atoms, or 3-6 carbon atoms; when the cycloalkyl is bicyclic or polycyclic cycloalkyl, the cycloalkyl contains 5-12 carbon atoms, or 5-11 carbon atoms, or 6-10 carbon atoms. Non-limiting examples of cycloalkyl include cyclopropyl, cyclobutyl, cyclopentyl, cyclohexyl, cycloheptyl, butenyl, cyclopentenyl, cyclohexenyl, 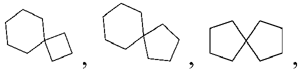 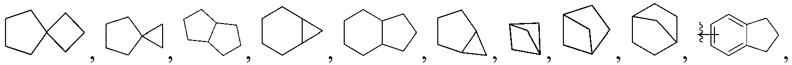 etc., and the cycloalkyl may be optionally substituted with a substituent.

The term "cycloalkylene" refers to a divalent group of cycloalkyl.

The term "aryl" refers to an aromatic carbocycle that does not contain heteroatoms, including monocyclic aryl and fused aryl. Generally, the aryl contains 6 to 13 carbon atoms, and further contains 6 to 9 carbon atoms, and it is further phenyl. Non-limiting examples of aryl include phenyl, naphthyl, anthryl and phenanthryl, and the aryl may be optionally substituted with a substituent.

"Carbocycle" or "carbocyclyl" refers to a saturated, partially unsaturated, or aromatic carbocycle, and its meaning includes aryl and cycloalkyl. The carbocycle may be monocyclic, bicyclic or polycyclic, and the bicyclic or polycyclic carbocycle may be in the form of a bridged ring, a fused ring, a spiro ring and a combination thereof. Generally, the carbocycle contains 3-12 carbon atoms, or 3-10 carbon atoms, or 3-6 carbon atoms. Non-limiting examples of the monocyclic carbocycle include cyclopropyl, cyclobutyl, cyclopentyl, cyclohexyl, cycloheptyl, phenyl, *etc.* A bicyclic bridged ring includes 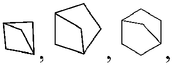 *etc.,* a bicyclic fused ring includes 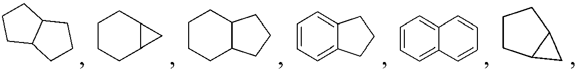 *etc.,* and a bicyclic spiro ring includes 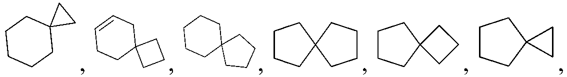 *etc.* The carbocycle may be optionally substituted with a substituent.

"Heterocycloalkyl" refers to a saturated or partially unsaturated non-aromatic carbocycle containing 1, 2, 3, or 4 heteroatoms selected from N, S or O. The heterocycloalkyl may be monocyclic, bicyclic or polycyclic, the bicyclic or polycyclic heterocycloalkyl may be in the form of a bridged ring, a fused ring, a spiro ring or a combination thereof, and may comprise one or more aromatic rings or heteroaromatic rings, but the ring system is non-aromatic as a whole, and the attachment site may be on an aromatic ring or a non-aromatic ring. Generally, the heterocycloalkyl is a 3- to 20-membered ring. When the heterocycloalkyl is monocyclic heterocycloalkyl, the heterocycloalkyl is usually a 3- to 15-membered ring, or a 3- to 10-membered ring, or a 3- to 8-membered ring, or a 3- to 6-membered ring; when the heterocycloalkyl is bicyclic or polycyclic heterocycloalkyl, the heterocycloalkyl is usually a 5- to 12-membered ring, or a 5-to 11-membered ring, or a 6- to 9-membered ring. The heteroatoms N and S include their oxidation states. Non-limiting examples of heterocycloalkyl include azetidinyl, morpholinyl, piperazinyl, piperidyl, tetrahydropyranyl, oxetanyl, pyranyl, azacyclopentenyl, azacyclohexenyl, oxacyclopentenyl, oxacyclohexenyl, etc., and the heterocycloalkyl may be optionally substituted with a substituent.

"Heteroaromatic ring" or "heteroaryl", unless otherwise specified, refers to an aromatic ring containing 1 to 4 heteroatoms selected from N, O or S and their oxidation states, which may be monocyclic, bicyclic or polycyclic, wherein the bicyclic or polycyclic heteroaromatic ring or heteroaryl may be in the form of a bridged ring, a fused ring, a spiro ring and a combination thereof. The bicyclic or polycyclic heteroaromatic ring or heteroaryl can be formed by fusion of heteroaryl to aryl, or of heteroaryl to heteroaryl, wherein the heteroaryl or aryl may be the attachment site. Non-limiting examples of heteroaromatic ring or heteroaryl include furyl, thienyl, pyrrolyl, oxazolyl, thiazolyl, imidazolyl, pyrazolyl, pyridyl, pyrimidyl, pyridazinyl, pyrazinyl, indolyl, purinyl, 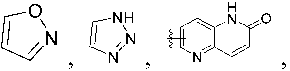 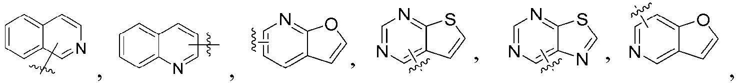 etc. The heteroaryl may be optionally substituted with a substituent.

The term "heterocycle" or "heterocyclyl" refers to a saturated or unsaturated, aromatic or non-aromatic ring containing 1 to 4 heteroatoms selected from N, O or S and their oxidation states, and its meaning includes heteroaryl and heterocycloalkyl. The heterocycle may be in the form of a monocyclic heterocycle, a bicyclic bridged heterocycle, a bicyclic fused heterocycle, a bicyclic spiro heterocycle or a combination thereof. The heterocycle is usually 3- to 12-membered heterocycle, or 5- to 12-membered heterocycle, or 5- to 7-membered heterocycle. Heterocyclyl can be connected to a heteroatom or a carbon atom. Non-limiting examples of heterocyclyl include oxiranyl, aziridinyl, oxetanyl, azetidinyl, 1,3-dioxolanyl, 1,4-dioxolanyl, 1,3-dioxanyl, piperazinyl, azacycloheptyl, pyridyl, furyl, thienyl, pyranyl, N-alkylpyrrolyl, pyrimidyl, pyrazinyl, pyrazolyl, pyridazinyl, imidazolyl, piperidyl, piperadinyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, oxazolyl, dihydrooxazolyl, tetrahydrooxazolyl, tetrahydrothiazolyl, tetrahydropyranyl, benzoimidazolyl, benzopyridyl, pyrrolopyridyl, benzodihydrofuryl, azabicyclo[3.2.1]octanyl, azabicyclo[5.2.0]nonanyl, oxatricyclo[5.3.1.1]dodecyl, azaadamantyl and oxaspiro[3.3]heptanyl, 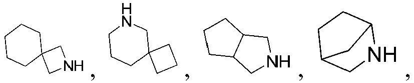 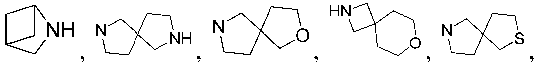 etc., and the heterocycle may be optionally substituted with a substituent.

The term "heterocyclene" refers to a substituted or unsubstituted, saturated or unsaturated, aromatic or non-aromatic, divalent heterocyclyl group. Non-limiting examples of heterocyclene include 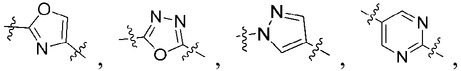 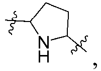 *etc.*

The term "spiro ring" refers to a polycyclic group sharing one carbon atom (referred to as a spiro atom) between rings, which may contain 0 or at least 1 double or triple bond, and may contain 0 to 5 heteroatoms selected from N, O, S, P, Si and their oxidation states. Generally, a spiro ring is a 6- to 14-membered ring, or a 6- to 12-membered ring, or a 6- to 10-membered ring. Generally, a spiro ring is a spiro ring formed by a three-membered ring and a three-membered ring, a three-membered ring and a four-membered ring, a three-membered ring and a five-membered ring, a three-membered ring and a six-membered ring, a four-membered ring and a four-membered ring, a four-membered ring and a five-membered ring, a four-membered ring and a six-membered ring, a five-membered ring and a five-membered ring or a five-membered ring and a six-membered ring. Non-limiting examples of the spiro ring include: 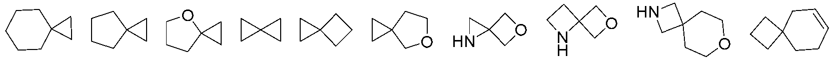 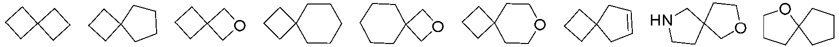 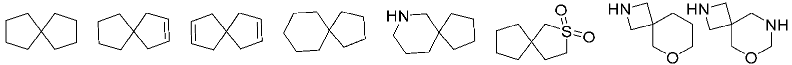 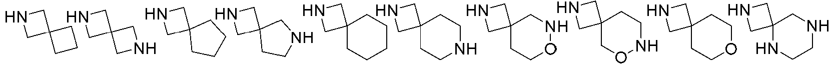 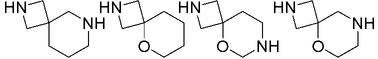 , and the spiro ring may be optionally substituted with a substituent.

The term "fused ring" refers to a polycyclic group in which the rings share two adjacent ring atoms and one chemical bond, which may contain one or more double or triple bonds, and may contain 0 to 5 heteroatoms selected from N, S, O, P, Si and their oxidation states. Generally, a fused ring is a 5- to 20-membered ring, or a 5- to 14-membered ring, or a 5- to 12-membered ring or a 5- to 10-membered ring. Generally, a fused ring is in the form of a three-membered ring fused a four-membered ring (indicating a fused ring formed by a three-membered ring and a four-membered ring, and either the three-membered ring or the four-membered ring may be possibly used as the basic ring according to the IUPC nomenclature; similarly hereinafter), a three-membered ring fused a five-membered ring, a three-membered ring fused a six-membered ring, a four-membered ring fused a four-membered ring, a four-membered ring fused a five-membered ring, a four-membered ring fused a six-membered ring, a five-membered ring fused a five-membered ring, a five-membered ring fused a six-membered ring, and a six-membered ring fused a six-membered ring. Non-limiting examples of the fused ring include purine, quinoline, isoquinoline, benzopyran, benzofuran, benzothiophene, and 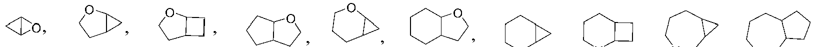 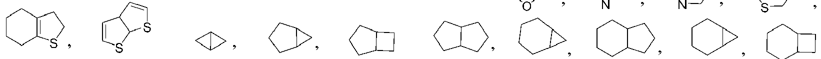 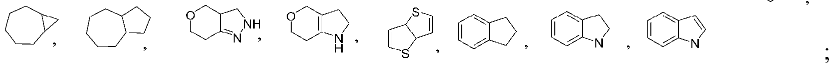 the fused ring may be optionally substituted with a substituent.

The term "bridged ring" refers to a ring system in which two non-adjacent ring atoms are shared between two rings, which may contain one or more double or triple bonds. The bridged ring may contain 0 to 5 heteroatoms selected from N, S, O, P, Si and their oxidation states. Generally, the bridged ring has 5 to 20, or 5 to 14, or 5 to 12, or 5 to 10 ring atoms. Non-limiting examples of the bridged ring include adamantane,

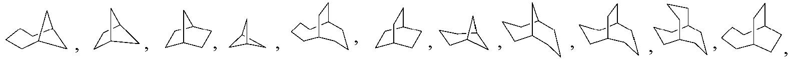

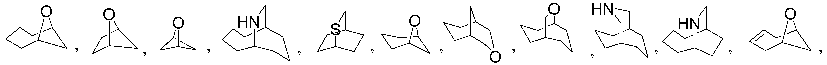

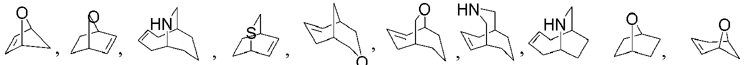

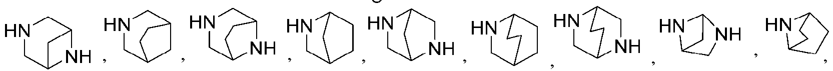

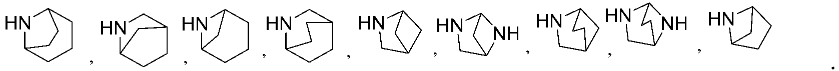

Unless otherwise specified, the term "substitution" or "substituent" refers to any substitution at a position allowed by chemical theory, and the number of substituents conforms to the rules of chemical bonding. Exemplary substituents include, but are not limited to: C₁₋₆ alkyl, C₂₋₆ alkenyl, C₂₋₆ alkynyl, C₃₋₈ heteroalkyl, C₅₋₁₂ aryl, 5- to 12-membered heteroaryl, hydroxyl, C₁₋₆ alkoxy, C₅₋₁₂ aryloxy, thiol, C₁₋₆ alkylthio, cyano, halogen, C₁₋₆ alkylthiocarbonyl, C₁₋₆ alkylcarbamoyl, N-carbamoyl, nitro, silyl, sulfinyl, sulphonyl, sulphoxide, halo C₁₋₆ alkyl, halo C₁₋₆ alkoxy, amino, phosphonic acid, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, -OC(=O)NH(C₁₋₆ alkyl), -NHC(=O)(C₁₋₆ alkyl), -N(C₁₋₆ alkyl)C(=O)(C₁₋₆ alkyl), -NHCO₂(C₁₋₆ alkyl), -NHC(=O)N(C₁₋₆ alkyl)₂, -HC(=O)NH(C₁₋₆ alkyl), -NHC(=O)NH₂, -NHSO₂(C₁₋₆ alkyl), -SO₂N(C₁₋₆ alkyl)₂, -SO₂NH(C₁₋₆ alkyl), -SO₂NH₂, -SO₂C₁₋₆ alkyl, etc.

The term "optional" or "optionally" refers to that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds or stereoisomers, solvates, pharmaceutically acceptable salts or co-crystals thereof as described herein and other components including physiologically/pharmaceutically acceptable carriers and/or excipients.

The term "preparation specification" refers to the weight of the active drug contained in each vial, tablet or other unit preparation.

The term "carrier" refers to: a system that does not cause significant irritation to the organism and does not eliminate the biological activity and characteristics of the administered compound and can change the way the drug enters the human body and the distribution of the drug in the body, control the release rate of the drug and deliver the drug to targeted organs. Non-limiting examples of the carrier include microcapsule, microsphere, nanoparticle, liposome, etc.

The term "excipient" refers to: a substance that is not a therapeutic agent per se, but is used as a diluent, auxiliary material, binder and/or vehicle added to a pharmaceutical composition, thereby improving the disposal or storage properties thereof, or allowing to or promoting the formation of a compound or a pharmaceutical composition into a unit dosage form for administration. As is known to those skilled in the art, pharmaceutically acceptable excipients can provide various functions and can be described as a wetting agent, a buffer, a suspending agent, a lubricant, an emulsifier, a disintegrating agent, an absorbent, a preservative, a surfactant, a colourant, a flavouring agent, and a sweetening agent. Examples of pharmaceutically acceptable excipients include, but are not limited to: (1) sugars, such as lactose, glucose and sucrose; (2) starch, such as corn starch and potato starch; (3) cellulose and its derivatives, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatine; (7) talc; (8) excipients, such as cocoa butter or suppository wax; (9) oils, such as peanut oil, cottonseed oil, safflower oil, sesame oil, olive oil, corn oil and soybean oil; (10) diols, such as propylene glycol; (11) polyols, such as glycerine, sorbitol, mannitol and polyethylene glycol; (12) esters, such as ethyl oleate and ethyl laurate; (13) agar; (14) buffers, such as magnesium hydroxide and aluminium hydroxide; (15) alginic acid; (16) pyrogen-free water; (17) isotonic saline; (18) Ringer's solution; (19) ethanol; (20) pH buffered solution; (21) polyester, polycarbonate and/or polyanhydride; and (22) other non-toxic compatible substances used in a pharmaceutical preparation.

The term "stereoisomer" refers to an isomer produced as a result of different spatial arrangement of atoms in molecules, including cis-trans isomers, optical isomers and conformational isomers.

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

### Detailed Description of Embodiments

The technical solutions of the present invention will be described in detail by the following examples, but the scope of protection of the present invention includes but is not limited thereto.

### Detection method

The structures of the compounds are determined by nuclear magnetic resonance (NMR) or (and) mass spectrometry (MS). The NMR shift (δ) is given in the unit of 10⁻⁶ (ppm). NMR is measured with NMR instruments (Bruker Avance III 400 and Bruker Avance 300), and the solvent for determination is deuterated dimethyl sulphoxide (DMSO-d₆), deuterated chloroform (CDCl₃), and deuterated methanol (CD₃OD), and the internal standard is tetramethylsilane (TMS);
MS is determined with Agilent 6120B (ESI) and Agilent 6120B (APCI); and
HPLC is determined with Agilent 1260DAD high pressure liquid chromatograph (Zorbax SB-C18 100 × 4.6 mm, 3.5 µM);
Yantai Huanghai HSGF₂₅₄ or Qingdao GF₂₅₄ silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm-0.5 mm;
and for the column chromatography, Yantai Huanghai silica gel of 200-300 mesh is generally used as a carrier.
RuPhos-Pd-G3: catalyst with CAS No. 1445085-77-7.

### Example 1

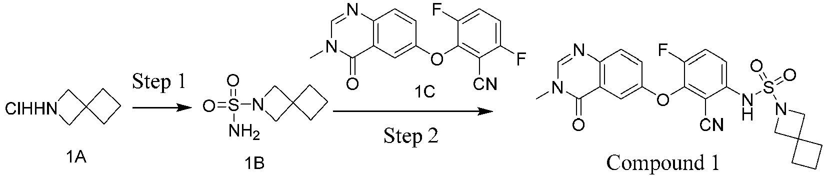

Step 1: 1A (800 mg, 5.99 mmol), triethylamine (1.82 g, 17.97 mmol) and dichloromethane (10 mL) were added in sequence into a 50 mL reaction flask. After the addition, the obtained mixture was stirred at 0°C for 20 min, a solution of sulfamoyl chloride (692 mg, 5.99 mmol) in dichloromethane (7 mL) was added dropwise slowly and the obtained mixture was reacted under stirring at room temperature for 1 h. The reaction solution was diluted with dichloromethane (50 mL), washed with water (30 mL × 1) and saturated brine (30 mL × 1) in sequence, the organic layer was dried over anhydrous sodium sulphate and filtered, and the filtrate was concentrated under reduced pressure, and the residue was separated and purified by column chromatography (DCM/MeOH = 15/1) to give 1B (180 mg, yield 17%).

Step 2: 1C (250 mg, 0.8 mmol, the preparation method refers to WO 2021116050A1), 1B (160 mg, 0.9 mmol), caesium carbonate (310 mg, 0.96 mmol) and N,N-dimethyl formamide (10 mL) were added in sequence into a 50 mL reaction flask. After the addition, the obtained mixture was reacted under stirring at 80°C for 18 h. Ethyl acetate (50 mL) was added to the reaction solution, and then washing was performed with water (40 mL × 2). The organic layer was dried with anhydrous sodium sulphate and filtered, and the filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-70% acetonitrile; cycle time: 15 min) to obtain compound 1 (86 mg, yield 23%).

LCMS m/z = 470.5 [M+H]⁺;
¹H NMR (400 MHz, DMSO-*d₆*) δ 10.36 (s, 1H), 8.36 (s, 1H), 7.90 - 7.84 (m, 1H), 7.78 (d, 1H), 7.68 (dd, 1H), 7.53 (dd, 1H), 7.39 (d, 1H), 3.84 (s, 4H), 3.48 (s, 3H), 2.10 (t, 4H), 1.78 - 1.69 (m, 2H).

### Example 2

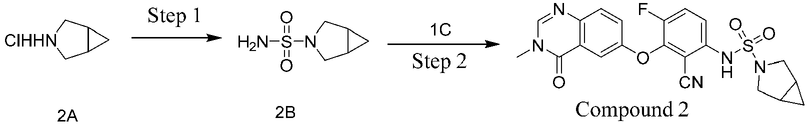

Step 1: 2A (520 mg, 4.34 mmol), triethylamine (2.19 g, 21.64 mmol) and dichloromethane (10 mL) were added in sequence into a 50 mL reaction flask. After the addition, the obtained mixture was stirred at 0°C for 20 min, a solution of sulfamoyl chloride (500 mg, 4.34 mmol) in dichloromethane (7 mL) was added dropwise slowly and the obtained mixture was reacted under stirring at room temperature for 1 h. The reaction solution was diluted with dichloromethane (50 mL), washed with water (50 mL × 1) and saturated brine (50 mL × 1) in sequence, the organic layer was dried over anhydrous sodium sulphate and filtered, and the filtrate was concentrated under reduced pressure, and the residue was separated and purified by column chromatography (DCM/MeOH = 15/1) to give 2B (450 mg, yield 32%).

Step 2: With reference to the reaction operation in step 2 of Example 1, compound 2 (60 mg, yield 16%) was obtained by separation and purification.

LCMS m/z = 456.2 [M+H]⁺;
¹H NMR (400 MHz, DMSO-*d₆*) δ 10.26 (s, 1H), 8.35 (s, 1H), 7.89-7.84 (m, 1H), 7.78 (d, 1H), 7.67 (dd, 1H), 7.50 (dd, 1H), 7.39 (d, 1H), 3.47 (s, 3H), 3.37-3.34 (m, 4H), 1.59-1.56 (m, 2H), 0.65-0.59 (m,1H), 0.19-0.16 (m, 1H).

### Example 3

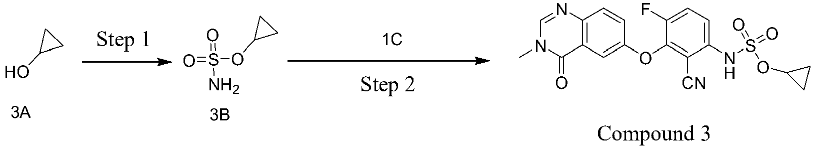

Step 1: Sulfamoyl chloride (1.0g, 8.68 mmol), triethylamine (2.63 g, 26.04) and dichloromethane (10 mL) were added in sequence into a 50 mL reaction flask. After the addition, the obtained mixture was stirred at 0°C for 20 min. Cyclopropanol (1.11g, 17.36 mmol) was slowly added dropwise. The obtained mixture was reacted under stirring at room temperature for 1 h. The reaction solution was concentrated under reduced pressure to obtain 3B, which was used directly in the next step without purification.

Step 2: With reference to the synthesis reaction in step 2 of Example 1, compound 3 (100 mg, yield 14.5%) was obtained by separation and purification.

LCMS m/z = 431.1 [M+H]⁺;
¹H NMR (400 MHz, CD₃OD) δ 8.26 (s, 1H), 7.77 (d, 1H), 7.67 - 7.54 (m, 3H), 7.48 (dd, 1H), 4.24 - 4.13 (m, 1H), 3.58 (s, 3H), 0.94 - 0.83 (m, 2H), 0.79 - 0.68 (m, 2H).

### Example 4

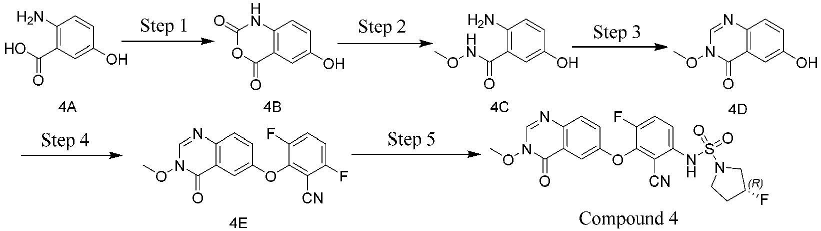

Step 1: 4A (4.0 g, 26.08 mmol) was dissolved in dry tetrahydrofuran (80 mL) in a 250 mL three-necked flask under nitrogen protection. Triphosgene (2.94 g, 9.91 mmol) was slowly added under an ice bath. After the addition, the obtained mixture was reacted under stirring at 70°C for 4 h. After the reaction was completed, the reaction solution was concentrated under reduced pressure. The residue was purified by slurrying with petroleum ether. The solid was filtered and dried to obtain 4B (3.86 g, yield 83%).

¹H NMR (400 MHz, DMSO-*d₆*) δ 11.49 (s, 1H), 9.84 (s, 1H), 7.24 - 7.18 (m, 2H), 7.03 (dd, 1H).

Step 2: 4B (3.85 g, 21.51 mmol) was dissolved in an aqueous solution of sodium hydroxide (1.03 g, 25.75 mmol) (60 mL) in a 250 mL single-neck flask. Methoxyamine hydrochloride (2.69 g, 32.27 mmol) was added. The obtained mixture was reacted under stirring at room temperature overnight. After the reaction was completed, the reaction solution was concentrated under reduced pressure. The residue was dissolved in a mixed solvent of dichloromethane (200 mL) and methanol (10 mL). The obtained mixture was dried over anhydrous sodium sulphate, and filtered through diatomaceous earth pad. The filtrate was concentrated under reduced pressure. The residue was separated and purified by column chromatography (DCM/MeOH = 20/1) to obtain 4C (2.8 g, yield 71%).

LCMS m/z = 183.1 [M+H]⁺;
Step 3: 4C (2.6 g,14.27 mmol) was dissolved in trimethyl orthoformate (54 mL) in a 50 mL single-neck flask. After the addition, the obtained mixture was reacted under stirring at 105°C for 4 h. After the reaction was completed, the reaction solution was concentrated under reduced pressure. The residue was purified by slurrying with petroleum ether. The solid was filtered and dried to obtain 4D (2.4 g, yield 88%).

LCMS m/z = 193.1 [M+H]⁺;
Step 4: 4D (2.4 g, 12.49 mmol) was dissolved in dry N,N-dimethylformamide (40 mL) in a 100 mL single-neck flask. Caesium carbonate (4.88 g, 14.99 mmol) was slowly added under an ice bath. After the addition, the obtained mixture was reacted under stirring at room temperature for 0.5 h. Under ice-bath conditions, a solution of 2,3,6-trifluorobenzonitrile (2.32 g, 14.99 mmol) in N,N-dimethylformamide (10 mL) was added slowly dropwise. After the addition was completed, the mixture was stirred and reacted at room temperature for 1 h. After the reaction was completed, the reaction solution was poured into ice water (250 mL). The obtained mixture was stirred for 30 min, and filtered. The obtained solid was dried and purified by slurrying with a mixed solvent of petroleum ether (100 mL) and ethyl acetate (10 mL). The solid was filtered and dried to obtain 4E (3.6 g, yield 88%).

LCMS m/z = 330.1 [M+H]⁺;
¹H NMR (400 MHz, DMSO-*d₆*) δ 8.64 (s, 1H), 7.98 (m, 1H), 7.83 (d, 1H), 7.75 (dd, 1H), 7.64 - 7.53 (m, 2H), 4.06 (s, 3H).

¹⁹F NMR (377 MHz, DMSO-*d₆*) δ -107.70 (s), -128.37 (s).

Step 5: With reference to the synthesis in step 2 of Example 1, compound 4 (32 mg, yield 7%) was obtained by separation and purification.

LCMS m/z = 478.1 [M+H]⁺;
¹H NMR (400 MHz, DMSO-*d₆*) δ 10.33 (s, 1H), 8.63 (s, 1H), 7.91 - 7.81 (m, 2H), 7.71 (dd, 1H), 7.56 (d, 1H), 7.41 (d, 1H), 5.40 (m, 0.5H), 5.27 (m, 0.5H), 4.05 (s, 3H), 3.58 - 3.32 (m, 4H), 2.17 (m, 2H).

¹⁹F NMR (377 MHz, DMSO-*d₆*) δ -127.22 (s), -172.76 (s).

### Example 5

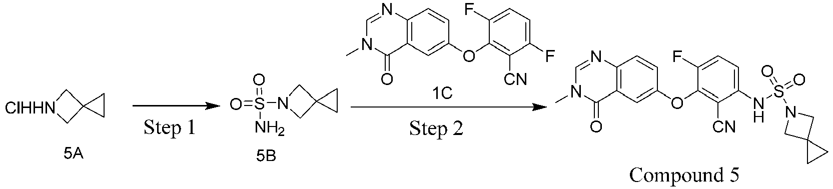

Step 1: 5A (720 mg, 6.04 mmol), triethylamine (1.83 g, 18.12 mmol) and dichloromethane (10 mL) were added in sequence into a 50 mL reaction flask. After the addition, the obtained mixture was stirred at 0°C for 20 min. A solution of sulfamoyl chloride (700 mg, 6.04 mmol) in dichloromethane (7 mL) was slowly added dropwise. The obtained mixture was reacted under stirring at room temperature for 1-2 h. The reaction solution was diluted with dichloromethane (100 mL), and washed with water (40 mL × 1) and saturated saline (40 mL × 1) in sequence. The organic layer was dried over anhydrous sodium sulphate and filtered. The filtrate was concentrated under reduced pressure. The residue was separated and purified by column chromatography (DCM/MeOH = 12/1) to obtain 5B (600 mg, yield 61%).

Step 2: With reference to the synthesis in step 2 of Example 1, compound 5 (12 mg, yield: 1%) was obtained by separation and purification.

LCMS m/z = 456.30 [M+H]⁺;
¹H NMR (400 MHz, DMSO-*d₆*) δ 8.33 (s, 1H), 7.76 (d, 1H), 7.63 (dd, 2H), 7.48 (dd, 1H), 7.38 (d, 1H), 7.08 (s, 1H), 3.83 (s, 4H), 3.47 (s, 3H), 0.56 (s, 4H).

¹⁹F NMR (377 MHz, DMSO-*d₆*) δ -134.36 (s).

### Example 6

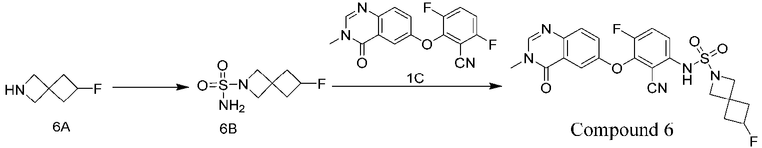

Step 1: 6A (synthesis method was referred to WO 2017/1660, 2017, A1) (620 mg, 5.38 mmol), triethylamine (1633.21 mg, 16.14 mmol) and dichloromethane (20 mL) were added in sequence into a 50 mL reaction flask. After the addition, the obtained mixture was stirred at 0°C for 20 min. A solution of sulfamoyl chloride (622 mg, 5.38 mmol) in dichloromethane (10 mL) was slowly added dropwise. The obtained mixture was reacted under stirring at room temperature for 1-2 h. The reaction solution was diluted with dichloromethane (100 mL), and washed with water (40 mL × 1) and saturated saline (40 mL × 1) in sequence. The organic layer was dried over anhydrous sodium sulphate and filtered. The filtrate was concentrated under reduced pressure. The residue was separated and purified by column chromatography (DCM/MeOH = 20/1) to obtain 6B (500 mg, yield 48%).

Step 2: With reference to the synthesis in step 2 of Example 1, compound 6 (8 mg, yield 1%) was obtained by separation and purification.

LCMS m/z = 488.20 [M+H]⁺;
¹H NMR (400 MHz, DMSO-*d₆*) δ 10.42 (s, 1H), 8.34 (s, 1H), 7.77 (d, 2H), 7.66 (dd, 1H), 7.47 (dd, 1H), 7.38 (d, 1H), 5.01 (s, 0.5H), 4.87 (s, 0.5H), 3.81 (d, 4H), 3.47 (s, 3H), 2.56 (m, 2H), 2.37 - 2.18 (m, 2H).

### Example 7

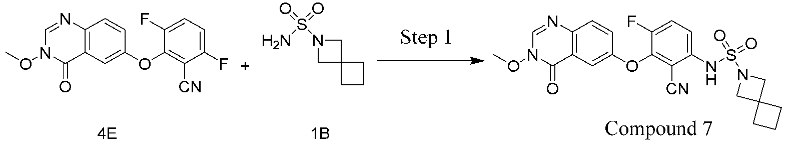

Step 1: With reference to the synthesis in step 2 of Example 1, compound 7 (58 mg, yield 8%) was obtained by separation and purification.

LCMS m/z = 486.1 [M+H]⁺;
¹H NMR (400 MHz, DMSO-*d₆*) δ 10.38 (s, 1H), 8.63 (s, 1H), 7.93 - 7.85 (t, 1H), 7.84 (d, 1H), 7.72 (dd, 1H), 7.54 (dd, 1H), 7.41 (d, 1H), 4.04 (s, 3H), 3.84 (s, 4H), 2.10 (t, 4H), 1.81 - 1.66 (m, 2H).

¹⁹F NMR (377 MHz, DMSO-*d₆*) δ -127.26 (s).

### Example 8

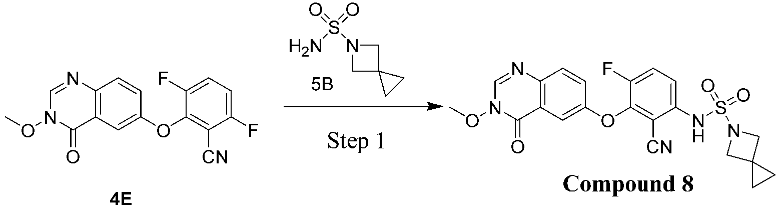

Step 1: With reference to the synthesis in step 2 of Example 1, compound 8 (0.3 g, yield 42%) was obtained by separation and purification.

LCMS m/z = 472.1 [M+H]⁺;
¹H NMR (400 MHz, DMSO-*d₆*) δ 10.48 (s, 1H), 8.63 (s, 1H), 7.93 - 7.86 (t, 1H), 7.84 (d, 1H), 7.72 (dd, 1H), 7.60 (dd, 1H), 7.43 (d, 1H), 4.05 (s, 3H), 3.98 (s, 4H), 0.62 (s, 4H).

¹⁹F NMR (377 MHz, DMSO-*d₆*) δ -127.15 (s).

### Example 9

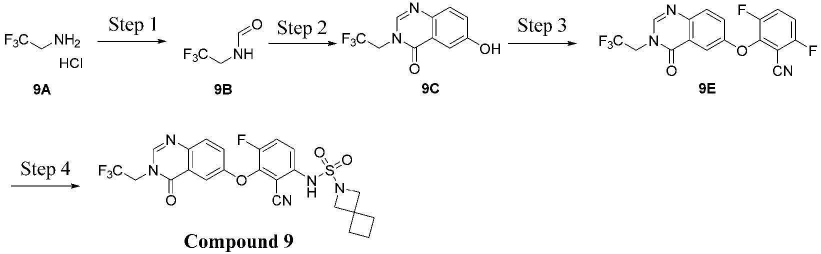

Step 1: 9A (25 g, 184.46 mmol) and sodium formate (15.55 g, 21.51 mmol) were added into a 250 mL single-neck flask. The obtained mixture was reacted at 130°C for 2 hrs. After the reaction was completed, the obtained mixture was cooled to room temperature and filtered. The filtrate was concentrated to obtain 9B (22.0 g, yield 94%).

LCMS m/z = 128.2 [M+H]⁺;
Step 2: 2-amino-5-hydroxybenzoic acid (1.0 g, 6.52 mmol) was added to 9B (6 mL, 36.2 mmol) in a 250 mL single-neck flask. The obtained mixture was reacted at 150°C for 21 hrs. After the reaction was completed, the obtained mixture was cooled to room temperature and filtered. The filter cake was washed twice with ethyl acetate (1 mL), and then the filter cake was concentrated to obtain 9C (1.3 g, yield 82%).

LCMS m/z = 245.2 [M+H]⁺;
Step 3: 9C (0.60 g, 2.46 mmol) was dissolved in dry N,N-dimethylformamide (6 mL) in a 25 mL single-neck flask. Caesium carbonate (1.6 g, 4.91 mmol) was slowly added under an ice bath. After the addition, the obtained mixture was reacted under stirring at room temperature for 0.5 h. 2,3,6-trifluorobenzonitrile (0.41 g, 2.61 mmol) was slowly added dropwise under an ice bath. After the addition, the obtained mixture was reacted under stirring at room temperature overnight. After the reaction was completed, ethyl acetate (10 mL) was added to the reaction system to dilute the reaction. Then water (20 mL) was added to quench the reaction, and extraction was performed with ethyl acetate (20 mL x 2). The organic phase was washed with water (20 mL x 2). The organic phase was concentrated and then purified by column chromatography (petroleum ether/ethyl acetate = 4/1) to obtain 9E (0.75 g, yield 80%).

LCMS m/z = 382.0 [M+H]⁺;
Step 4: With reference to the synthesis in step 2 of Example 1, separation and purification were carried out to obtain 100 mg of a solid. The solid was purified by column chromatography (petroleum ether/ethyl acetate = 5/1) to afford compound 9 (22 mg, yield 3%).

LCMS m/z = 538.1 [M+H]⁺;
¹H NMR (400 MHz, DMSO-*d₆*) δ 10.36 (s, 1H), 8.39 (s, 1H), 7.89-7.82 (dd, 2H), 7.75-7.72 (dd, 1H), 7.55-7.52 (dd, 1H), 7.44-7.43 (d, 1H), 4.98-4.91 (q, 2H), 3.83 (s, 4H), 2.11-2.07 (t, 4H), 1.77 - 1.70 (m, 2H).

¹⁹F NMR (377 MHz, DMSO-*d₆*) δ -127.30 (s), -67.08 (s).

### Example 10

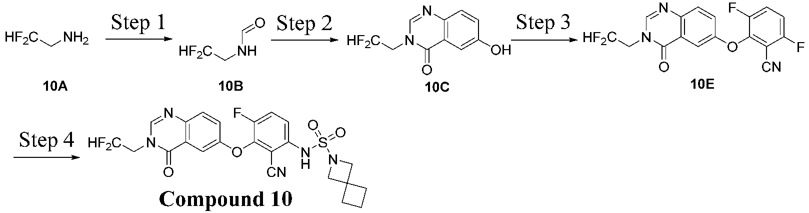

Step 1: 10A (20 g, 184.46 mmol) was added to ethyl formate (20 mL). The obtained mixture was reacted at 55°C overnight. After the reaction was completed, the reaction solution was cooled to room temperature and concentrated to obtain residue 10B (3.1 g, yield 12%).

LCMS m/z = 100.2 [M+H]⁺;
¹H NMR (400 MHz, CDCl₃) δ 8.23 (s, 1H), 6.32 (s, 1H), 6.01 - 5.67 (m, 1H), 3.71-3.60 (m, 2H).

Step 2: In a 25 mL single-neck flask, 2-amino-5-hydroxybenzoic acid (0.5 g, 3.31 mmol) was added to 10B (3.1 mL, 28.4 mmol), and the mixture was reacted at 150°C for 21 hours. After the reaction was completed, the obtained mixture was cooled to room temperature and filtered. The filter cake was washed with ethyl acetate (0.5 mL × 2), and then the filter cake was concentrated to obtain 10C (0.70 g, yield 94%).

LCMS m/z = 227.2 [M+H]⁺;
Step 3: 10C (0.70 g, 3.09 mmol) was dissolved in dry N,N-dimethylformamide (6 mL) in a 25 mL single-neck flask. Caesium carbonate (2.01 g, 6.17 mmol) was slowly added under an ice bath. After the addition, the obtained mixture was reacted under stirring at room temperature for 0.5 h. 2,3,6-trifluorobenzonitrile (0.51 g, 3.20 mmol) was slowly added dropwise under an ice bath. After the addition, the obtained mixture was reacted under stirring at room temperature overnight. After the reaction was completed, ethyl acetate (5 mL) was added to the reaction system to dilute the reaction, followed by the addition of water (20 mL) to quench the reaction. The mixture was extracted with ethyl acetate (20 mL × 2). The organic phases were washed with water (20 mL × 2). After concentration, the organic phase was purified by column chromatography (petroleum ether/ethyl acetate (v/v) = 4/1) to afford 10E (1.1 g, yield 98%).

LCMS m/z = 364.2 [M+H]⁺;
Step 4: In a 25 mL single-neck flask, 10E (0.50 g, 1.38 mmol) was dissolved in dry N,N-dimethylformamide (5 mL). Under ice-bath conditions, caesium carbonate (0.90 g, 2.76 mmol) and 1B (0.24 g, 1.38 mmol) were slowly added. After the addition was completed, the mixture was stirred at 100°C for 4 hours. After the reaction was completed, the reaction was filtered, and the filtrate was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-70% acetonitrile; cycle time: 15 min) to obtain compound 10 (150 mg, yield 21%).

LCMS m/z = 520.0 [M+H]⁺;
¹H NMR (400 MHz, DMSO-*d6*) δ 10.37 (s, 1H), 8.34 (s, 1H), 7.87-7.80 (m, 2H), 7.74-7.71 (dd, 1H), 7.54-7.50 (dd, 1H), 7.41-7.40 (d, 1H), 6.51 - 6.22 (m, 1H), 4.51-4.43 (m, 2H), 3.82 (s, 4H), 2.11-2.07 (t, 4H), 1.78 - 1.70 (m, 2H).

¹⁹F NMR (377 MHz, DMSO-*d6*) δ -127.22 (s), -120.46 (s).

### Example 11

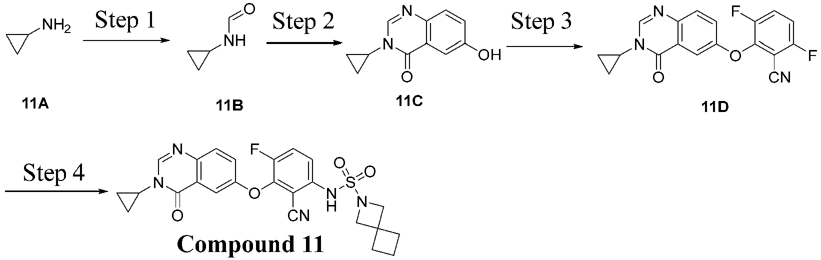

Step 1: 11A (5.0 g, 87.62 mmol) and ethyl formate (40 mL) were added in sequence into a 100 mL reaction flask. After the addition, the obtained mixture was stirred at 55°C for 12 h. After the reaction was completed, concentration was performed under reduced pressure to obtain 11B (7.0 g, yield 93%).

LCMS m/z = 86.20 [M+H]⁺;
Step 2: 11B (7.0 g, 82.26 mmol) and 4A (1.5 g, 9.83 mmol) were added in sequence into a 100 mL reaction flask. After the addition, the obtained mixture was stirred at 145°C for 10 h. After the reaction was completed, filtration was performed. The obtained solid was purified by slurrying with ethyl acetate. The solid was filtered and dried to obtain 11C (1.5 g, yield 75%).

LCMS m/z = 203.10 [M+H]⁺;
Step 3: 11C (1.5 g, 7.42 mmol) was dissolved in dry N,N-dimethylformamide (20 mL) in a 100 mL reaction flask. Caesium carbonate (3.63 g, 11.13 mmol) was added under an ice bath. After the addition, the obtained mixture was reacted at room temperature for 0.5 h. A solution of 2,3,6-trifluorobenzonitrile (1.28 g, 8.16 mmol) in N,N-dimethylformamide (15 mL) was slowly added dropwise under an ice bath. After the addition, the obtained mixture was reacted at room temperature for 2 h. The reaction solution was diluted with ethyl acetate (50 mL), and washed with water (50 mL × 1) and saturated saline (50 mL × 1) in sequence. The organic layer was dried over anhydrous sodium sulphate, filtered, and concentrated under reduced pressure. The residue was separated and purified by column chromatography (PE/EA = 2/1) to obtain 11D (1.4 g, yield 56%).

LCMS m/z = 340.10 [M+H]⁺;
Step 4: 11D (0.2 g, 0.59 mmol), 1B (0.2 g, 1.12 mmol), caesium carbonate (0.38 g, 1.18 mmol) and N,N-dimethylformamide (10 mL) were added in sequence into a 100 mL reaction flask. After the addition, the obtained mixture was reacted under stirring at 100°C for 12 h. Ethyl acetate (30 mL) was added to the reaction solution. Then washing was performed with water (30 mL × 2). The organic layer was dried over anhydrous sodium sulphate and filtered. The filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-70% acetonitrile; cycle time: 15 min) to obtain compound 11 (150 mg, yield 52%).

LCMS m/z = 496.20 [M+H]⁺;
¹H NMR (400 MHz, DMSO-*d₆*) δ 10.37 (s, 1H), 8.27 (s, 1H), 7.91-7.85 (m, 1H), 7.76 (d, 1H), 7.67 (dd, 1H), 7.53 (dd, 1H), 7.37 (d, 1H), 3.84 (s, 4H), 3.28-3.14 (m, 1H), 2.13-2.06 (m, 4H), 1.79-1.70 (m, 2H), 1.06-0.90 (m, 4H).

### Example 12

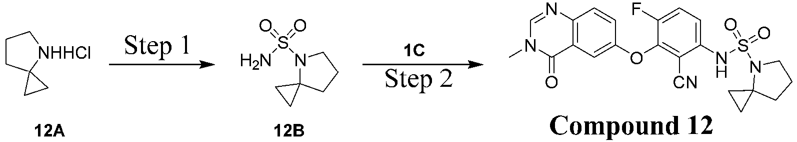

Step 1: 12A (100 mg, 0.75 mmol), triethylamine (150 mg, 1.50 mmol) and dichloromethane (10 mL) were added in sequence into a 50 mL reaction flask. After the addition, the obtained mixture was stirred at 0°C for 20 min. A solution of sulfamoyl chloride (92 mg, 0.80 mmol) in dichloromethane (5 mL) was slowly added dropwise. The obtained mixture was reacted under stirring at room temperature for 2 h. The reaction solution was diluted with dichloromethane (40 mL), and washed with water (30 mL × 1) and saturated saline (30 mL × 1) in sequence. The organic layer was dried over anhydrous sodium sulphate and filtered. The filtrate was concentrated under reduced pressure. The residue was separated and purified by column chromatography (DCM/MeOH = 15/1) to obtain 12B (80 mg, yield 55%).

Step 2: 1C (100 mg, 0.32 mmol), 12B (80 mg, 0.45 mmol), caesium carbonate (200 mg, 0.61 mmol) and N,N-dimethylformamide (10 mL) were added in sequence into a 50 mL reaction flask. After the addition, the obtained mixture was reacted under stirring at 100°C for 12 h. Ethyl acetate (40 mL) was added to the reaction solution. Then washing was performed with water (40 mL × 2). The organic layer was dried over anhydrous sodium sulphate and filtered. The filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-70% acetonitrile; cycle time: 15 min) to obtain compound 12 (35 mg, yield 23%).

LCMS m/z = 470.50 [M+H]⁺;
¹H NMR (400 MHz, DMSO-*d₆*) δ 10.23 (s, 1H), 8.35 (s, 1H), 7.92-7.84 (m, 1H), 7.78 (d, 1H), 7.68 (dd, 1H), 7.54 (dd, 1H), 7.36 (d, 1H), 3.56-3.50 (m, 2H), 3.47 (s, 3H), 1.94-1.84 (m, 2H), 1.80-1.72 (m, 2H), 1.08-0.98 (m, 2H), 0.57-0.51 (m, 2H).

### Example 13

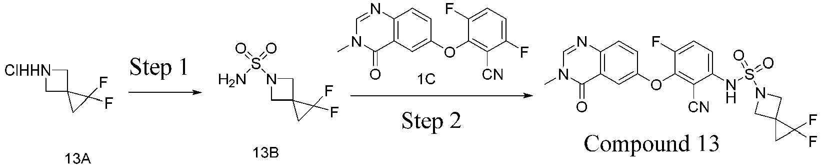

Step 1: 13A (500 mg, 3.21 mmol), triethylamine (0.97 g, 9.59 mmol) and dichloromethane (10 mL) were added in sequence into a 50 mL reaction flask. After the addition, the obtained mixture was stirred at 0°C for 20 min. A solution of sulfamoyl chloride (740 mg, 6.40 mmol) in dichloromethane (7 mL) was slowly added dropwise. The obtained mixture was reacted under stirring at room temperature for 12 h. The reaction solution was concentrated under reduced pressure to obtain 13B (500 mg, crude product).

Step 2: 1C (1.58 g, 5.04 mmol), 13B (500 mg, 2.52 mmol), caesium carbonate (2.46 g, 7.62 mmol) and N,N-dimethylformamide (15 mL) were added in sequence into a 50 mL reaction flask. After the addition, the obtained mixture was reacted under stirring at 100°C for 12 h. The reaction solution was filtered. The filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-70% acetonitrile; cycle time: 15 min) to obtain compound 13 (46.76 mg, yield 3.78%).

LCMS m/z = 492.4 [M+H]⁺;
¹H NMR (400 MHz, CDCl₃) δ 8.30 (s, 1H), 7.83 (dd, 1H), 7.63-7.58 (m, 3H), 7.47 (t, 1H), 6.86 (s, 1H), 4.20-4.14 (m, 4H), 3.63 (s, 3H), 1.52 (t, 2H).

### Example 14

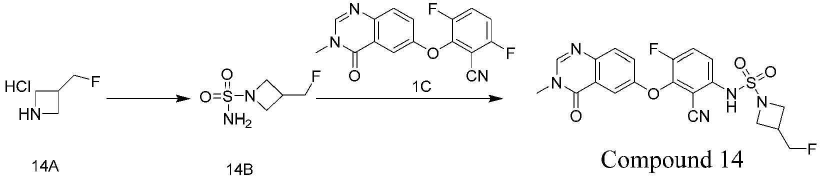

Step 1: Sulfamoyl chloride (0.18g, 1.59 mmol), triethylamine (0.48 g, 4.74 mmol) and dichloromethane (5 mL) were added in sequence into a 50 mL reaction flask. After the addition, the obtained mixture was stirred at 0°C for 20 min. 14A (0.20g, 1.59 mmol) was slowly added dropwise. The obtained mixture was reacted under stirring at room temperature for 1 h. The reaction solution was concentrated under reduced pressure, and the crude product was used directly in the next step without purification.

Step 2: 1C (490 mg, 1.56 mmol), 14B (0.30 g crude product), caesium carbonate (1.52 mg, 4.67 mmol) and N,N-dimethylformamide (5 mL) were added in sequence into a 50 mL reaction flask. After the addition, the obtained mixture was reacted under stirring at 100°C for 18 h. Ethyl acetate (50 mL) was added to the reaction solution. Then washing was performed with water (40 mL × 2). The organic layer was dried over anhydrous sodium sulphate and filtered. The filtrate was concentrated under reduced pressure. The residue was separated and purified by medium-pressure preparative chromatography (instrument: Biotage Isolera One; chromatographic column: C18 spherical 20-35um 100A 80g (Agela Technologies); composition of mobile phase: mobile phase A: acetonitrile; mobile phase B: water; gradient: isocratic elution using 5%-90% acetonitrile; cycle time: 20 min; retention time: 7 min) to obtain compound 14 (28 mg, yield 3.89%).

LCMS m/z = 462.1 [M+H]⁺;
¹H NMR (400 MHz, CD₃OD) δ 8.22 (s, 1H), 7.75 - 7.70 (m, 1H), 7.57 (dd, 2H), 7.47 (dd, 1H), 7.33 - 7.25 (m, 1H), 4.59 (d, 1H), 4.47 (d, 1H), 3.88 (t, 2H), 3.64 (dd, 2H), 3.57 (s, 3H),2.88 - 2.76 (m, 1H).

¹⁹F NMR (377 MHz, DMSO-*d₆*) δ -146.12 (s), -220.65 (s).

### Example 15

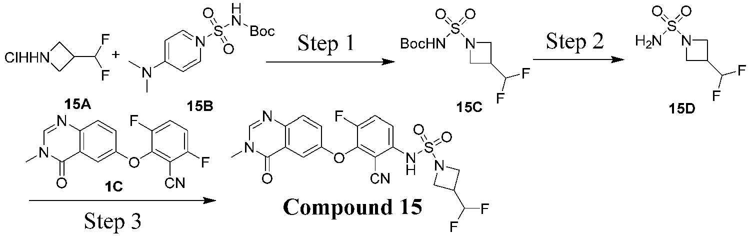

Step 1: 15A (0.25 g, 1.74 mmol) was dissolved in dry acetonitrile (15 mL) in a 100 mL single-neck flask. 15B (0.53 g, 1.74 mmol) was added. After the addition, the obtained mixture was reacted under stirring at 30°C for 1 h. After the reaction was completed, the reaction solution was concentrated and the obtained solid was the crude compound 15C, which was directly used in the next reaction without purification.

Step 2: Compound 15C (crude product) and dichloromethane (12 ml) were added into a single-neck flask. Trifluoroacetic acid (3 ml) was added. The obtained mixture was reacted at room temperature for 2 h. Concentration was performed to obtain compound 15D, which was directly used in the next reaction without purification.

Step 3: 15D (crude product) was dissolved in dry N,N-dimethylformamide (10 mL) in a 100 mL single-neck flask. Caesium carbonate (2.27 g, 6.97 mmol) and 1C (300 mg, 0.96 mmol) were slowly added under an ice bath. After the addition, the obtained mixture was reacted under stirring at 100°C for 6 h. After the reaction was completed, the reaction solution was filtered. The filtrate was concentrated to obtain an oily liquid. The residue was separated and purified by medium-pressure preparative chromatography (instrument: Biotage Isolera One; chromatographic column: C18 spherical 20-35um 100A 80g (Agela Technologies); composition of mobile phase: mobile phase A: acetonitrile; mobile phase B: water; gradient: isocratic elution using 5%-90% acetonitrile; cycle time: 20 min; retention time: 7 min) to obtain compound 15 (120 mg, total yield over three steps: 14.4%).

LCMS m/z = 480.4 [M+H]⁺;
¹H NMR (400 MHz, CD₃OD) δ 8.25 (s, 1H), 7.77 - 7.72 (m, 1H), 7.59 (dd, 2H), 7.46 (dd, 1H), 7.30 (t, 1H), 6.11 (td, 1H), 3.91 (t, 2H), 3.82 - 3.75 (m, 2H), 3.59 (s, 3H), 2.98 - 2.84 (m, 1H).

¹⁹F NMR (377 MHz, DMSO-*d₆*) δ -121.73 (s), -145.77 (s).

### Example 16

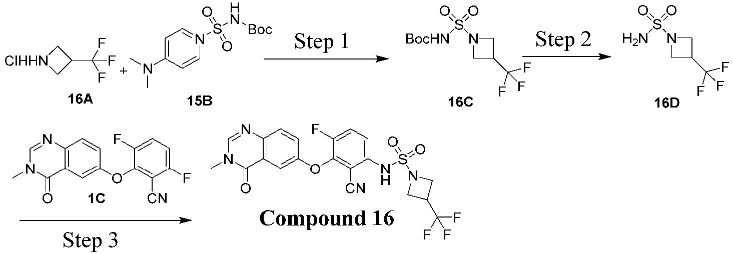

Step 1: 3-(trifluoromethyl)azetidine hydrochloride (16A) (0.25 g, 1.55 mmol) was dissolved in dry acetonitrile (15 mL) in a 100 mL single-neck flask. 15B (0.47 g, 1.55 mmol) was added. After the addition, the obtained mixture was reacted under stirring at 30°C for 1 h. After the reaction was completed, the reaction solution was concentrated and the obtained solid was the crude compound 16C, which was directly used in the next reaction without purification.

Step 2: Compound 16C (crude product) and dichloromethane (12 ml) were added into a single-neck flask. Trifluoroacetic acid (3 ml) was added. The obtained mixture was reacted at room temperature for 2 h. Concentration was performed to obtain compound 16D, which was directly used in the next reaction without purification.

Step 3: 16D (crude product) was dissolved in dry N,N-dimethylformamide (10 mL) in a 100 mL single-neck flask. Caesium carbonate (2.02 g, 6.20 mmol) and 1C (300 mg, 0.96 mmol) were slowly added under an ice bath. After the addition, the obtained mixture was reacted under stirring at 100°C for 6 h. After the reaction was completed, the reaction solution was filtered. The filtrate was concentrated to obtain an oily liquid. The residue was separated and purified by medium-pressure preparative chromatography (instrument: Biotage Isolera One; chromatographic column: C18 spherical 20-35um 100A 80g (Agela Technologies); composition of mobile phase: mobile phase A: acetonitrile; mobile phase B: water; gradient: isocratic elution using 5%-90% acetonitrile; cycle time: 20 min; retention time: 8 min) to obtain compound 16 (100 mg, total yield over three steps: 12.9%).

LCMS m/z = 498.5 [M+H]⁺;
¹H NMR (400 MHz, CD₃OD) δ 8.23 (s, 1H), 7.75 - 7.70 (m, 1H), 7.57 (dd, 2H), 7.45 (dd, 1H), 7.34 - 7.26 (m, 1H), 3.96 (t, 2H), 3.89 - 3.82 (m, 2H), 3.57 (s, 3H), 3.33 - 3.24 (m, 1H).

¹⁹F NMR (377 MHz, DMSO-*d₆*) δ -70.81 (s), -145.19 (s).

### Example 17

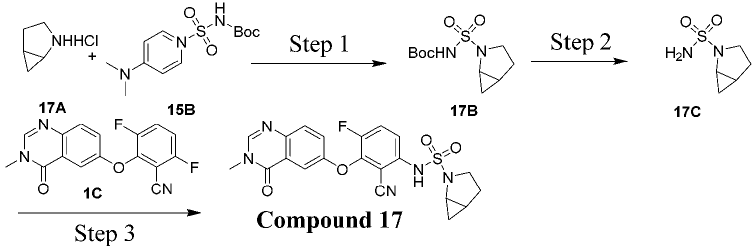

Step 1: 2-azabicyclo[3.1.0]hexane hydrochloride (17A) (0.47 g, 3.97 mmol) was dissolved in dry acetonitrile (25 mL) in a 100 mL single-neck flask. 15B (1.2 g, 3.97 mmol) was added. After the addition, the obtained mixture was reacted under stirring at 70°C for 4 h. After the reaction was completed, the reaction solution was concentrated and the obtained solid was the crude compound 17B, which was directly used in the next reaction without purification.

Step 2: Compound 17B (680 mg, 2.59 mmol) and dichloromethane (12 ml) were added into a single-neck flask. Trifluoroacetic acid (3 ml) was added, and the obtained mixture was reacted at room temperature for 2 h. After the obtained mixture was concentrated, the residue was separated by column chromatography (petroleum ether: ethyl acetate (v/v) = 1 : 0 - 0 : 1) to obtain compound 17C (420 mg, yield: 99%).

Step 3: 17C (217 mg, 1.34 mmol) was dissolved in dry N,N-dimethylformamide (10 mL) in a 100 mL single-neck flask. Caesium carbonate (655 mg, 2.01 mmol) was slowly added under an ice bath. After the addition, the obtained mixture was reacted under stirring at 50°C for 0.5 h. A solution of 1C (420 mg, 1.34 mmol) in N,N-dimethylformamide (5 mL) was slowly added dropwise under an ice bath. After the addition, the obtained mixture was reacted under stirring at 100°C for 2 h. After the reaction was completed, the reaction solution was filtered. The filtrate was concentrated to obtain an oily liquid, which was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-80% acetonitrile; cycle time: 15 min) to obtain compound 17 (56 mg, yield: 9%).

LCMS m/z = 456.5 [M+H]⁺;
¹H NMR (400 MHz, DMSO-*d₆*) δ 10.39 (s, 1H), 8.36 (s, 1H), 7.86 (t, 1H), 7.78 (d, 1H), 7.68 (dd, 1H), 7.63 (dd, 1H), 7.38 (d, 1H), 3.48 (s, 3H), 3.39 (t, 1H), 3.18 (m, 1H), 2.93 (m, 1H), 2.05 (m, 1H), 1.98 (m, 1H), 1.61 (m, 1H), 0.78 (m, 1H), 0.52 (m, 1H).

¹⁹F NMR (377 MHz, DMSO-*d₆*) δ -128.22 (s).

### Example 18

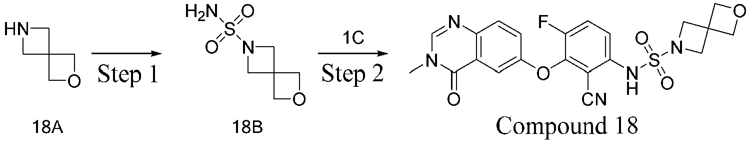

Step 1: 18A (500 mg, 5.08 mmol), triethylamine (1.54 g, 15.24 mmol) and dichloromethane (10 mL) were added in sequence into a 50 mL reaction flask. After the addition, the obtained mixture was stirred at 0°C for 20 min. A solution of sulfamoyl chloride (1.17 g, 10.16 mmol) in dichloromethane (7 mL) was slowly added dropwise. The obtained mixture was reacted under stirring at room temperature for 12 h. The reaction solution was concentrated under reduced pressure to obtain 18B (500 mg, crude product).

Step 2: 1C (1.76 g, 5.62 mmol), 18B (500 mg, 2.81 mmol), caesium carbonate (2.75 g, 8.37 mmol) and N,N-dimethylformamide (15 mL) were added in sequence into a 50 mL reaction flask. After the addition, the obtained mixture was reacted under stirring at 100°C for 12 h. The reaction solution was filtered. The filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-70% acetonitrile; cycle time: 15 min) to obtain compound 18 (22.34 mg, yield 1.7%).

LCMS m/z = 472.5 [M+H]⁺;
¹H NMR (400 MHz, CDCl₃) δ 8.31 (s, 1H), 7.84 (d, 1H), 7.62 (dd, 1H), 7.57 (d, 1H), 7.54 (dd, 1H), 7.45 (t, 1H), 6.95 (s, 1H), 4.78 (s, 4H), 4.18 (s, 4H), 3.63 (s, 3H).

### Example 19

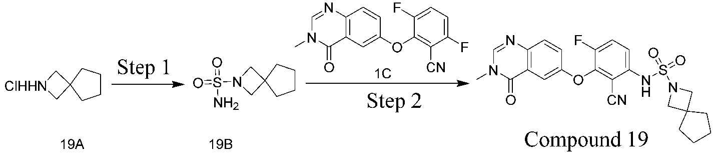

Step 1: 19A (500 mg, 4.49 mmol), triethylamine (1.36 g, 13.47 mmol) and dichloromethane (10 mL) were added in sequence into a 50 mL reaction flask. After the addition, the obtained mixture was stirred at 0°C for 20 min. A solution of sulfamoyl chloride (520 mg, 4.49 mmol) in dichloromethane (7 mL) was slowly added dropwise. The obtained mixture was reacted under stirring at room temperature for 1-2 h. The reaction solution was diluted with dichloromethane (100 mL), and washed with water (40 mL × 1) and saturated saline (40 mL × 1) in sequence. The organic layer was dried over anhydrous sodium sulphate and filtered. The filtrate was concentrated under reduced pressure. The residue was separated and purified by column chromatography (DCM/MeOH = 12/1) to obtain 19B (600 mg, yield 70%).

Step 2: 1C (0.9 g, 2.87 mmol), 19B (0.6 g, 3.16 mmol), caesium carbonate (1.12 g, 3.44 mmol) and N,N-dimethylformamide (20 mL) were added in sequence into a 100 mL reaction flask. After the addition, the obtained mixture was reacted under stirring at 80°C for 18 h. Ethyl acetate (100 mL) was added to the reaction solution. Then washing was performed with water (40 mL × 2). The organic layer was dried over anhydrous sodium sulphate and filtered. The filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-70% acetonitrile; cycle time: 15 min) to obtain compound 19 (12 mg, yield 1%).

LCMS m/z = 484.60 [M+H]⁺;
¹H NMR (400 MHz, DMSO-*d₆*) δ 10.36 (s, 1H), 8.36 (s, 1H), 7.88 (t, 1H), 7.78 (d, 1H), 7.68 (dd, 1H), 7.56 (dd, 1H), 7.39 (d, 1H), 3.72 (s, 4H), 3.48 (s, 3H), 1.71 (m, 4H), 1.50 (m, 4H).

¹⁹F NMR (377 MHz, DMSO-*d₆*) δ -127.31 (s).

### Example 20 and example 21

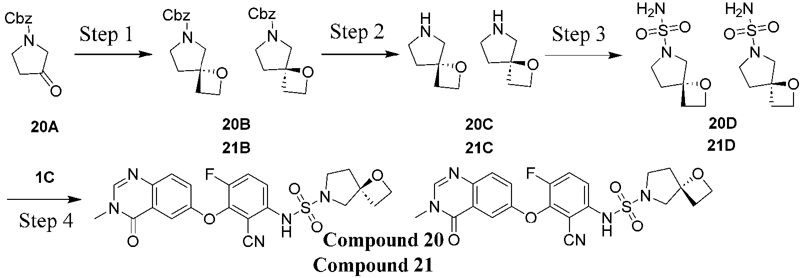

Step 1: A 500 mL reaction flask was taken, and trimethylsulphoxide iodide (100.45 g, 456.62 mmol) was dissolved in tert-butanol (350 mL) at room temperature. Then potassium tert-butoxide (45.0 g, 401.83 mmol) was added. The obtained mixture was reacted at 50°C for 1 h, and then cooled to room temperature. 20A (40.0 g, 182.65 mmol) was slowly added. After the addition, the obtained mixture was reacted at 50°C for 48 h. After the reaction was completed, saturated ammonium chloride solution (20 mL) was added to quench the reaction. Water (50 mL) was added. Extraction was performed with ethyl acetate (150 mL × 2). The combined organic phase was washed with saturated brine (50 mL × 1), dried over anhydrous sodium sulphate and filtered. After the filtrate was concentrated, the residue was separated and purified by silica gel column chromatography (petroleum ether: ethyl acetate (v/v)= 2 : 1) to obtain 10.0 g of a racemate, which was separated by chiral preparative HPLC to obtain peak 1 (3.0 g, retention time: 8.45 min, set to 20B) and peak 2 (3.0 g, retention time: 11.62 min, set to 21B), respectively. The preparative chromatographic separation conditions are as follows: instrument SFC Prep 150 AP; chromatographic column: Daicel AD-H (19 mm × 250 mm); mobile phase system: A for CO₂ and B for MeOH; gradient: B 10%; 5. Flow rate: 45 mL/min.

Step 2: A 100 mL reaction flask was taken, and compound 20B (3.0 g, 12.13 mmol) was dissolved in methanol (30 mL) at room temperature. Pd/C (0.3 g, Pd content 10%) was added. The obtained mixture was reacted under hydrogen atmosphere for 3 h. After the reaction was completed, filtration was performed, and the filtrate was concentrated to obtain a crude title compound 20C (1.1 g, 80%), which was used directly in the next step without purification.

LCMS m/z = 114.2 [M+H]⁺.

Step 3: A 50 mL reaction flask was taken, and compound 20C (1.1 g, 9.72 mmol) was dissolved in 1,4-dioxane (15 mL) at room temperature. Sulphonamide (0.78 g, 8.11 mmol) was added. The obtained mixture was reacted at 100°C for 12 h. After the reaction was completed, water (10 mL) was added, and the mixture was extracted with ethyl acetate (15 mL × 2). The combined organic phases were washed with a saturated sodium chloride solution (10 mL × 1), dried over anhydrous sodium sulphate, and filtered. The filtrate was concentrated, and the residue was separated and purified by silica gel column chromatography (petroleum ether: ethyl acetate (v/v) = 1 : 1) to afford the title compound 20D (1.3 g, 70%).

LCMS m/z = 193.1 [M+H]⁺.

Step 4: 1C (407.4 mg, 1.30 mmol), 20D (500 mg, 2.60 mmol), caesium carbonate (813.8 mg, 2.60 mmol) and N,N-dimethylformamide (10 mL) were added in sequence into a 50 mL reaction flask at room temperature. After the addition, the obtained mixture was reacted under stirring at 100°C for 12 h. After the reaction was completed, water (10 mL) was added, and ethyl acetate (30 mL×2) was used for extraction. The combined organic phase was washed with saturated brine (10 mL×1), dried over anhydrous sodium sulphate and filtered. The filtrate was concentrated. The residue was prepared by preparative liquid chromatography, HPLC separation method: 1. Instruments: waters 2767 preparative liquid chromatographic instrument; chromatographic column: SunFire@ Prep C18 (19 mm × 250 mm). 2. The sample was filtered with a 0.45 µm filter head to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min; d. elution time: 20 min. retention time 7.0 min, to obtain compound 20 (230 mg, 18%).

LCMS m/z = 486.1 [M+H]⁺.

¹H NMR (400 MHz, CDCl₃) δ 8.00 (s, 1H), 7.73 (d, 1H), 7.68 (s, 1H), 7.61 (d, 1H), 7.58 (dd, 1H), 7.52 (dd, 1H), 7.41 - 7.34 (m, 1H), 4.57 - 4.45 (m, 2H), 3.93 - 3.87 (m, 1H), 3.56 (s, 3H), 3.56 - 3.51 (m, 2H), 3.40 (d, 1H), 2.73 - 2.63 (m, 2H), 2.49 - 2.40 (m, 1H), 2.07 - 1.98 (m, 1H).

Compound 21 (200 mg) can be obtained by using 21B (3 g) as raw material through the same synthesis method as compound 20.

LCMS m/z = 486.1 [M+H]⁺.

¹H NMR (400 MHz, CDCl₃) δ 8.01 (s, 1H), 7.75 (s, 1H), 7.73 (d, 1H), 7.61 (d, 1H), 7.57 (dd, 1H), 7.51 (dd, 1H), 7.40 - 7.34 (m, 1H), 4.56 - 4.45 (m, 2H), 3.92 - 3.86 (m, 1H), 3.56 (s, 3H), 3.55 - 3.51 (m, 2H), 3.40 (d, 1H), 2.71 - 2.63 (m, 2H), 2.47 - 2.40 (m, 1H), 2.06 - 1.98 (m, 1H).

### Example 22

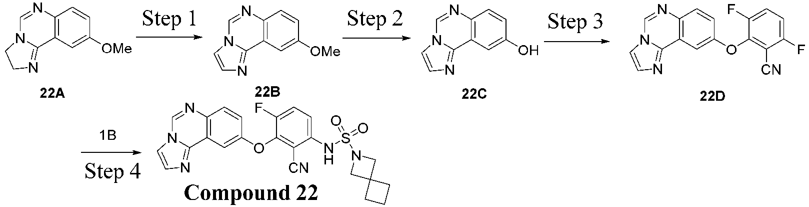

Step 1: 22A (0.8 g, 3.98 mmol) (synthesis of compound 22A was referred to patent WO2019/60611, 2019, A1) was dissolved in acetonitrile (20 mL) in a 100 mL reaction flask. Potassium permanganate (1.20 g, 7.61 mmol) and basic alumina (1.0 g, 9.79 mmol) were added in sequence. After the addition, the obtained mixture was reacted at room temperature for 5 h. After the reaction was completed, filtration was performed. The resulting filtrate was concentrated. The residue was separated and purified by column chromatography (PE/EA = 1/1) to obtain 22B (0.65 g, yield 82%).

LCMS m/z = 200.10 [M+H]⁺;
Step 2: 22B (0.6 g, 3.01 mmol) was dissolved in N, N-dimethylformamide (10 mL) in a 100 mL reaction flask. Then sodium thioethylate (0.50 g, 5.94 mmol) was added. After the reaction solution was subjected to nitrogen replacement three times, the reaction solution was heated to 130°C and reacted for 10 h. After the reaction was completed, filtration was performed. The obtained filtrate was concentrated to obtain 22C (0.45 g, yield 80%).

LCMS m/z = 186.10 [M+H]⁺;
Step 3: 22C (0.45 g, 2.43 mmol) was dissolved in N, N-dimethylformamide (10 mL) in a 100 mL reaction flask. Caesium carbonate (1.20 g, 3.75 mmol) was slowly added under an ice-water bath. After the addition, the obtained mixture was stirred at room temperature for 0.5 h. A solution of 2,3,6-trifluorobenzonitrile (0.38 g, 2.43 mmol) N,N-dimethylformamide (5 mL) was slowly added dropwise under an ice-water bath. After the addition, the obtained mixture was reacted at room temperature for 1 h. The reaction solution was diluted with ethyl acetate (50 mL), and washed with water (50 mL × 1) and saturated saline (50 mL × 1) in sequence. The organic layer was dried over anhydrous sodium sulphate, filtered, and concentrated under reduced pressure. The residue was separated and purified by column chromatography (PE/EA = 2/1) to obtain 22D (0.55 g, yield 70%).

LCMS m/z = 323.30 [M+H]⁺;
Step 4: 22D (0.25 g, 0.78 mmol), 1B (0.21 g, 1.17 mmol), caesium carbonate (0.51 g, 1.56 mmol) and N,N-dimethylformamide (15 mL) were added in sequence into a 100 mL reaction flask. After the addition, the obtained mixture was reacted under stirring at 100°C for 12 h. Ethyl acetate (30 mL) was added to the reaction solution. Then washing was performed with water (30 mL × 2). The organic layer was dried over anhydrous sodium sulphate and filtered. The filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-70% acetonitrile; cycle time: 10 min) to obtain compound 22 (150 mg, yield 40%).

LCMS m/z = 479.10 [M+H]⁺;
¹H NMR (400 MHz, DMSO-*d₆*) δ 10.38 (s, 1H), 9.27 (s, 1H), 8.09 (d, 1H), 8.01 (d, 1H), 7.87 (t, 1H), 7.62 (d, 1H), 7.59 (d, 1H), 7.58-7.53 (m, 2H), 3.83 (s, 4H), 2.08 (t, 4H), 1.75-1.66 (m, 2H).

### Example 23

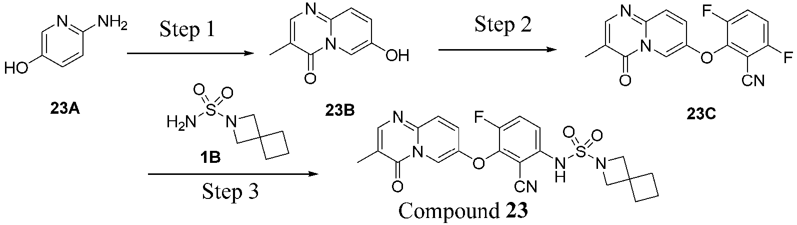

Step 1: 23A (1.00 g, 9.11 mmol) and acetic acid (15 mL) were added into a single-neck flask. Then ethyl 3-ethoxy-2-methyl-2-acrylate (2.88 g, 18.22 mmol) was added. The obtained mixture was heated to reflux and reacted for 16 h. After the reaction was completed as monitored by LCMS, the acetic acid was spun off, and the remaining solid was slurried with ethyl acetate (20 mL). The solid was filtered and dried to obtain product 23B(1.2 g, 74.7%).

LC-MS (ESI): m/z = 177.1 [M+H]⁺.

Step 2: 23B (0.20 g, 1.14 mmol) was dissolved in N,N-dimethylformamide (5 mL). Then caesium carbonate (0.74 g, 2.27 mmol) was added. 2,3,6-trifluorobenzonitrile (0.23 g, 1.48 mmol) was added under stirring at 0°C. After the addition, the temperature was slowly raised to room temperature for reaction for 2 h. After the reaction was completed as monitored by LCMS, the reaction solution was poured into water (15 mL), and a large amount of solid was precipitated. The solid was filtered and dried to obtain the product 23C (0.12 g, 33.6%).

LC-MS (ESI): m/z = 314.1 [M+H]⁺.

Step 3: 23C (120.0 mg, 0.38 mmol), 1B (87.0 mg, 0.49 mmol), caesium carbonate (250.0 mg, 0.76 mmol) and N,N-dimethylformamide (5 mL) were added in sequence into a single-neck flask. After the addition, the obtained mixture was reacted under stirring at 80°C for 18 h. After the reaction was completed as monitored by LCMS, ethyl acetate (50 mL) was added to the reaction solution. Then washing was performed with water (40 mL × 2). The organic layer was dried over anhydrous sodium sulphate and filtered. The filtrate was concentrated under reduced pressure. The residue was separated and purified by column chromatography (DCM/MeOH = 10/1) to obtain compound 23 (50.0 mg, 28.0%).

¹H NMR ((400 MHz, DMSO-*d₆*) δ 10.41 (s, 1H), 8.38 (d, 1H), 8.30 (d, 1H), 8.00 (dd, 1H), 7.86 (t, 1H), 7.78 (d, 1H), 7.53 (dd, 1H), 3.82 (s, 4H), 2.12 (s, 3H), 2.09 (t, 4H), 1.77-1.69 (m, 2H).

LC-MS (ESI): m/z = 470.1 [M+H]⁺.

### Example 24

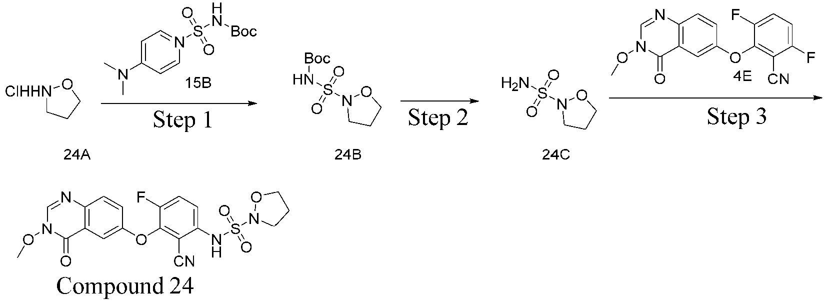

Step 1: 24A (1.2 g, 10.95 mmol), triethylamine (3.32 g, 32.85 mmol) and acetonitrile (20 mL) were added in sequence into a 50 mL reaction flask. After the addition, the obtained mixture was stirred for 30 min. 15B (4.97g, 16.43 mmol) was slowly added dropwise. The obtained mixture was reacted under stirring at 40°C for 3 h. The reaction solution was concentrated under reduced pressure to obtain 24B (1 g, crude product).

Step 2: 24B (0.8 g, 3.17 mmol) and dichloromethane (6 mL) were added in sequence into a 50 mL reaction flask. Trifluoroacetic acid (2 mL) was slowly added dropwise. The obtained mixture was reacted under stirring at room temperature for 1 h. The reaction solution was concentrated under reduced pressure to obtain 24C (0.8 g, crude product).

Step 3: 24C (0.8 g, 5.26 mmol), caesium carbonate (2.57 g, 7.89 mmol) and N,N-dimethylformamide (15 mL) were added in sequence into a 50 mL reaction flask. After the addition, the obtained mixture was reacted under stirring at 50°C for 2 h. Then 4E (2.08 g, 6.31 mmol) was added. The obtained mixture was reacted under stirring at 80°C for 12 h. The reaction solution was filtered, the filtrate was concentrated under reduced pressure, and the residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-70% acetonitrile; cycle time: 15 min) to obtain compound 24 (91.8 mg, yield 3.79%).

LCMS m/z = 462.1 [M+H]⁺;
¹H NMR (400 MHz, CDCl₃) δ 8.26 (s, 1H), 7.80 (d, 1H), 7.65-7.61 (m, 2H), 7.58 (dd, 1H), 7.44 (t, 1H), 4.24 (t, 2H), 4.15 (s, 3H), 3.73 (t, 2H), 2.47-2.40 (m, 2H).

### Example 25

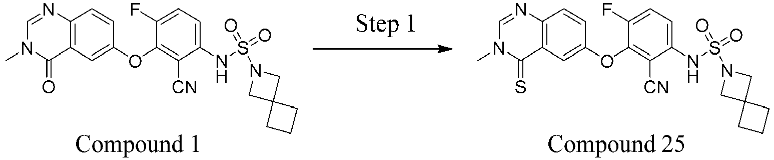

Step 1: Compound 1 (180 mg, 0.38 mmol), Lawesson's reagent (768 mg, 1.90 mmol) and dry tetrahydrofuran (9 mL) were added in sequence into a 25 mL reaction flask. After the addition, the obtained mixture was reacted under stirring at 80°C for 4 h. The reaction solution was directly concentrated under reduced pressure. The residue was separated and purified by column chromatography (DCM/MeOH = 15/1) to obtain a crude product. Compound 25 (35 mg, yield 19%) was obtained through separation and purification by preparative liquid chromatography.

LCMS m/z = 486.1 [M+H]⁺;
¹H NMR (400 MHz, DMSO-*d₆*) δ 10.41 (s, 1H), 8.70 (s, 1H), 7.99 (d, 1H), 7.89 (dd, 2H), 7.77 (dd, 1H), 7.56 (dd, 1H), 3.87 (s, 3H), 3.83 (s, 4H), 2.09 (t, 4H), 1.78 - 1.65 (m, 2H).

¹⁹F NMR (377 MHz, DMSO-*d₆*) δ -127.24 (s).

### Example 26

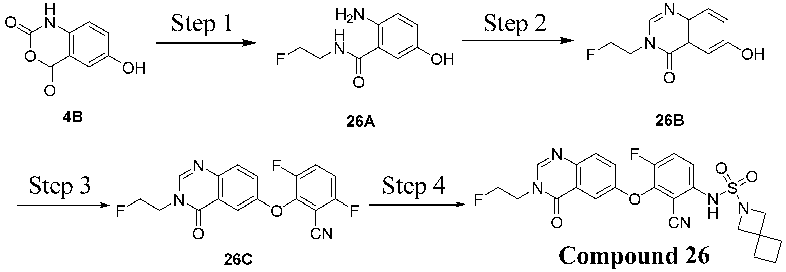

Step 1: 2-fluoroethylamine hydrochloride (9.0 g, 90.53 mmol) was dissolved in water (80 mL) in a 250 mL reaction flask. An aqueous solution of sodium hydroxide (3.3 g, 82.54 mmol) was added under an ice-water bath. After the obtained mixture was stirred for 30 min, 4B (4.0 g, 22.29 mmol) was added and the obtained mixture was stirred at room temperature for 4 h. After the reaction was completed, concentration was performed under reduced pressure. The residue was dissolved in a mixed solvent of dichloromethane (100 mL) and methanol (50 mL). After filtration, the filtrate was concentrated under reduced pressure. The residue was separated and purified by column chromatography (PE/EA = 1/1) to obtain 26A (3.8 g, yield 86%).

LCMS m/z = 199.10 [M+H]⁺;
Step 2: 26A (1.5 g, 8.58 mmol) was dissolved in triethyl orthoformate (10 mL) in a 20 mL microwave tube. The reaction solution was heated to 185°C and reacted for 2 h. After the reaction was completed, filtration was performed. The obtained filter cake was spin-dried to obtain 26B (1.6 g, yield 89%).

LCMS m/z = 209.10 [M+H]⁺;
Step 3: 26B (1.5 g, 7.20 mmol) was dissolved in N, N-dimethylformamide (20 mL) in a 100 mL reaction flask. Caesium carbonate (3.52 g, 10.80 mmol) was slowly added under an ice-water bath. After the obtained mixture was stirred at room temperature for 30 min, a solution of 2,3,6-trifluorobenzonitrile (1.36 g, 8.64 mmol) in N,N-dimethylformamide (5 mL) was slowly added dropwise under an ice-water bath. After the addition, the obtained mixture was reacted at room temperature for 2 h. The reaction solution was diluted with ethyl acetate (100 mL), and washed with water (100 mL × 1) and saturated saline (100 mL × 1) in sequence. The organic layer was dried over anhydrous sodium sulphate, filtered, and concentrated under reduced pressure. The residue was separated and purified by column chromatography (PE/EA = 2/1) to obtain 26C (1.35 g, yield 54%).

LCMS m/z = 346.10 [M+H]⁺;
Step 4: 26C (0.4 g, 1.16 mmol), 1B (0.31 g, 1.74 mmol), caesium carbonate (0.76 g, 2.32 mmol) and N,N-dimethylformamide (15 mL) were added in sequence into a 100 mL reaction flask. After the addition, the obtained mixture was reacted at 100°C for 12 h. Ethyl acetate (30 mL) was added to the reaction solution. Then washing was performed with water (30 mL × 2). The organic layer was dried over anhydrous sodium sulphate and filtered. The filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-70% acetonitrile; cycle time: 10 min) to obtain compound 26 (320 mg, yield 55%).

LCMS m/z = 502.10 [M+H]⁺;
¹H NMR (400 MHz, DMSO-*d₆*) δ 10.37 (s, 1H), 8.33 (s, 1H), 7.88 (t, 1H), 7.80 (d, 1H), 7.76-7.68 (m, 1H), 7.56-7.51 (m, 1H), 7.39 (d, 1H), 4.79-4.62 (m, 2H), 4.36-4.25 (m, 2H), 3.83 (s, 4H), 2.09 (t, 4H), 1.78-1.69 (m, 2H).

### Example 27

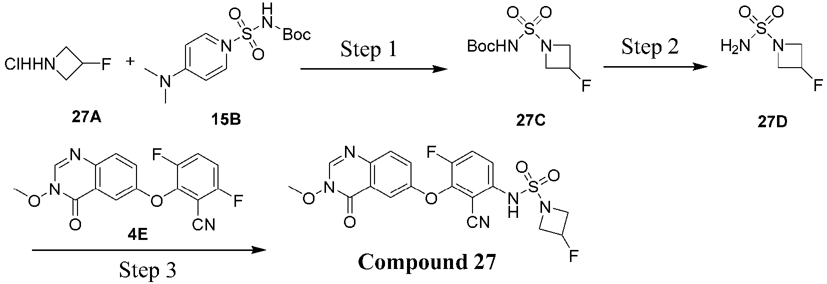

Step 1: 27A (1.0 g, 8.96 mmol) was dissolved in dry acetonitrile (15 mL) in a 100 mL single-neck flask. 15B (2.71 g, 8.96 mmol) and triethylamine (1.81 g, 17.96 mmol) were added. After the addition, the obtained mixture was reacted under stirring at 30°C for 1 h. After the reaction was completed, the reaction solution was concentrated and the obtained solid was the compound 27C, which was directly used in the next reaction without purification.

Step 2: Compound 27C and dichloromethane (12 ml) were added into a single-neck flask. Trifluoroacetic acid (6 ml) was added. The obtained mixture was reacted at room temperature for 2 h. After concentration, the crude product was purified by silica gel column chromatography (petroleum ether:ethyl acetate = 3 : 1) to obtain compound 27D (800 mg, total yield over two steps: 57.9%).

Step 3: 27D (0.45 g, 2.92 mmol) was dissolved in dry N,N-dimethylformamide (6 mL) in a 100 mL single-neck flask. Caesium carbonate (0.95 g, 2.92 mmol) and 4E (800 mg, 2.43 mmol) were slowly added. After the addition, the obtained mixture was reacted under stirring at 80°C for 6 h. After the reaction was completed, the reaction solution was filtered. The filtrate was concentrated to obtain an oily liquid. The residue was separated and purified by medium-pressure preparative chromatography (instrument: Biotage Isolera One; chromatographic column: C18 spherical 20-35um 100A 80g (Agela Technologies); composition of mobile phase: mobile phase A: acetonitrile; mobile phase B: water; gradient: isocratic elution using 5%-90% acetonitrile; cycle time: 20 min; retention time: 7.5 min) to obtain compound 27 (85 mg, yield 7.5%).

LCMS m/z = 464.1 [M+H]⁺;
¹H NMR (400 MHz, DMSO-*d₆*) δ 10.63 (s, 1H), 7.96 - 7.78 (m, 2H), 7.73 (dd, 1H), 7.56 (dd, 1H), 7.44 (d, 1H), 5.47-5.28 (m, 1H), 4.25-4.16 (m, 2H), 4.05 (s, 3H), 4.04 - 3.95 (m, 2H).

¹⁹F NMR (377 MHz, DMSO-*d₆*) δ -126.63 (s), -177.31 (s).

### Example 28

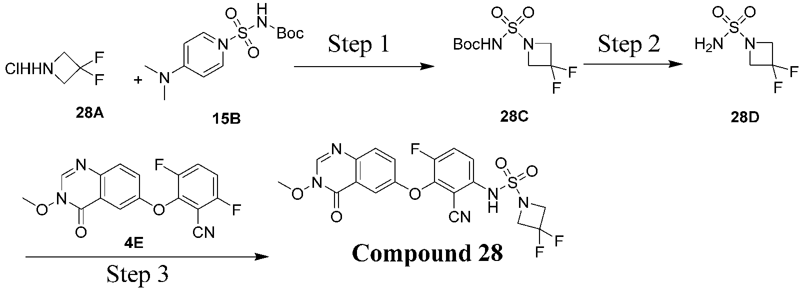

Step 1: 28A (1.0 g, 7.72 mmol) was dissolved in dry acetonitrile (15 mL) in a 100 mL single-neck flask. 15B (2.33 g, 7.72 mmol) and triethylamine (1.56 g, 15.44 mmol) were added. After the addition, the obtained mixture was reacted under stirring at 30°C for 1 h. After the reaction was completed, the reaction solution was concentrated and the obtained solid was the crude compound 28C, which was directly used in the next reaction without purification.

Step 2: Compound 28C (crude product) and dichloromethane (12 ml) were added into a single-neck flask. Trifluoroacetic acid (6 ml) was added. The obtained mixture was reacted at room temperature for 2 h. After concentration, the crude product was purified by silica gel column chromatography (petroleum ether : ethyl acetate = 3 : 1) to obtain compound 28D (670 mg, total yield over two steps: 56.30%).

Step 3: 28D (0.47 g, 2.74 mmol) was dissolved in dry N,N-dimethylformamide (6 mL) in a 100 mL single-neck flask. Caesium carbonate (0.89 g, 2.74 mmol) and 4E (750 mg, 2.28 mmol) were slowly added. After the addition, the obtained mixture was reacted under stirring at 80°C for 6 h. After the reaction was completed, the reaction solution was filtered. The filtrate was concentrated to obtain an oily liquid. The residue was separated and purified by medium-pressure preparative chromatography (instrument: Biotage Isolera One; chromatographic column: C18 spherical 20-35um 100A 80 g (Agela Technologies); composition of mobile phase: mobile phase A: acetonitrile; mobile phase B: water; gradient: isocratic elution using 5%-90% acetonitrile; cycle time: 20 min; retention time: 8 min) to obtain compound 28 (180 mg, yield 16.4%).

LCMS m/z = 482.1 [M+H]⁺;
¹H NMR (400 MHz, DMSO-*d₆*) δ 8.64 (s, 1H), 7.91 (dd, 1H), 7.84 (d, 1H), 7.73 (dd, 1H), 7.58 (dd, 1H), 7.45 (d, 1H), 4.41 (t, 4H), 4.05 (s, 3H).

¹⁹F NMR (377 MHz, DMSO-*d₆*) δ -90.00 (s), -126.12. (s).

### Example 29

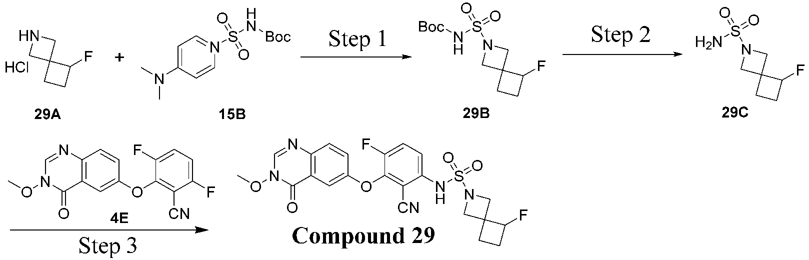

Step 1: Compound 29A (0.4 g, 2.64 mmol) was dissolved in acetonitrile (25 mL) in a 100 mL single-neck flask. Then 15B (1.10 g, 3.64 mmol) was added. After the addition, the obtained mixture was reacted at 40°C for 3 h. After the reaction was completed, the reaction solution was concentrated to obtain compound 29B (1.5 g, crude product), which was directly used in the next reaction.

Step 2: Compound 29B (1.5 g, 5.10 mmol) was dissolved in dichloromethane (30 mL) in a 100 mL single-neck flask. Then trifluoroacetic acid (10 mL) was added. The obtained mixture was reacted at room temperature for 2 h. After the reaction was completed, concentration was performed, and the residue was purified by column chromatography (PE/EA = 1/1) to obtain compound 29C (420 mg, yield 43%).

LCMS m/z = 502.10 [M+H]⁺;
Step 3: Compound 4E (0.5 g, 1.52 mmol) was dissolved in dry N,N-dimethylformamide (20 mL) in a 100 mL single-neck flask. After the addition of caesium carbonate (1.0 g, 3.07 mmol), the obtained mixture was reacted under stirring at 50°C for 0.5 h. Then a solution of 29C (0.42 g,2.16 mmol) in N,N-dimethylformamide (5 mL) was added dropwise. After the addition, the reaction solution was stirred at 100°C for 12 h. After the reaction was completed, filtration was performed. The filtrate was concentrated, and the obtained residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-70% acetonitrile; cycle time: 10 min) to obtain compound 29 (80 mg, yield 10%).

LCMS m/z = 504.10 [M+H]⁺;
¹H NMR (400 MHz, DMSO-*d₆*) δ 10.49 (s, 1H), 8.63 (s, 1H), 7.89 (t, 1H), 7.83 (d, 1H), 7.75-7.68 (m, 1H), 7.57-7.53 (m, 1H), 7.42 (d, 1H), 5.04-4.84 (m, 1H), 4.18 (d, 1H), 4.04 (s, 3H), 3.94 (d, 1H), 3.86-3.79 (m, 2H), 2.19-2.10 (m, 1H), 2.02-1.78 (m, 2H), 1.70-1.62 (m, 1H).

### Example 30

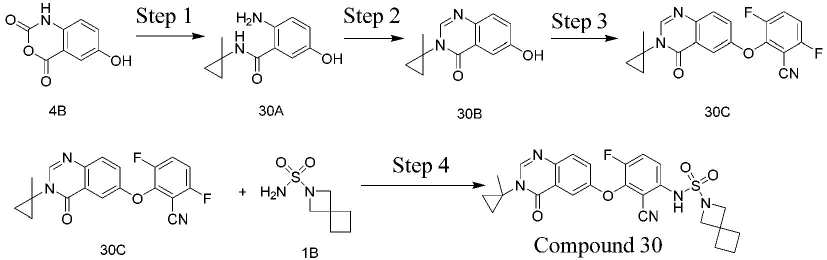

Step 1: 4B (4 g, 22.29 mmol) was dissolved in an aqueous solution of sodium hydroxide (2.85 g, 71.33 mmol) (40 mL) in a 250 mL single-neck flask. 1-methylcyclopropylamine hydrochloride (8.39 g, 78.02 mmol) was added. The obtained mixture was reacted under stirring at room temperature overnight. After the reaction was completed, the reaction solution was concentrated under reduced pressure. The residue was dissolved in a mixed solvent of dichloromethane (200 mL) and methanol (10 mL). The obtained mixture was dried over anhydrous sodium sulphate, and filtered through diatomaceous earth pad. The filtrate was concentrated under reduced pressure. The residue was separated and purified by column chromatography (DCM/MeOH = 20/1) to obtain 30A (4.0 g, yield 87%).

Step 2: 30A (4.0 g, 19.39 mmol) was dissolved in trimethyl orthoformate (32 mL) in a 50 mL single-neck flask. After the addition, the obtained mixture was reacted under stirring at 105°C for 4 h. After the reaction was completed, the reaction solution was concentrated under reduced pressure. The residue was purified by slurrying with petroleum ether. The solid was filtered and dried to obtain 30B (2.4 g, yield 57%).

LCMS m/z = 217.1 [M+H]⁺;
Step 3: 30B (2.0 g, 9.25 mmol) was dissolved in dry N,N-dimethylformamide (40 mL) in a 100 mL single-neck flask. Caesium carbonate (3.62 g, 11.1 mmol) was slowly added under an ice bath. After the addition, the obtained mixture was reacted under stirring at room temperature for 0.5 h. A solution of 2,3,6-trifluorobenzonitrile (1.45 g,9.25 mmol) in N,N-dimethylformamide (10 mL) was slowly added dropwise under an ice bath. After the addition, the obtained mixture was reacted under stirring at room temperature for 1 h. After the reaction was completed, the reaction solution was poured into ice water (250 mL). The obtained mixture was stirred for 30 min, and filtered. The obtained solid was dried and purified by slurrying with a mixed solvent of petroleum ether (100 mL) and ethyl acetate (10 mL). The solid was filtered and dried to obtain 30C (2.5 g, yield 76%).

LCMS m/z = 354.1 [M+H]⁺;
Step 4: Compound 1B (240 mg, 1.36 mmol) was dissolved in dry N,N-dimethylformamide (8 mL) in a 100 mL single-neck flask. Caesium carbonate (552 mg, 1.69 mmol) was slowly added under an ice bath. After the addition, the obtained mixture was reacted under stirring at 50°C for 0.5 h. A solution of compound 30C (400 mg, 1.13 mmol) in N,N-dimethylformamide (2 mL) was slowly added dropwise under an ice bath. After the addition, the obtained mixture was reacted under stirring at 100°C for 6 h. After the reaction was completed, the reaction solution was poured into ice water (50 mL). The obtained mixture was stirred for 30 min, and filtered. The obtained solid was dried and then separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-70% acetonitrile; cycle time: 15 min) to obtain compound 30 (240 mg, yield 42%).

LCMS m/z = 510.2 [M+H]⁺;
¹H NMR (400 MHz, DMSO-*d₆*) δ 10.38 (s, 1H), 8.36 (s, 1H), 7.93 - 7.84 (m, 1H), 7.77 (d, 1H), 7.68 (dd, 1H), 7.54 (dd, 1H), 7.35 (d, 1H), 3.84 (s, 4H), 2.10 (t, 4H), 1.75 (dd, 2H), 1.45 (s, 3H), 1.06 (t, 2H), 0.94 (t, 2H).

¹⁹F NMR (377 MHz, DMSO-*d₆*) δ -127.33 (s).

### Example 31

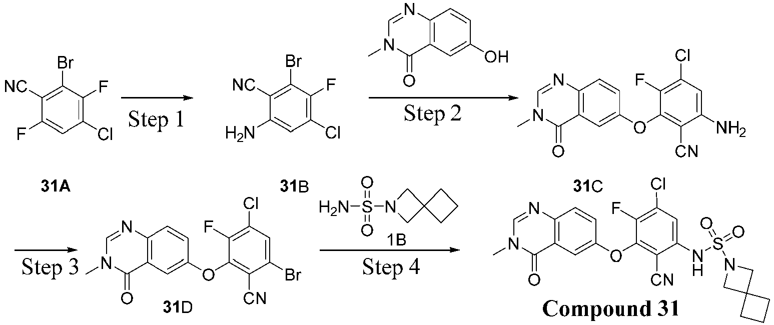

Step 1: 31A (10 g, 39.61 mmol), ammonia (20 mL) and 1,4-dioxane (100 mL) were added in sequence into a 250 mL reaction flask. The obtained mixture was reacted under stirring at 100°C for 24 h. The reaction solution was extracted (ethyl acetate), washed, and concentrated to obtain a crude product. Column chromatography separation was performed (petroleum ether:ethyl acetate = 1 : 0-0 : 1) to obtain 31B (8.5 g, yield 86.02%).

Step 2: In a 50 mL reaction flask, 31B (2 g, 8.02 mmol), 6-hydroxy-3-methylquinazolin-4(3H)-one (CAS: 19181-69-2, 2.31 g, 12.03 mmol), 18-crown-6 (2.12 g, 8.02 mmol), potassium carbonate (3.33 g, 24.06 mmol), and N-methylpyrrolidone (20 mL) were added sequentially. After the addition was completed, the mixture was stirred at 110°C for 12 hours. The reaction solution was extracted (ethyl acetate), washed, and concentrated to obtain a crude product. Column chromatography separation was performed (petroleum ether:ethyl acetate = 1:0-0:1) to obtain 31C (500 mg, yield 17.28%).

Step 3: 31C (0.25 g, 0.73 mmol), copper bromide (0.49 g, 2.19 mmol), tert-butyl nitrite (0.23 g, 2.19 mmol) and acetonitrile (3 mL) were added into a 50 mL reaction flask. After the addition, the obtained mixture was reacted under stirring at room temperature for 1 h. The reaction solution was extracted (ethyl acetate), washed, and concentrated to obtain a crude product. Column chromatography separation was performed (petroleum ether : ethyl acetate = 1 : 0-0 : 1) to obtain 31D (250 mg, yield 83.81%).

Step 4: 31D (250 mg, 0.61 mmol), 1B (0.22 g, 1.22 mmol), potassium carbonate (100 mg, 0.73 mmol), Xantphos (71 mg, 0.12 mmol), and Pd₂(dba)₃ (56 mg, 0.06 mmol) and 1,4-dioxane (5 mL) were added in sequence into a 50 mL reaction flask. The obtained mixture was reacted under stirring at 120°C for 5 h. The reaction solution was filtered, and the filtrate was concentrated under reduced pressure. The residue was separated and purified by preparative liquid chromatography to obtain compound 31 (88.1 mg, yield 28.6%).

LCMS m/z = 504.8 [M+H]⁺;
¹H NMR (400 MHz, CDCl₃) δ 8.09 (s, 1H), 7.79 (d, 1H), 7.64-7.56 (m, 3H), 3.99 (s, 4H), 3.60 (s, 3H), 2.20 (t, 4H), 1.89-1.81 (m, 2H).

### Example 32

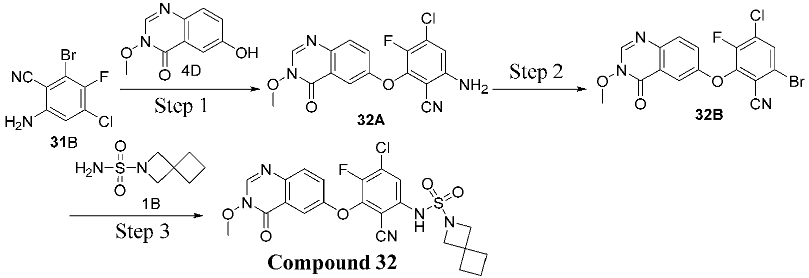

Step 1: 31B (2 g, 8.02 mmol), 4D (2.31 g, 12.03 mmol), 18-crown-6 (2.12 g, 8.02 mmol), potassium carbonate (3.33 g, 24.06 mmol) and N-methylpyrrolidone (20 mL) were added in sequence into a 50 mL reaction flask. After the addition, the obtained mixture was reacted under stirring at 110°C for 12 h. The reaction solution was extracted (ethyl acetate), washed, and concentrated to obtain a crude product. Column chromatography separation was performed (petroleum ether : ethyl acetate = 1 : 0-0 : 1) to obtain 32A (500 mg, yield 17.28%).

Step 2: 32A (0.13 g, 0.36 mmol), copper bromide (0.24 g, 1.08 mmol), tert-butyl nitrite (0.11 g, 1.08 mmol) and acetonitrile (3 mL) were added into a 50 mL reaction flask. After the addition, the obtained mixture was reacted under stirring at room temperature for 2 h. The reaction solution was extracted (ethyl acetate), washed, and concentrated to obtain a crude product. Column chromatography separation was performed (petroleum ether : ethyl acetate = 1 : 0-0 : 1) to obtain 32B (90 mg, yield 58.88%).

Step 3: 32B (90 mg, 0.21 mmol), 1B (74 g, 0.42 mmol), potassium carbonate (35 mg, 0.25 mmol), Xantphos (24 mg, 0.04 mmol), and Pd₂(dba)₃ (19 mg, 0.02 mmol) and 1,4-dioxane (3 mL) were added in sequence into a 50 mL reaction flask. The obtained mixture was reacted under stirring at 120°C for 5 h. The reaction solution was filtered, the filtrate was concentrated under reduced pressure, and the residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-70% acetonitrile; cycle time: 15 min) to obtain compound 32 (18.28 mg, yield 16.74%).

LCMS m/z = 520.9 [M+H]⁺;
¹H NMR (400 MHz, CDCl₃) δ 8.33 (s, 1H), 7.84 (d, 1H), 7.66-7.59 (m, 3H), 4.17 (s, 3H), 3.99 (s, 4H), 2.20 (t, 4H), 1.89-1.81 (m, 2H).

### Example 33

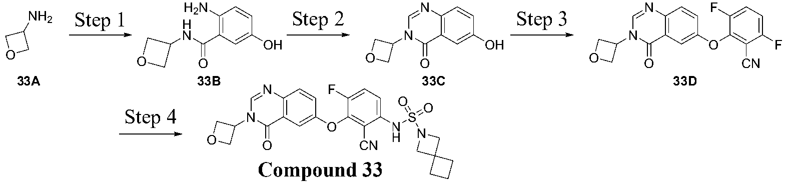

Step 1: 33A (2.92 g, 39.98 mmol) and water (40 mL) were added into a 250 mL single-neck flask. 4B (2.10 g, 11.76 mmol) was added at 0-5°C, and the obtained mixture was reacted at room temperature for 4 hrs. After the reaction was completed, the reaction was concentrated and purified by column chromatography (dichloromethane/methanol=10/1) to obtain 33B (2.2 g, yield 89%).

LCMS m/z = 209.1 [M+H]⁺;
Step 2: 33B (2.0 g, 9.61 mmol) was added to trimethyl orthoformate (20 mL) in a 100 mL single-neck flask. The obtained mixture was reacted at 105°C overnight. After the reaction was completed, the reaction was cooled to room temperature, concentrated and purified by column chromatography (petroleum ether/ethyl acetate = 1/2) to obtain 33C (870 mg, yield 41%).

LCMS m/z = 219.1 [M+H]⁺;
Step 3: 33C (0.87 g, 3.99 mmol) was dissolved in dry N,N-dimethylformamide (10 mL) in a 25 mL single-neck flask. Caesium carbonate (1.56 g, 4.79 mmol) was slowly added under an ice bath. After the addition, the obtained mixture was reacted under stirring at room temperature for 0.5 h. 2,3,6-trifluorobenzonitrile (0.66 g, 4.19 mmol) was slowly added dropwise under an ice bath. After the addition, the obtained mixture was reacted under stirring at room temperature overnight. After the reaction was completed, ethyl acetate (15 mL) was added to the reaction system to dilute the reaction. Then water (20 mL) was added to quench the reaction, and extraction was performed with ethyl acetate (20 mL × 2). The organic phase was washed with water (20 mL × 2). The organic phase was concentrated and then purified by column chromatography (petroleum ether/ethyl acetate = 1/1) to obtain 33D (0.71 g, yield 50%).

LCMS m/z =356.2 [M+H]⁺;
¹H NMR (400 MHz, DMSO-*d₆*) δ 8.39 (s, 1H), 8.00 - 7.94(m, 1H), 7.82-7.80(d, 1H), 7.76-7.73 (dd,1H), 7.61-7.55 (m, 2H), 5.55-4.47 (p, 1H), 4.97-4.93 (t, 2H), 4.89-4.85 (t, 2H).

Step 4: 1B (0.22 g, 1.24 mmol) and caesium carbonate (0.44 g, 1.36 mmol) were added to dry N,N-dimethylformamide (10 mL) in a 25 mL single-neck flask. The obtained mixture was reacted at 50°C for 30 min. A solution of 33D (0.40 g, 1.13 mmol) in N,N-dimethylformamide (2 mL) was added dropwise at 50°C. After the addition, the obtained mixture was reacted under stirring at 80°C for 4 hrs. After the reaction was completed, the reaction was filtered, and the filtrate was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-70% acetonitrile; cycle time: 15 min) to obtain 220 mg of solid, which was purified by column chromatography (petroleum ether/ethyl acetate = 1/1) to obtain compound 33 (160 mg, yield 27%).

LCMS m/z = 512.6 [M+H]⁺;
¹H NMR (400 MHz, DMSO-*d₆*) δ 10.37 (s, 1H), 8.38 (s, 1H), 7.89 - 7.80 (m, 2H), 7.72-7.69 (dd,1H), 7.55-7.52 (dd, 1H), 7.37-7.36 (d,1H), 5.53-5.45 (p, 1H), 4.96-4.92 (t, 2H), 4.88-4.84 (t, 2H), 3.83 (s, 4H), 2.12-2.08 (t, 4H), 1.78 - 1.70 (m, 2H).

¹⁹F NMR (377 MHz, DMSO-*d₆*) δ -127.30 (s).

### Example 34

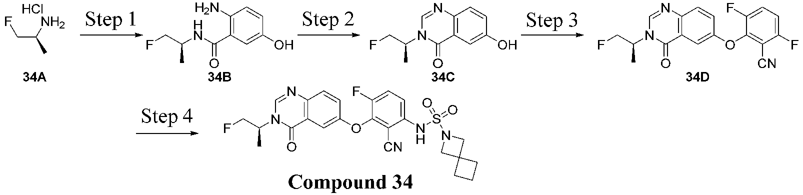

Step 1: 34A (2.00 g, 17.57 mmol) and water (20 mL) were added into a 250 mL single-neck flask. A solution of sodium hydroxide (0.62 g, 15.62 mmol) in water (20 mL) was slowly added dropwise at 0-5°C. The obtained mixture was reacted at 0-5°C for 10 min. Then 4B (0.87 g, 4.88 mmol) was added at 0-5°C and the obtained mixture was reacted at room temperature for 4 hrs. After the reaction was completed, the reaction was concentrated and then purified by column chromatography (dichloromethane/methanol=10/1) to obtain 34B (0.83 g, yield 80%).

LCMS m/z = 213.1 [M+H]⁺;
Step 2: 34B (0.72 g, 3.39 mmol) was added to trimethyl orthoformate (10 mL) in a 100 mL single-neck flask. The obtained mixture was reacted at 105°C overnight. After the reaction was completed, the reaction was cooled to room temperature, concentrated and purified by column chromatography (petroleum ether/ethyl acetate = 1/2) to obtain 34C (280 mg, yield 37%).

LCMS m/z = 223.2 [M+H]⁺;
Step 3: 34C (0.28 g, 1.26 mmol) was dissolved in dry N,N-dimethylformamide (4 mL) in a 25 mL single-neck flask. Caesium carbonate (0.49 g, 1.51 mmol) was slowly added under an ice bath. After the addition, the obtained mixture was reacted under stirring at room temperature for 0.5 h. 2,3,6-trifluorobenzonitrile (0.22 g, 1.39 mmol) was slowly added dropwise under an ice bath. After the addition, the obtained mixture was reacted under stirring at room temperature overnight. After the reaction was completed, ethyl acetate (10 mL) was added to the reaction system to dilute the reaction. Then water (10 mL) was added to quench the reaction, and extraction was performed with ethyl acetate (10 mL × 2). The organic phase was washed with water (10 mL × 2). The organic phase was concentrated and then purified by column chromatography (petroleum ether/ethyl acetate = 3/1) to obtain 34D (0.25 g, yield 55%).

LCMS m/z = 360.1 [M+H]⁺;
¹H NMR (400 MHz, DMSO-*d₆*) δ 8.44 (s, 1H), 8.01 - 7.95 (m, 1H), 7.81 - 7.73 (m, 2H), 7.62 - 7.56 (m, 2H), 5.17 - 5.04 (m, 1H), 4.91-4.86 (dd, 0.5H), 4.79 - 4.75 (m, 1H), 4.67-4.63 (dd, 0.5H), 1.48-1.45 (dd, 3H).

Step 4: 1B (0.13 g, 0.73 mmol) and caesium carbonate (0.27 g, 0.84 mmol) were added to dry N,N-dimethylformamide (6 mL) in a 25 mL single-neck flask. The obtained mixture was reacted at 50°C for 30 min. A solution of 34D (0.25 g, 0.70 mmol) in N,N-dimethylformamide (2 mL) was added dropwise at 50°C. After the addition, the obtained mixture was reacted under stirring at 80°C for 4 hrs. After the reaction was completed, the reaction was filtered, and the filtrate was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-70% acetonitrile; cycle time: 15 min) to obtain compound 34 (110 mg, yield 30%).

LCMS m/z = 516.2 [M+H]⁺;
¹H NMR (400 MHz, DMSO-*d₆*) δ 10.37 (s, 1H), 8.42 (s, 1H), 7.90-7.85 (dd, 1H), 7.82-7.79 (d, 1H), 7.73-7.70 (dd, 1H), 7.56-7.52 (dd,1H), 7.38-7.37 (d, 1H), 5.13-5.04 (m, 1H), 4.90-4.85 (dd, 0.5H), 4.78 - 4.73 (m, 1H), 4.65-4.62 (dd, 0.5H), 3.84 (s, 4H), 2.11-2.07 (t, 4H), 1.77 - 1.70 (m, 2H), 1.45 - 1.44 (m, 3H).

¹⁹F NMR (377 MHz, DMSO-*d₆*) δ -72.80 (s), -127.34 (s).

### Example 35

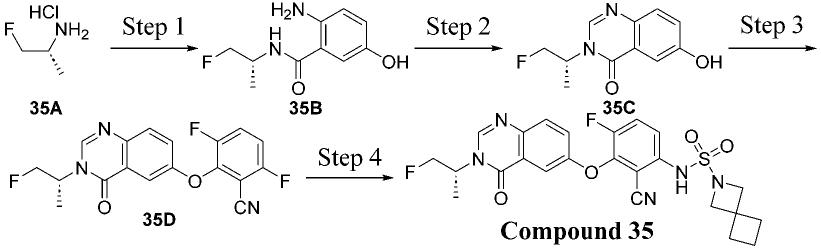

Step 1: 35A (2.50 g, 22.01 mmol) and water (25 mL) were added into a 250 mL single-neck flask. A solution of sodium hydroxide (0.79 g, 19.78 mmol) in water (25 mL) was slowly added dropwise at 0-5°C. The obtained mixture was reacted at 0-5°C for 10 min. Then 4B (1.10 g, 6.18 mmol) was added at 0-5°C and the obtained mixture was reacted at room temperature for 4 hrs. After the reaction was completed, the reaction was concentrated and then purified by column chromatography (dichloromethane/methanol=10/1) to obtain 35B (0.65 g, yield 49%).

LCMS m/z = 213.1 [M+H]⁺;
Step 2: 35B (0.72 g, 3.39 mmol) was added to triethyl orthoformate (10 mL) in a 100 mL single-neck flask. The obtained mixture was reacted under microwave at 180°C for 2.5 hrs. After the reaction was completed, the reaction was cooled to room temperature, and concentrated to obtain crude product 35C, which was directly used in the next reaction.

LCMS m/z =223.2 [M+H]⁺;
Step 3: Crude product 35C was dissolved in dry N,N-dimethylformamide (10 mL) in a 25 mL single-neck flask. Caesium carbonate (1.1 g, 3.41 mmol) was slowly added under an ice bath. After the addition, the obtained mixture was reacted under stirring at room temperature for 0.5 h. 2,3,6-trifluorobenzonitrile (0.49 g, 3.12 mmol) was slowly added dropwise under an ice bath. After the addition, the obtained mixture was reacted under stirring at room temperature overnight. After the reaction was completed, ethyl acetate (20 mL) was added to the reaction system to dilute the reaction. Then water (20 mL) was added to quench the reaction, and extraction was performed with ethyl acetate (20 mL × 2). The organic phase was washed with water (20 mL × 2). The organic phase was concentrated and then purified by column chromatography (petroleum ether/ethyl acetate = 3/1) to obtain 35D (0.16 g, yield 15%).

LCMS m/z = 360.1 [M+H]⁺;
Step 4: 1B (83.27 mg, 0.47 mmol) and caesium carbonate (0.17 g, 0.54 mmol) were added to dry N,N-dimethylformamide (4 mL) in a 25 mL single-neck flask. The obtained mixture was reacted at 50°C for 30 min. A solution of 35D (0.16 g, 0.45 mmol) in N,N-dimethylformamide (1 mL) was added dropwise at 50°C. After the addition, the obtained mixture was reacted under stirring at 80°C for 3 hrs. After the reaction was completed, the reaction was filtered, and the filtrate was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-70% acetonitrile; cycle time: 15 min) to obtain compound 35 (105 mg, yield 45%).

LCMS m/z = 516.6 [M+H]⁺;
¹H NMR (400 MHz, DMSO-*d₆*) δ 10.37 (s, 1H), 8.42 (s, 1H), 7.90 - 7.79 (m, 2H), 7.73-7.70 (dd,1H), 7.56-7.52 (dd, 1H), 7.38-7.37 (d, 1H), 5.13 - 5.04 (m, 1H), 4.89-4.85 (dd, 0.5H), 4.78 - 4.73 (m, 1H), 4.65-4.62 (dd, 0.5H), 3.83 (s, 4H), 2.11-2.07 (t, 4H), 1.77 - 1.70 (m, 2H), 1.46-1.44 (d, 3H).

¹⁹F NMR (377 MHz, DMSO-*d₆*) δ -74.66 (s), -129.42 (s).

### Example 36 and example 37

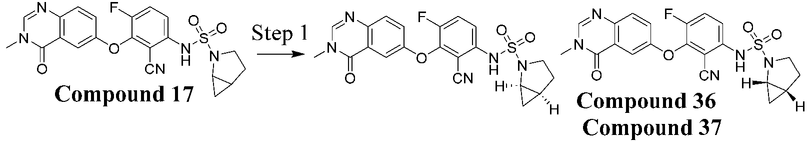

Compound 17 (300 mg) was subjected to chiral SFC resolution to obtain P1 (retention time: 0.588 min, set as compound 36) and P2 (retention time: 0.694 min, set as compound 37). Resolution method: instrument name: Waters 150 Prep-SFC F; chromatographic column: Chiralcel AD-Column; mobile phase: A for CO₂ and B for 0.1%NH₃•H₂O in MEOH and ACN; gradient: isocratic elution, mobile phase B: 35%; flow rate: 120 mL/min. Sample preparation: the compound was dissolved in acetonitrile at a concentration of 10 mg/mL; injection: 2.0 mL/injection; treatment: after the separation, the obtained product was concentrated by a rotary evaporator at 35°C, and then the solvent was dried by a lyophiliser at -80°C to obtain compound 36 (86 mg, 29%) and compound 37 (74 mg, 25%).

### Compound 36:

LCMS m/z = 456.1 [M+H]⁺;
¹H NMR (400 MHz, DMSO-*d₆*) δ 10.40 (s, 1H), 8.35 (s, 1H), 7.84 (t, 1H), 7.78 (d, 1H), 7.68 (dd, 1H), 7.62 (dd, 1H), 7.37 (d, 1H), 3.47 (s, 3H), 3.40 - 3.34 (m, 1H), 3.17 (dd, 1H), 2.93 (dd, 1H), 2.10 - 2.01 (m, 1H), 1.96 (dd, 1H), 1.60 (dt, 1H), 0.81 - 0.75 (m, 1H), 0.50 (dd, 1H).

¹⁹F NMR (377 MHz, DMSO-*d₆*) δ -128.22 (s).

### Compound 37:

LCMS m/z = 456.1 [M+H]⁺;
¹H NMR (400 MHz, DMSO-*d₆*) δ 10.42 (s, 1H), 8.36 (s, 1H), 7.85 (t, 1H), 7.78 (d, 1H), 7.68 (dd, 1H), 7.62 (dd, 1H), 7.38 (d, 1H), 3.47 (s, 3H), 3.37 (s, 1H), 3.17 (s, 1H), 2.93 (dd, 1H), 2.09 - 2.00 (m, 1H), 1.98 (d, 1H), 1.60 (s, 1H), 0.78 (s, 1H), 0.50 (d, 1H).

¹⁹F NMR (377 MHz, DMSO-*d₆*) δ -128.22 (s).

### Example 38

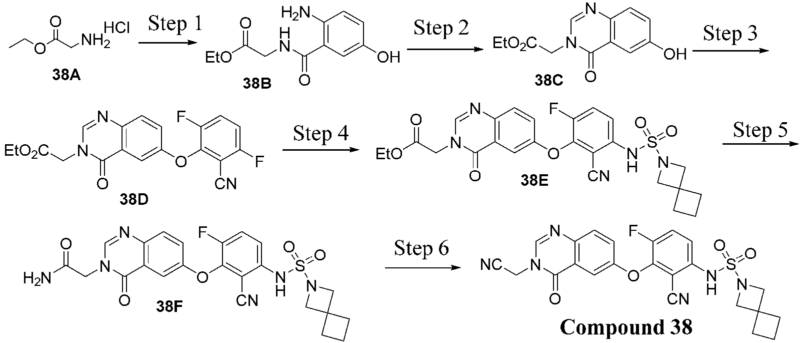

Step 1: 38A (16.66 g, 119.37 mmol) and water (120 mL) were added into a 1000 mL single-neck flask. A solution of sodium hydroxide (4.29 g, 107.30 mmol) in water (120 mL) was slowly added dropwise at 0-5°C. The obtained mixture was reacted at 0-5°C for 10 min. Then 6-hydroxy-2H-benzo[d][1,3]oxazine-2,4(1H)-dione (6.00 g, 33.53 mmol) was added at 0-5°C. The obtained mixture was reacted at room temperature for 4 hrs. After the reaction was completed, the reaction was concentrated and then purified by column chromatography (dichloromethane/methanol=10/1) to obtain 38B (3.0 g, yield 37.56%).

LCMS m/z = 239.1 [M+H]⁺;
Step 2: 38B (2.7 g, 11.33 mmol) was added to dry N,N-dimethylformamide (30 mL) in a 100 mL single-neck flask. Triethyl orthoformate (30 mL) was added at room temperature. The obtained mixture was reacted at 150°C for 4 hrs. After the reaction was completed, the reaction was cooled to room temperature, and concentrated to obtain crude product 38C, which was directly used in the next reaction.

LCMS m/z = 249.1 [M+H]⁺;
Step 3: 38C was dissolved in dry N,N-dimethylformamide (30 mL) in a 25 mL single-neck flask. Caesium carbonate (4.11 g, 13.54 mmol) was slowly added under an ice bath. After the addition, the obtained mixture was reacted under stirring at room temperature for 0.5 h. 2,3,6-trifluorobenzonitrile (1.95 g, 12.41 mmol) was slowly added dropwise under an ice bath. After the addition, the obtained mixture was reacted under stirring at room temperature overnight. After the reaction was completed, ethyl acetate (100 mL) was added to the reaction system to dilute the reaction. Then water (100 mL) was added to quench the reaction, and extraction was performed with ethyl acetate (100 mL × 2). The organic phase was washed with water (100 mL × 2), concentrated and then purified by column chromatography (petroleum ether/ethyl acetate = 3/1) to obtain 38D (2.4 g, yield 55%).

LCMS m/z = 386.2 [M+H]⁺;
Step 4: 1B (1.32 g, 7.48 mmol) and caesium carbonate (3.04 g, 9.37 mmol) were added to dry N,N-dimethylformamide (20 mL) in a 25 mL single-neck flask. The obtained mixture was reacted at 50°C for 30 min. A solution of 38D (2.40 g, 6.23 mmol) in N,N-dimethylformamide (10 mL) was added dropwise at 50°C. After the addition, the obtained mixture was reacted under stirring at 80°C overnight. After the reaction was completed, the reaction was filtered, and the organic phase of the filtrate was concentrated and then purified by column chromatography (dichloromethane/petroleum ether = 10/1) to obtain compound 38E (2.1 g, yield 62%).

LCMS m/z = 542.4 [M+H]⁺;
Step 5: 38E (2.00 g, 3.69 mmol) was added to methanol (5 mL) in a 25 mL single-neck flask. A solution of ammonia in methanol (7.0 M, 40mL) was added dropwise at 0-5°C. After the addition, the obtained mixture was reacted under stirring at room temperature overnight. After the reaction was completed, the reaction solution was concentrated to obtain compound 38F (crude product), which was directly used in the next reaction.

LCMS m/z = 513.1 [M+H]⁺;
Step 6: 38F (0.21 g, 0.41 mmol) was added to dry dichloromethane (6 mL) in a 25 mL single-neck flask. Burgess reagent (195.41 g, 0.82 mmol) was added at 0-5°C. After the addition, the obtained mixture was reacted under stirring at room temperature overnight. The reaction was filtered, and the filtrate was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-70% acetonitrile; cycle time: 15 min) to obtain compound 38 (35 mg, yield 17%).

LCMS m/z = 495.1 [M+H]⁺;
¹H NMR (400 MHz, DMSO-*d₆*) δ 10.36 (s, 1H), 8.40 (s, 1H), 7.90 - 7.82 (m, 2H), 7.75-7.73 (dd, 1H), 7.56-7.52 (dd, 1H), 7.44-7.43 (d, J = 2.9 Hz, 1H), 5.08 (s, 2H), 3.84 (s, 4H), 2.11-2.08 (t, 4H), 1.78 - 1.70 (m, 2H).

¹⁹F NMR (377 MHz, DMSO-*d₆*) δ -127.33 (s).

### Example 39

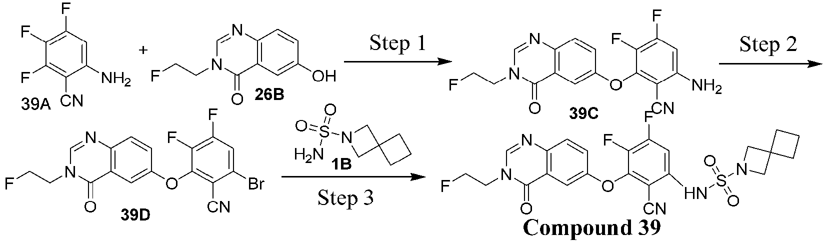

Step 1: 26B (627 mg, 3.01 mmol), 39A (701 mg, 3.01 mmol), and caesium carbonate (1.47 g, 4.51 mmol) were dissolved in dry N,N-dimethylformamide (25 mL) in a 100 mL single-neck flask. After the addition, the obtained mixture was reacted under stirring at room temperature for 2 h. After the reaction was completed, the reaction solution was diluted with ethyl acetate (200 mL). The organic phase was washed twice with water (60 mL), washed twice with saturated saline (50 mL), dried over anhydrous sodium sulphate and filtered. The filtrate was concentrated and purified by column chromatography (PE : EA=1 : 1) to obtain compound 39C (800 mg, yield: 74%).

Step 2: Compound 39C (800 mg, 2.22 mmol), copper bromide (992 mg, 4.44 mmol), and acetonitrile (40 ml) were added into a single-neck flask. Tert-butyl nitrite (458 mg, 4.44 mmol) was slowly added dropwise under an ice bath. After the addition, the obtained mixture was reacted at room temperature for 1 h. After the reaction was completed, the obtained mixture was filtered through diatomaceous earth pad. After the filtrate was concentrated, the residue was separated by column chromatography (petroleum ether : ethyl acetate (v/v) = 1 : 0 - 0 : 1) to obtain compound 39D (814 mg, yield: 86%).

Step 3: 39D (550 mg, 1.30 mmol), 39E (275 mg, 1.56 mmol), 2-di-tert-butylphosphino-2',4',6'-triisopropylbiphenyl (110 mg, 0.26 mmol), allylpalladium chloride (71 mg, 0.20 mmol), and potassium carbonate (629 mg, 4.55 mmol) were dissolved in dry methyltetrahydrofuran (40 mL) in a 100 mL single-neck flask. After the addition, nitrogen replacement was performed. The obtained mixture was reacted under stirring at 70°C for 5 h. After the reaction was completed, the reaction solution was filtered and the filtrate was concentrated. The resulting oily liquid was purified by column chromatography (DCM : EA = 1 : 2) to obtain a crude compound. The crude compound was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 30%-70% acetonitrile; cycle time: 15 min) to obtain compound 39 (210 mg, yield: 31%).

LCMS m/z = 520.50 [M+H]⁺;
¹H NMR (400 MHz, DMSO-*d₆*) δ 8.34 (s, 1H), 7.80 (d, 1H), 7.75 (dd, 1H), 7.59 (d, 1H), 7.53 (dd, 1H), 4.72 (dt, 2H), 4.32 (dt, 2H), 3.85 (s, 4H), 2.10 (t, 4H), 1.79 - 1.68 (m, 2H).

¹⁹F NMR (376 MHz, DMSO-*d₆*) δ -121.46 (s), -150.87 (s), -222.31 (s).

### Example 40 and example 41:

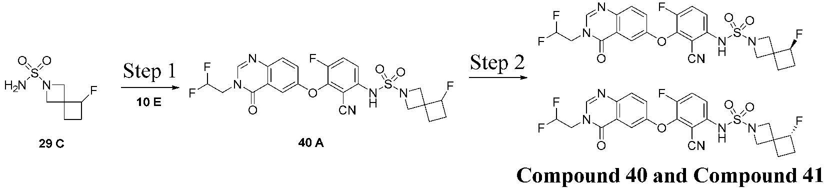

Step 1: 29C (0.400 g, 2.06 mmol) and caesium carbonate (1.01 g, 3.09 mmol) were added to dry N,N-dimethylformamide (10 mL) in a 25 mL single-neck flask. The obtained mixture was reacted at 50°C for 30 min. A solution of 10E (0.748 g, 2.06 mmol) in N,N-dimethylformamide (2 mL) was added dropwise at 50°C. After the addition, the obtained mixture was reacted under stirring at 100°C overnight. After the reaction was completed, the reaction solution was filtered. The filter cake was washed with N,N-dimethylformamide (4 mL). The filtrate was concentrated and then dissolved in water (15 mL) and ethyl acetate (10 mL). Liquid separation was performed. After the aqueous phase was extracted once with ethyl acetate (10 mL), the pH was adjusted to about 7 with saturated ammonium chloride solution. Then the aqueous phase was extracted with dichloromethane (20 mL × 3). The organic phase was dried over anhydrous sodium sulphate and then concentrated to obtain 40A crude product. The crude product was directly used in the next reaction.

LCMS m/z = 538.5 [M+H]⁺;
Step 2: The crude product 40A was subjected to chiral SFC resolution to obtain P1 (retention time: 14.73 min, set as compound 40) and P2 (retention time: 24.02 min, set as compound 41). Resolution method: instrument name: Waters 150 SFC; chromatographic column: AD; mobile phase: A for CO₂ and B for IPA+MeOH (0.05%NH₃·H₂O); flow rate: 42 mL/min; column pressure: 100 bar; column temperature: 25°C; absorption wavelength: 220 nm cycle time: 40 min). Sample preparation: the compound was dissolved in methanol at a concentration of 40 mg/mL; injection: 5 mL/injection; treatment: after the separation, the obtained product was concentrated by a rotary evaporator at 35°C, and then the solvent was dried by a lyophiliser at -80°C to obtain P1 and P2.

Compound 40: (P1: 120 mg, 11%), ¹H NMR (400 MHz, DMSO-*d₆*) δ 10.45 (s, 1H), 8.33 (s, 1H), 7.83 - 7.80 (m, 2H), 7.72-7.69 (dd, 1H), 7.54-7.50 (dd, 1H), 7.43-7.42 (d, 1H), 6.51 - 6.22 (m, 1H), 5.02-4.99 (dt, 1H), 4.51-4.43 (td, 2H), 4.16-4.14 (d, 1H), 3.92 - 3.77 (m, 3H), 2.18-2.07 (m, 1H), 2.00 - 1.79 (m, 2H), 1.69-1.62 (m, 1H).

¹⁹F NMR (376 MHz, DMSO-*d₆*) δ -120.44 (s), -171.59 (s), -216.41 (s).

Compound 41: (P2: 140mg, 13%), ¹H NMR (400 MHz, DMSO-*d₆*) δ 10.45 (s, 1H), 8.34 (s, 1H), 7.88-7.80 (m, 2H), 7.73-7.70 (dd, 1H), 7.54-7.51 (dd, 1H), 7.43-7.42 (d, 1H), 6.51 - 6.22 (m, 1H), 5.03-4.99 (dt, 1H), 4.51-4.43 (td, 2H), 4.17-4.15 (d, 1H), 3.93 - 3.78 (m, 3H), 2.18 - 2.10 (m, 1H), 2.00 - 1.79 (m, 2H), 1.69-1.62 (m, 1H).

¹⁹F NMR (376 MHz, DMSO-*d₆*) δ -120.44 (s), -127.17 (s), -171.59 (s).

### Example 42

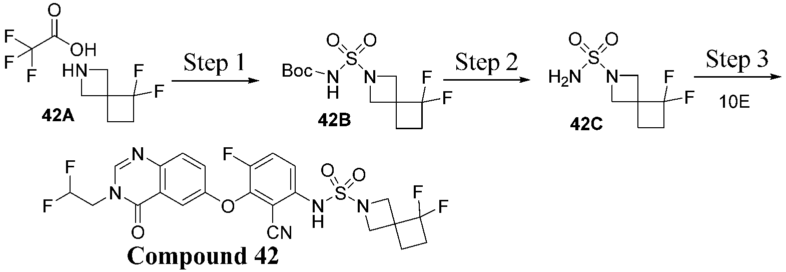

Step 1: 42A (420 mg, 1.70 mmol) and acetonitrile (5 mL) were added into a 25 mL single-neck flask. Triethylamine (0.430 g, 4.25 mmol) and (tert-butoxycarbonyl)((4-(dimethylimino)pyridin-1(4H)yl)sulphonyl)amide (0.615 g, 2.04 mmol) were slowly added dropwise at 0-5°C. The obtained mixture was reacted at room temperature overnight. After the reaction was completed, the reaction was concentrated to obtain crude product 42B, which was directly used in the next reaction.

LCMS m/z = 257.1 [M+H]⁺;
Step 2: The crude product 42B was dissolved in dichloromethane (10 mL) in a 25 mL single-neck flask. Trifluoroacetic acid (5 mL) was slowly added dropwise at 0-5°C. After the addition, the obtained mixture was reacted at room temperature overnight. After the reaction was completed, the reaction was concentrated, and the organic phase was concentrated and then purified by column chromatography (petroleum ether/ethyl acetate = 2/1) to obtain 42C (0.250 g, yield 70%).

LCMS m/z = 213.2 [M+H]⁺;
Step 3: 42C (0.250 g, 1.18 mmol) and caesium carbonate (0.577 g, 1.77 mmol) were added to dry N,N-dimethylformamide (5 mL) in a 25 mL single-neck flask. The obtained mixture was reacted at 50°C for 30 min. A solution of 10E (0.429 g, 1.18 mmol) in N,N-dimethylformamide (2 mL) was added dropwise at 50°C. After the addition, the obtained mixture was reacted under stirring at 80°C overnight. After the reaction was completed, the reaction solution was filtered, and the filtrate was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-70% acetonitrile; cycle time: 15 min) to obtain compound 42 (110 mg, yield 17%).

LCMS m/z = 556.0 [M+H]⁺.

¹H NMR (400 MHz, DMSO-*d₆*) δ 10.57 (s, 1H), 8.34 (s, 1H), 7.92 - 7.87 (m, 1H), 7.83-7.80 (d, 1H), 7.73-7.70 (dd, 1H), 7.55-7.52 (dd, 1H), 7.44 (d, 1H), 6.51-6.22 (m, 1H), 4.52-4.43 (m , 2H), 4.08-4.06 (d, 2H), 3.91-3.88 (d, 2H), 2.47 - 2.42 (m, 2H), 2.04-2.00 (m , 2H).

¹⁹F NMR (376 MHz, DMSO-*d₆*) δ -98.98 (s), -120.45 (s), -126.75 (s).

### Example 43

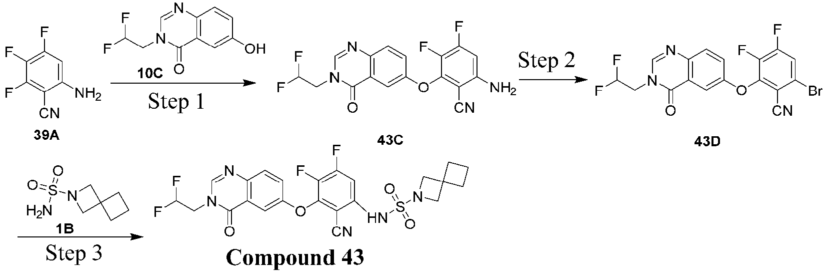

Step 1: 10C (700 mg, 3.09 mmol), 39A (720 mg, 3.09 mmol), and caesium carbonate (1.53 g, 4.63 mmol) were dissolved in dry N,N-dimethylformamide (25 mL) in a 100 mL single-neck flask. After the addition, the obtained mixture was reacted under stirring at room temperature for 2 h. After the reaction was completed, the reaction solution was diluted with ethyl acetate (200 mL). The organic phase was washed twice with water (60 mL), washed twice with saturated saline (50 mL), dried over anhydrous sodium sulphate and filtered. The filtrate was concentrated and purified by column chromatography (PE : EA=1 : 1) to obtain compound 43C (830 mg, yield: 71%).

Step 2: Compound 43C (830 mg, 2.22 mmol), copper bromide (992 mg, 4.44 mmol), and acetonitrile (40 ml) were added into a single-neck flask. Tert-butyl nitrite (458 mg, 4.44 mmol) was slowly added dropwise under an ice bath. After the addition, the obtained mixture was reacted at room temperature for 1 h. After the reaction was completed, the obtained mixture was filtered through diatomaceous earth pad. After the filtrate was concentrated, the residue was separated by column chromatography (petroleum ether : ethyl acetate (v/v) = 1 : 0 - 0 : 1) to obtain compound 43D (800 mg, yield: 82%).

Step 3: 43D (800 mg, 1.81 mmol), 1B (400 mg, 2.26 mmol), 2-di-tert-butylphosphino-2',4',6'-triisopropylbiphenyl (154 mg, 0.36 mmol), allylpalladium chloride (99 mg, 0.27 mmol), and potassium carbonate (625 mg, 4.25 mmol) were dissolved in dry methyltetrahydrofuran (40 mL) in a 100 mL single-neck flask. After the addition, nitrogen replacement was performed. The obtained mixture was reacted under stirring at 70°C for 5 h. After the reaction was completed, the reaction solution was filtered and the filtrate was concentrated. The resulting oily liquid was purified by column chromatography (DCM : EA = 1 : 2) to obtain a crude compound. The crude compound was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 30%-70% acetonitrile; cycle time: 15 min) to obtain compound 43 (56 mg, yield: 6%).

LCMS m/z = 538.1 [M+H]⁺;
¹H NMR (400 MHz, DMSO-*d₆*) δ 8.35 (s, 1H), 7.81 (d, 1H), 7.77 (dd, 1H), 7.59 (d, 1H), 7.52 (dd, 1H), 6.52 - 6.23 (m, 1H), 4.49 (td, 2H), 3.83 (s, 4H), 2.08 - 2.11 (m, 4H), 1.77 - 1.70 (m, 2H).

### Example 44

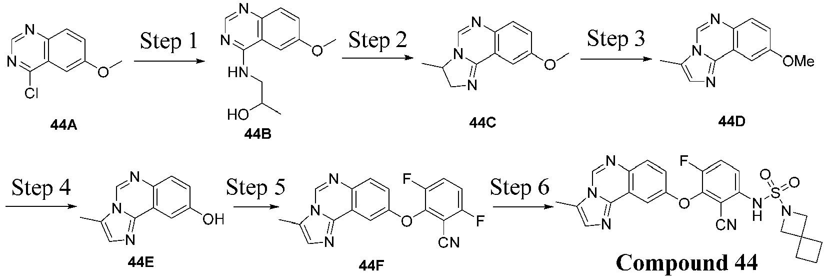

Step 1: 44A (4.5 g, 23.13 mmol) and isopropyl alcohol (50 mL) were added into a 250 mL single-neck flask. Then triethylamine (4.68 g, 46.26 mmol) and isopropanolamine (2.08 g, 27.76 mmol) were added. The obtained mixture was reacted at 80°C for 1 hr. After the reaction was completed, the reaction was concentrated, slurried with ethyl acetate (50 mL), and filtered, and the filter cake was washed with petroleum ether (50 mL) to obtain 44B (5.30 g, yield 98%).

LCMS m/z = 234.3 [M+H]⁺;
Step 2: Phosphorus oxychloride (60 mL) was added into a 250 mL single-neck flask. 44B (5.3 g, 22.72 mmol) was slowly added in portions at 0-5°C. After the addition, the obtained mixture was reacted at 110°C for 5 hrs. After the reaction was completed, the reaction solution was concentrated, then water (100 mL) was slowly added to quench the reaction at 0-5°C, and extraction was performed with ethyl acetate (100 mL × 2). The organic phase was concentrated and then purified by column chromatography (petroleum ether/ethyl acetate = 1/1) to obtain 44C (3.6 g, yield 73%).

LCMS m/z = 216.2 [M+H]⁺;
Step 3: 4C (2.80 g, 13.01 mmol) and acetonitrile (30 mL) were added into a 100 mL single-neck flask. Then alumina (2.65 g, 25.99 mmol) and potassium permanganate (3.08 g, 19.49 mmol) were added. After the addition, the obtained mixture was reacted under stirring at room temperature overnight. After the reaction was completed, the reaction solution was filtered, and the filtrate was concentrated and then purified by column chromatography (petroleum ether/ethyl acetate = 1/2) to obtain 44D (0.88 g, yield 31%).

LCMS m/z = 214.1 [M+H]⁺;
¹H NMR (400 MHz, DMSO-d6) δ 9.01 (s, 1H), 7.86-7.84 (d, 1H), 7.74-7.73 (d, 1H), 7.35 (s, 1H), 7.32-7.29 (dd, 1H), 3.94 (s, 3H), 2.58 (s, 3H).

Step 4: 4D (0.88 g, 4.13 mmol) and N,N-dimethylformamide (10 mL) were added into a 50 mL single-neck flask. Then sodium thioethylate (1.04 g, 12.35 mmol) was added. After the addition, nitrogen protection was performed. The obtained mixture was reacted under stirring at 130°C overnight. After the reaction was completed, the reaction solution was concentrated. Dilute hydrochloric acid was added to adjust the pH to acidic. After concentration, a residue was obtained. The residue was purified by column chromatography (dichloromethane/methanol=20/1) to obtain 44E (0.68 g, yield 82%).

LCMS m/z =200.1 [M+H]⁺;
Step 5: 44E (0.30 g, 1.51 mmol) was dissolved in dry N,N-dimethylformamide (8 mL) in a 25 mL single-neck flask. Caesium carbonate (0.74 g, 2.26 mmol) was slowly added under an ice bath. After the addition, the obtained mixture was reacted under stirring at room temperature for 0.5 h. 2,3,6-trifluorobenzonitrile (0.28 g, 1.81 mmol) was slowly added dropwise under an ice bath. After the addition, the obtained mixture was reacted under stirring at room temperature overnight. After the reaction was completed, the reaction solution was filtered, and the filtrate was concentrated and then purified by column chromatography (petroleum ether/ethyl acetate = 1/1) to obtain 44F (0.45 g, yield 88%).

LCMS m/z =337.0 [M+H]⁺;
Step 6: 1B (0.21 g, 1.16 mmol) and caesium carbonate (0.47 g, 1.46 mmol) were added to dry N,N-dimethylformamide (5 mL) in a 25 mL single-neck flask. The obtained mixture was reacted at 50°C for 30 min. A solution of 44F (0.36 g, 0.97 mmol) in N,N-dimethylformamide (1 mL) was added dropwise at 50°C. After the addition, the obtained mixture was reacted under stirring at 80°C for 4 hrs. After the reaction was completed, the reaction solution was filtered. The filter cake was washed with N,N-dimethylformamide (2 mL). The filtrate was concentrated, and then ethyl acetate (15 mL) and water (15 mL) were added for dissolution. Liquid separation was performed, and the aqueous phase was adjusted to neutral pH with saturated ammonium chloride, and extracted with dichloromethane (20 mL × 3). The organic phases were combined, dried over anhydrous sodium sulphate, and then purified by column chromatography (petroleum ether/ethyl acetate = 1/1) to obtain compound 44 (61 mg, yield 12%).

LCMS m/z = 493.5 [M+H]⁺;
¹H NMR (400 MHz, DMSO-*d₆*) δ 10.44 (s, 1H), 9.19 (s, 1H), 8.00-7.98 (d, 1H), 7.93-7.88 (t, 1H), 7.81 (s, 1H), 7.59-7.55 (m, 2H), 7.52-7.51 (d, 1H), 3.84 (s, 4H), 2.36 (s, 3H), 2.10-2.06 (t, 4H), 1.73 - 1.65 (m, 2H).

¹⁹F NMR (376 MHz, DMSO-*d₆*) δ -127.37 (s).

### Example 45

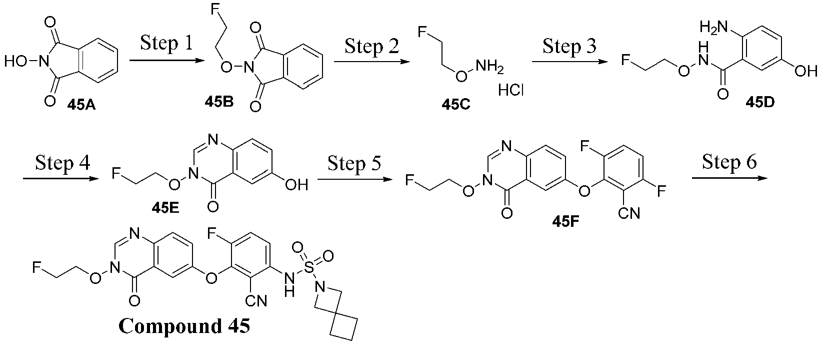

Step 1: 45A (10 g, 61.33 mmol) and N,N-dimethylformamide (100 mL) were added into a 250 mL single-neck flask. N,N-diisopropylethylamine (15.85 g, 122.64 mmol) and 1-fluoro-2-iodoethane (12.80 g, 73.63 mmol) were slowly added at 0-5°C. The obtained mixture was reacted at 80°C overnight. After the reaction was completed, the reaction solution was added dropwise to water (500 mL), and a large amount of solids were precipitated. The obtained mixture was reacted under stirring for 30 min, and filtered. The filter cake was washed with water (100 mL) and petroleum ether (50 mL), and then concentrated to afford 45B (10.0 g, yield 78%).

LCMS m/z = 210.2 [M+H]⁺;
Step 2: 45B (10 g, 47.81 mmol) was dissolved with dichloromethane (260 mL) and methanol (26 mL) in a 500 mL single-neck flask. Hydrazine hydrate (5.98 g, 95.62 mmol) was slowly added dropwise at room temperature. After the addition, the obtained mixture was reacted at room temperature 2.5 hrs. After the reaction was completed, the reaction solution was filtered. The filtrate was washed with 5N ammonia (200 mL) and extracted with dichloromethane (200 mL × 2). The organic phase was dried over anhydrous sodium sulphate and then concentrated. The residue was dissolved in ethanol (50 mL). Then concentrated hydrochloric acid (8 mL) was added. The obtained mixture was stirred for 30 min and then concentrated. The resulting residue was slurried with ethyl acetate (30 mL). Filtration was performed to obtain 45C (1.90 g, yield 34%).

¹H NMR (400 MHz, DMSO-*d₆*) δ 11.25 (s, 3H), 4.74 - 4.72 (m, 1H), 4.62 - 4.60 (m, 1H), 4.36 - 4.34 (m, 1H), 4.29 - 4.27 (m, 1H).

Step 3: 45C (0.49 g, 3.23 mmol) and water (10 mL) were added into a 250 mL single-neck flask. A solution of sodium hydroxide (0.12 g, 3.04 mmol) in water (10 mL) was slowly added dropwise at 0-5°C. The obtained mixture was reacted at 0-5°C for 10 min. Then 6-hydroxy-2H-benzo[d][1,3]oxazine-2,4(1H)-dione (0.17 g, 0.95 mmol) was added at 0-5°C and the obtained mixture was reacted at room temperature overnight. After the reaction was completed, the reaction solution was concentrated and purified by column chromatography (dichloromethane/methanol=10/1) to obtain 45D (0.20 g, yield 98%).

LCMS m/z = 215.1 [M+H]⁺;
Step 4: 45D (0.2 g, 0.93 mmol) was added to triethyl orthoformate (3 mL) and N,N-dimethylformamide (3 mL) in a 100 mL single-neck flask. The obtained mixture was reacted at 150°C for 4 hrs. After the reaction was completed, the reaction solution was cooled to room temperature, and concentrated to obtain crude product 45E, which was directly used in the next reaction.

LCMS m/z = 225.1 [M+H]⁺;
Step 5: 45E (0.31 g, 1.38 mmol) was dissolved in dry N,N-dimethylformamide (5 mL) in a 25 mL single-neck flask. Caesium carbonate (0.54 g, 1.66 mmol) was added under an ice bath. 2,3,6-trifluorobenzonitrile (0.24 g, 1.52 mmol) was slowly added dropwise. After the addition, the obtained mixture was reacted under stirring at room temperature overnight. After the reaction was completed, the reaction was filtered, and the filtrate was concentrated and then purified by column chromatography (petroleum ether/ethyl acetate = 2/3) to obtain 45F (0.36 g, yield 72%).

LCMS m/z =362.4 [M+H]⁺;
Step 6: 1B (0.21 g, 1.20 mmol) and caesium carbonate (0.49 g, 1.5 mmol) were added to dry N,N-dimethylformamide (8 mL) in a 25 mL single-neck flask. The obtained mixture was reacted at 50°C for 30 min. A solution of 45F (0.36 g, 1.00 mmol) in N,N-dimethylformamide (2 mL) was added dropwise at 50°C. After the addition, the obtained mixture was reacted under stirring at 80°C overnight. After the reaction was completed, the reaction solution was filtered, and the filtrate was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-70% acetonitrile; cycle time: 15 min) to obtain compound 45 (83.0 mg, yield 16%).

LCMS m/z = 518.2 [M+H]⁺;
¹H NMR (400 MHz, DMSO-*d₆*) δ 10.38 (s, 1H), 8.58 (s, 1H), 7.91 - 7.83 (m, 2H), 7.74-7.71 (dd, 1H), 7.56-7.53 (dd, 1H), 7.42-7.41 (d,1H), 4.83 - 4.69 (m, 2H), 4.59 - 4.49 (m, 2H), 3.84 (s, 4H), 2.12-2.08 (t, 4H), 1.78 - 1.70 (m, 2H).

¹⁹F NMR (376 MHz, DMSO-*d₆*) δ -127.24 (s), -220.60 (s).

### Example 46

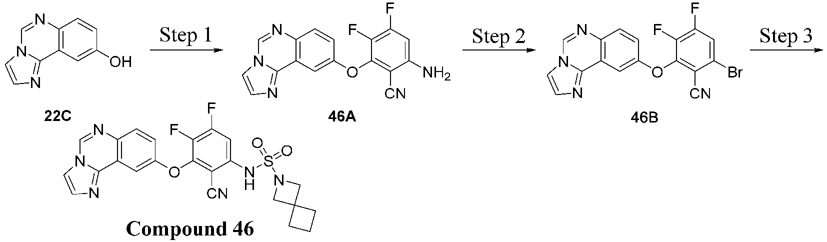

Step 1: 22C (1.35 g, 7.29 mmol) was dissolved in dry N,N-dimethylformamide (30 mL) in a 25 mL single-neck flask. Caesium carbonate (4.75 g, 14.58 mmol) was slowly added under an ice bath. After the addition, the obtained mixture was reacted under stirring at room temperature for 0.5 h. 39A (1.51 g, 8.75 mmol) was slowly added dropwise under an ice bath. After the addition, the obtained mixture was reacted under stirring at 30°C overnight. After the reaction was completed, the reaction solution was filtered, and the filtrate was concentrated and then purified by column chromatography (petroleum ether/ethyl acetate = 3/7) to obtain 46A (2.3 g, yield 93%).

LCMS m/z =338.3 [M+H]⁺;
Step 2: 46A (2.30 g, 6.82 mmol) was dissolved in dry acetonitrile (50 mL) in a 25 mL single-neck flask. Tert-butyl nitrite (0.71 g, 6.89 mmol) and copper bromide (1.54 g, 6.90 mmol) were slowly added at 0-5°C. After the addition, the obtained mixture was reacted under stirring at room temperature overnight. After the reaction was completed, the reaction solution was concentrated. After the concentration, saturated sodium bicarbonate (100 mL) was added to quench the reaction, and ethyl acetate (100 mL) was used for dissolution. Then filtration was performed. The filtrate was extracted with ethyl acetate (100 mL × 2). The organic phase was washed with water (100 mL) and saturated brine (100 mL), dried over anhydrous sodium sulphate, and then concentrated. The residue was purified by column chromatography (petroleum ether/ethyl acetate = 2/3) to obtain 46B (1.00 g, yield 36%).

LCMS m/z =401.0 [M+H]⁺;
Step 3: 46B (0.80 g, 1.99 mmol), 1B (0.46 g, 2.59 mmol), 2-dicyclohexylphosphine-2',4',6'-triisopropylbiphenyl (0.10 g, 0.20 mmol), potassium carbonate (0.41 g, 2.98 mmol), and tris(dibenzylidene-BASEacetone)dipalladium (0.11 g, 0.19 mmol) were added to dry 1,4-dioxane (16 mL). The obtained mixture was purged with nitrogen for 2 min and then reacted under microwave at 120°C for 1 hr. After the reaction was completed, the filtrate was concentrated, and then ethyl acetate (20 mL) and water (20 mL) were added for dissolution. Liquid separation was performed, and the aqueous phase was adjusted to neutral pH with saturated ammonium chloride, and extracted with ethyl acetate (20 mL × 3). The organic phases were combined, dried over anhydrous sodium sulphate, and then concentrated. The residue was purified by column chromatography (petroleum ether/ethyl acetate = 3/2), and then subjected to chiral SFC resolution to obtain compound 46 (retention time: 2.08 min). Resolution method: instrument name: Waters 150 Prep-SFC F; chromatographic column: Chiralcel AD column; mobile phase: A for CO₂ and B for MeOH (0.1%NH₃·H₂O); gradient: B 40%; flow rate: 100 mL/min; column pressure: 100 bar; column temperature: 25°C; absorption wavelength: 220 nm cycle time: 3.8 min). Sample preparation: the compound was dissolved in acetonitrile at a concentration of 10 mg/mL; injection: 3.5 mL/injection; treatment: after the separation, the obtained product was concentrated by a rotary evaporator at 35°C, and then the solvent was dried by a lyophiliser at -80°C to obtain compound 46 (0.18 mg, yield 18%).

LCMS m/z = 497.8 [M+H]⁺;
¹H NMR (400 MHz, DMSO-*d₆*) δ 9.28 (s, 1H), 8.10-8.09 (d, 1H), 8.02-7.99 (d, 1H), 7.78-7.77 (d, 1H), 7.62 - 7.58 (m, 2H), 7.50-7.46 (m, 1H), 3.79 (s, 4H), 2.10-2.06 (t, 4H), 1.75 - 1.67 (m, 2H).

### Example 47

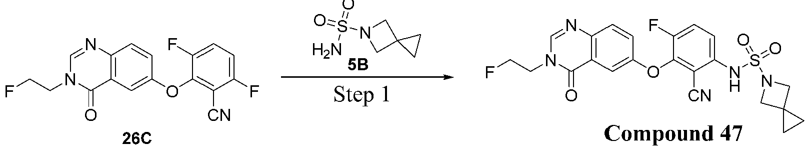

Step 1: Compound 5B (0.55 g, 3.39 mmol) was dissolved in dry N,N-dimethylformamide (10 mL) in a 250 mL single-neck flask. After the addition of caesium carbonate (1.42 g, 4.36 mmol), the obtained mixture was reacted at 50°C for 0.5 h. Then a solution of 26C (1 g,2.90 mmol) in N,N-dimethylformamide (10 mL) was added dropwise. After the addition, the reaction solution was stirred at 85°C for 12 h. After the reaction was completed, ethyl acetate (100 mL) was added. Then washing was performed with water (70 mL × 2). The organic layer was dried over anhydrous sodium sulphate and filtered. The filtrate was concentrated under reduced pressure. The residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 25%-65% acetonitrile; cycle time: 15 min) to obtain compound 47 (700 mg, yield 49%).

LCMS m/z = 488.30 [M+H]⁺;
¹H NMR (400 MHz, DMSO-*d₆*) δ 10.47 (s, 1H), 8.33 (s, 1H), 7.88 (t, 1H), 7.80 (d, 1H), 7.76-7.68 (m, 1H), 7.61-7.56 (m, 1H), 7.41 (d, 1H), 4.79-4.62 (m, 2H), 4.36-4.25 (m, 2H), 3.97 (s, 4H), 0.61 (s, 4H).

### Example 48

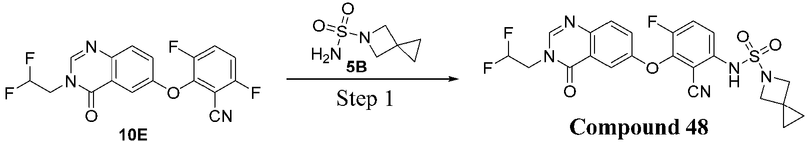

Step 1: Compound 5B (8.2 g, 50.55 mmol) was dissolved in dry N,N-dimethylformamide (50 mL) in a 250 mL single-neck flask. After the addition of caesium carbonate (16.6 g, 7.41 mmol), the obtained mixture was stirred at 50°C for 0.5 h. Then a solution of 9E (2.5 g,6.88 mmol) in N,N-dimethylformamide (15 mL) was added dropwise. After the addition, the reaction solution was stirred at 85°C for 12 h. After the reaction was completed, filtration was performed. The filtrate was concentrated, and the obtained residue was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-70% acetonitrile; cycle time: 10 min) to obtain compound 48 (250 mg, yield 7%).

LCMS m/z = 506.90 [M+H]⁺;
¹H NMR (400 MHz, DMSO-*d₆*) δ 8.34 (s, 1H), 7.93 (t, 1H), 7.86-7.76 (m, 3H), 7.74-7.69 (m, 1H), 7.48 (d, 1H), 6.54-6.18 (m, 1H), 4.53-4.43 (m, 1H), 3.55 (s, 2H), 3.25 (s, 2H), 0.64 (s, 4H).

### Example 49

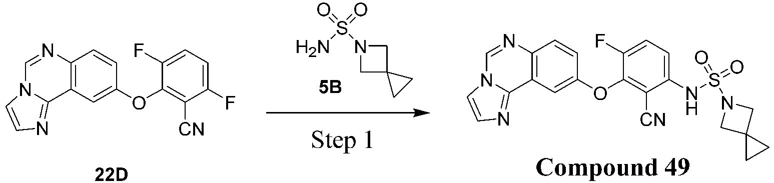

Step 1: Raw material 5B (0.45 g, 2.77 mmol) was dissolved in DMF (10mL). Then caesium carbonate (0.91 g, 2.79 mmol) was added. The reaction solution was heated to 50°C and stirred for 30 min. Then raw material 22D (0.6g, 1.86mmol) was added, and the reaction solution was heated to 85°C and stirred overnight. After the reaction was completed, the reaction mixture was filtered through diatomaceous earth, and the filtrate was concentrated. The resulting residue was purified by slurrying with EA to obtain 0.50 g of a white solid. Then, the white solid was purified through a silica gel column (dichloromethane : methanol = 20 : 1) to obtain compound 49 (0.38 g, 43.99%).

LCMS m/z = 465.3 [M+H]⁺.

¹H NMR (400 MHz, DMSO-*d₆*) δ 9.27 (s, 1H), 8.09 (d, 1H), 8.01 (d, 1H), 7.74 (t, 1H), 7.63 (d, 1H), 7.60 - 7.52 (m, 3H), 3.89 (s, 4H), 0.57 (s, 4H).

### Example 50

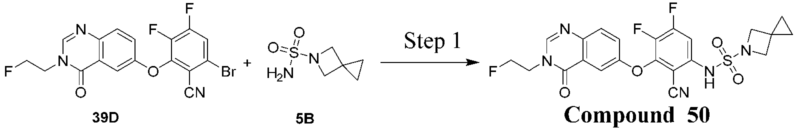

Step 1: 39D (500 mg, 1.18 mmol), 5B (230 mg, 1.42 mmol), 2-di-tert-butylphosphino-2',4',6'-triisopropylbiphenyl (100 mg, 0.24 mmol), allylpalladium chloride (65 mg, 0.18 mmol), and potassium carbonate (571 mg, 4.13 mmol) were dissolved in dry methyltetrahydrofuran (40 mL) in a 100 mL single-neck flask. After the addition, nitrogen replacement was performed. The obtained mixture was reacted under stirring at 70°C for 5 h. After the reaction was completed, the reaction solution was filtered and the filtrate was concentrated. The resulting oily liquid was purified by column chromatography (DCM : EA = 1 : 2) to obtain a crude compound. The crude compound was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 30%-80% acetonitrile; cycle time: 20 min) to obtain compound 50 (72 mg, yield: 12%).

LCMS m/z = 506.1 [M+H]⁺;
¹H NMR (400 MHz, DMSO-*d₆*) δ 8.33 (s, 1H), 7.79 (d, 1H), 7.74 (dd, 1H), 7.57 (d, 1H), 7.51 (dd, 1H), 4.71 (dt, 2H), 4.32 (dt, 2H), 3.93 (s, 4H), 0.59 (s, 4H).

### Example 51

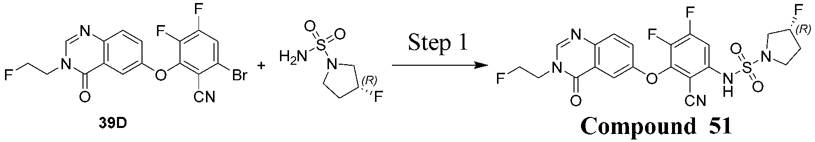

Step 1: 39D (500 mg, 1.18 mmol), (3R)-3-fluoropyrrolidine-1-sulphonamide (238 mg, 1.42 mmol), 2-di-tert-butylphosphino-2',4',6'-triisopropylbiphenyl (100 mg, 0.24 mmol), allylpalladium chloride (65 mg, 0.18 mmol), and potassium carbonate (571 mg, 4.13 mmol) were dissolved in dry methyltetrahydrofuran (40 mL) in a 100 mL single-neck flask. After the addition, nitrogen replacement was performed. The obtained mixture was reacted under stirring at 70°C for 5 h. After the reaction was completed, the reaction solution was filtered and the filtrate was concentrated. The resulting oily liquid was purified by column chromatography (DCM:EA=1:2) to obtain a crude compound. The crude compound was separated and purified by preparative liquid chromatography (instrument: waters 2767 preparative liquid chromatographic instrument; chromatographic column: SunFire@ Prep C18 (19 mm × 150 mm); composition of mobile phase: mobile phase A: acetonitrile; mobile phase B: water (containing 0.1% trifluoroacetic acid); gradient: isocratic elution using 20%-80% acetonitrile; cycle time: 15 min) to obtain compound 51 (210 mg, yield: 31%).

LCMS m/z = 512.1 [M+H]⁺;
¹H NMR (400 MHz, DMSO-*d₆*) δ 8.34 (s, 1H), 7.80 (d, 1H), 7.74 (dd, 1H), 7.58 (d, 1H), 7.52 (dd, 1H), 5.40 (s, 1H), 5.26 (s, 1H), 4.72 (dt, 2H), 4.32 (dt, 2H), 3.57 - 3.31 (m, 4H), 2.23 - 1.99 (m, 2H).

### Example 52

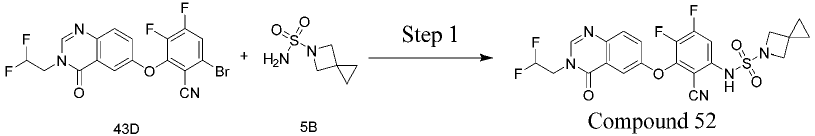

Step 1: 43D (800 mg, 1.81 mmol), 5B (352 mg, 2.17 mmol), 2-di-tert-butylphosphino-2',4',6'-triisopropylbiphenyl (80 mg, 0.18 mmol), and potassium carbonate (876 mg, 6.33 mmol) were dissolved in dry 1,4-dioxane (40 mL). Allylpalladium chloride (33 mg, 0.09 mmol) was added. After the addition, nitrogen replacement was performed. The obtained mixture was reacted under stirring at 85°C for 10 h. After the reaction was completed as monitored by TLC (dichloromethane: methanol =20:1 (v/v)), the reaction solution was filtered, the filtrate was concentrated, and the crude product was purified by column chromatography (eluent: dichloromethane: methanol = 50:1 (v/v)) to obtain the target compound 52(340 mg, yield: 36%).

LCMS *m*/*z* = 524.2 [M+1]⁺.

¹H NMR (400 MHZ, DMSO-*d₆*) *δ* 8.35 (s, 1H), 7.81 (d, 1H), 7.77 (dd, 1H), 7.62 (d, 1H), 7.57 (dd, 1H), 6.37 (tt, 1H), 4.49 (td, 2H), 3.99 (s, 4H), 0.61 (s, 4H).

### Example 53

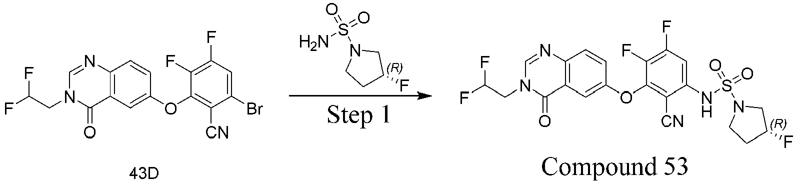

Step 1: 43D (800 mg, 1.81 mmol), (3R)-3-fluoropyrrolidine-1-sulphonamide (365 mg, 2.17 mmol), 2-di-tert-butylphosphino-2',4',6'-triisopropylbiphenyl (80 mg, 0.18 mmol), and potassium carbonate (876 mg, 6.33 mmol) were dissolved in dry 1,4-dioxane (40 mL). Allylpalladium chloride (33 mg, 0.09 mmol) was added. After the addition, nitrogen replacement was performed. The obtained mixture was reacted under stirring at 85°C for 10 h. After the reaction was completed as monitored by TLC (dichloromethane : methanol =20 : 1 (v/v)), the reaction solution was filtered, the filtrate was concentrated, and the crude product was purified by column chromatography (eluent: dichloromethane : methanol = 65 : 1 (v/v)) to obtain the target compound 53 (650 mg, yield: 68%).

LCMS m/z = 530.1 [M+1]⁺.

¹H NMR (400 MHZ, DMSO-*d₆*) *δ* 8.35 (s, 1H), 7.81 (d, 1H), 7.75 (dd, 1H), 7.60 (d, 1H), 7.52 (dd, 1H), 6.37 (tt, 1H), 5.33 (d, 1H), 4.49 (td, 2H), 3.58 - 3.34 (m, 4H), 2.21 - 2.01 (m, 2H).

### Preparing a preparation:

### 1. Preparation 1-1: specification 20 mg/tablet:

| Name of raw/auxiliary material | Amount (mg/tablet) | Function |
|---|---|---|
| Compound 10 | 20 | Active drug |
| Microcrystalline cellulose | 80 | Filler |
| Hypromellose | 5 | Binder |
| Hydroxypropyl betacyclodextrin | 85 | Solubilizer |
| Colloidal silicon dioxide | 2 | Glidant |
| Crospovidone | 6 | Disintegrant |
| Sodium stearyl fumarate | 2 | Lubricant |
| Total | 200 | / |

### 2. Preparation 1-2; specification 20 mg/tablet

| Name of raw/auxiliary material | Amount (mg/tablet) | Function |
|---|---|---|
| Compound 10 | 20 | Active drug |
| Microcrystalline cellulose | 160 | Filler |
| Hypromellose | 5 | Binder |
| Magnesium oxide | 5 | PH regulator |
| Colloidal silicon dioxide | 2 | Glidant |
| Crospovidone | 6 | Disintegrant |
| Sodium stearyl fumarate | 2 | Lubricant |
| Total | 200 | / |

### 3. Preparation 1-3; specification 20 mg/tablet

| Name of raw/auxiliary material | Amount (mg/tablet) | Function |
|---|---|---|
| Compound 10 | 20 | Active drug |
| Microcrystalline cellulose | 145 | Filler |
| Hypromellose | 5 | Binder |
| Sodium lauryl sulphate | 20 | Surfactant |
| Colloidal silicon dioxide | 2 | Glidant |
| Crospovidone | 6 | Disintegrant |
| Sodium stearyl fumarate | 2 | Lubricant |
| Total | 200 | / |

### 4. Preparation 1-4: specification 200 mg/tablet:

| Name of raw/auxiliary material | Amount (mg/tablet) | Function |
|---|---|---|
| Compound 10 | 200 | Active drug |
| Hydroxypropyl betacyclodextrin | 256 | Solubilizer |
| Hypromellose | 16 | Binder |
| Microcrystalline cellulose | 296 | Filler |
| Colloidal silicon dioxide | 16 | Glidant |
| Crospovidone | 8 | Disintegrant |
| Sodium stearyl fumarate | 8 | Lubricant |
| Total | 800 | / |

### 5. Preparation 1-5: specification 1000 mg/tablet:

| Name of raw/auxiliary material | Amount (mg/tablet) | Function |
|---|---|---|
| Compound 10 | 1000 | Active drug |
| Microcrystalline cellulose | 950 | Filler |
| Hypromellose | 60 | Binder |
| Sodium lauryl sulphate | 285 | Surfactant |
| Colloidal silicon dioxide | 45 | Glidant |
| Crospovidone | 48 | Disintegrant |
| Sodium stearyl fumarate | 12 | Lubricant |
| Total | 2400 | / |

### Method for preparing the above-mentioned prescription preparations:

1). Active pharmaceutical ingredient (API)-auxiliary micronized blend Compound 10, together with a surfactant (if any) and/or a pH regulator (if any), was weighed and mixed according to the formulation ratio. After mixing uniformly, the mixture was micronized.
2). Weighing: The API-auxiliary micronized blend, filler, binder, glidant, disintegrant, and lubricant were weighed according to the formulation;
3). Mixing: The API-auxiliary micronized blend, filler, binder, glidant, and disintegrant were mixed for 5 minutes, then the lubricant was added and mixing was continued for 2 minutes;
4). Tabletting: The powder obtained in 3) was compressed into tablets using suitable punches and dies, and the tablet weight and hardness were controlled during the compression process.

Other compounds of the present invention, such as the compounds of the Examples, were formulated into corresponding preparations with reference to the above preparation method.

### Biological Assay Examples

### 1. BRAF_{V600E} enzyme activity test

BRAF_{V600E} (ABCAM, ab204154) and MEK1_{K97R} (USBio, M2865-06J) proteins were diluted at an appropriate dilution factor using 1 × Assay buffer (PH = 7.4 Tris-HCl buffer, supplemented with 10 mM MgCl₂) such that the final concentration of BRAF_{V600E} was 10 ng/µL and the final concentration of substrate MEK1_{K97R} was 1 µM. 1 µL of BRAF_{V600E}, 1 µL of compound serial dilution (final concentration starting from 2 µM, 5-fold dilution, 8 concentrations), and 6 µL of Assay buffer were pipetted to a 384-well reaction plate with a capacity of 20 µL. The plate was pre-incubated in a constant temperature incubator at 37°C for 30 min. 1 µL of 200 µM ATP and 1 µM MEK1 _{K97R} were added to the reaction wells corresponding to the compound incubation, mixed evenly by vortexing, and preincubation was performed in a constant temperature incubator at 37°C for 60 min for enzymatic reaction. After the reaction was completed, 5 µL of the above reaction product was pipetted to another 384-well plate, and 5 µL of formulated ADP-Glo^{™} Reagent (Promega, V9101) was added and mixed evenly by pipetting, and then the plate was placed at room temperature for 40 min. 10 µL Kinase Detection Reagent was added to the 384-well plate and incubated at room temperature for 40 min. Finally, a microplate reader (BMG LRBTECH) was used and Luminescence module was selected to detect the LUM fluorescence value of each well (the gain value was fixed at 3600), and the formula $Inhibition rate % = \left(1-\frac{LUM Compound - LUM Background}{LUM Solvent - LUM Background}\right) ∗ 100 %$ was used to calculate the inhibition rate of the compound on BRAF_{V600E}. The Graphpad software log(inhibitor) vs. response -- Variable slope (four parameters) equation was used to perform fitting analysis and calculate the IC₅₀value of the sample.

The compounds of the present invention, such as the compounds in the examples, have very good enzymatic activities, with IC₅₀ ≤ 100 nM. The inhibitory activities of some compounds on BRAF_{V600E} are shown in Table 1.

**Table 1 Inhibitory activities of compounds on BRAFV600E**

| Compound No. | IC₅₀/nM |
|---|---|
| Compound 1 | B |
| Compound 9 | B |
| Compound 10 | A |
| Compound 11 | B |
| Compound 26 | A |
| Compound 39 | A |
| Compound 40 | A |

| | |
|---|---|
| A represents IC₅₀ ≤ 10 nM, B represents 10 nM < IC₅₀ ≤ 50 nM, C represents 50 nM < IC₅₀ ≤ 100 nM. | |

Conclusion: the compounds of the present invention, such as the compounds in the examples, show high inhibitory activities on BRAF_{V600E}.

### 2. Inhibition of A375 cell proliferation

A375 cells (ATCC, CRL-1619) were cultured in DMEM complete medium (+10% FBS) in a CO₂ incubator at 37°C for 48 h. The cells were trypsinized and counted, and then the density was adjusted to 1.67 × 10⁴ cells/mL. 90 µL (1500 cells) of cells were inoculated into each well of a 96-well transparent bottom plate, and the plate was transferred to a CO₂ incubator and the cells were cultured overnight at 37°C. After the cells were incubated overnight, 10 µL of the diluted compound (final concentration starting from 10 µM, 3-fold dilution, 11 concentrations) was added to each well using a multi-channel pipette. The positive control was a serum-free medium containing DMSO. After mixing evenly, the cells were placed in a CO₂ incubator at 37°C for 72 h. After the incubation, the CellCounting-Lite^{®}2.0 kit detection solution (Vazyme, DD1101-03) was taken out and returned to room temperature. 100 µL of CellCounting-Lite2.0 detection solution was added to each well, the plate was sealed with a sealing film, and the plate was placed on a shaker for shaking for 15 min (the whole process should be kept away from light). The Luminescence module of the microplate reader (BMG LRBTECH) was used to detect the fluorescence signal value LUM of each well. The formula $Inhibition rate % = \left(\frac{LUM Solvent - LUM Compound}{LUM Solvent}\right) ∗ 100 %$ was used to calculate the inhibition rate of the compound. The Graphpad software log(inhibitor) vs. response -- Variable slope (four parameters) equation was used to perform fitting analysis and calculate the IC₅₀value of the sample. The ordinate of the data is the percentage of inhibition rate, and the abscissa is the logarithm of the sample concentration (Log₁₀).

The compounds of the present invention, such as the compounds in the examples, have very good cell activities, with IC₅₀ ≤ 100 nM. The inhibitory activities of some compounds on A357 cells are shown in Table 2.

**Table 2 Inhibitory activities of compounds on A375 cells**

| Compound No. | IC₅₀/nM | Compound No. | IC₅₀/nM |
|---|---|---|---|
| Compound 1 | A | Compound 39 | A |
| Compound 4 | A | Compound 40 | A |
| Compound 5 | A | Compound 42 | A |
| Compound 9 | A | Compound 44 | A |
| Compound 10 | A | Compound 46 | A |
| Compound 14 | A | Compound 47 | A |
| Compound 17 | A | Compound 49 | A |
| Compound 22 | A | Compound 50 | A |
| Compound 26 | A | Compound 51 | A |
| Compound 33 | A | | |

| | | | |
|---|---|---|---|
| A represents IC₅₀ ≤ 10 nM, B represents 10 nM < IC₅₀ ≤ 50 nM, C represents 50 nM < IC₅₀ ≤ 100 nM. | | | |

Conclusion: the compounds of the present invention, such as the compounds in the examples, show high inhibitory activities at the cellular level.

### 3. Mouse pharmacokinetic assay

**1.1 Experimental animals:** male ICR mice, 20-25 g, 12 mice/compound. purchased from Chengdu Dossy Experimental Animals Co., Ltd.

**1.2 Experimental design:** On the day of the experiment, the ICR mice were randomly grouped according to their body weights. The animals were fasted with water available for 12 to 14 h one day before the administration and were fed 4 h after the administration.

**Table 3. Administration information**

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | Control compoun d 1 | 2.5 | 0.5 | 5 | Plasma | Intravenous administration |
| G2 | 9 | | 10 | 1 | 10 | Plasma | Intragastric administration |
| G3 | 3 | Test compound | 2.5 | 0.5 | 5 | Plasma | Intravenous administration |
| G4 | 9 | | 10 | 1 | 10 | Plasma | Intragastric administration |

| | | | | | | | |
|---|---|---|---|---|---|---|---|
| Note: Control compound 1 was compound Example 1 in the document WO 2021116055 A1; vehicle for intravenous administration: 5%DMA+5%HS-15+90%NS; vehicle for intragastric administration: 0.5% MC | | | | | | | |

Before and after administration, 0.06 mL of blood was taken from the orbit under isoflurane anaesthesia and placed in an EDTAK2 centrifuge tube for centrifugation at 5000 rpm at 4°C for 10 min, followed by plasma collection. The blood collection time points for the intravenous administration group and intragastric administration group were 0, 5 min, 15 min, 30 min, 1 h, 2 h, 4 h, 7 h and 24 h. Before analysis and detection, all samples were stored at -80°C, and a quantitative analysis of samples was performed using LC-MS/MS.

**Table 4. Pharmacokinetic parameters of test compounds in plasma of mice**

| Test compound | Mode of administration | CL (mL/min/kg) | Vdₛₛ (L/kg) | AUC₀₋ₜ (hr×ng/mL) | T_{1/2} (h) | F (%) | brain/plas ma ratio |
|---|---|---|---|---|---|---|---|
| Control compound 1 | i.v. (2.5 mg/kg) | 0.532 | 0.195 | 75119 | 5.32 | - | - |
| | i.g. (10 mg/kg) | - | - | 240899 | 2.46 | 80.2 | 0.00292 |
| Compound 1 | i.v. (2.5 mg/kg) | 0.133 | 0.148 | 223249 | 14.8 | - | - |
| | i.g. (10 mg/kg) | - | - | 861406 | 10.7 | 96.5 | 0.0130 |
| Compound 4 | i.v. (2.5 mg/kg) | 0.37 | 0.147 | 107297 | 5.27 | - | - |
| | i.g. (10 mg/kg) | - | - | 282018 | 4.02 | - | 0.053 |
| Compound 5 | i.v. (2.5 mg/kg) | 0.0627 | 0.159 | 289734 | 30.2 | - | - |
| | i.g. (10 mg/kg) | - | - | 1084669 | 19.2 | 93.6 | 0.0181 |
| Compound 7 | i.v. (2.5 mg/kg) | 0.144 | 0.111 | 239760 | 9.79 | - | - |
| | i.g. (10 mg/kg) | - | - | 792179 | 13.7 | 92.6 | 0.0425 |
| Compound 8 | i.v. (2.5 mg/kg) | 0.147 | 0.0941 | 246506 | 8.59 | - | - |
| | i.g. (10 mg/kg) | - | - | 1303073 | 12.9 | ≥ 98 | 0.0173 |
| Compound 10 | i.v. (2.5 mg/kg) | 0.117 | 0.111 | 272309 | 12 | - | - |
| | i.g. (10 mg/kg) | - | - | 914322 | 11.8 | 83.9 | 0.0107 |
| Compound 11 | i.v. (2.5 mg/kg) | 0.0822 | 0.0837 | 376040 | 12.8 | - | - |
| | i.g. (10 mg/kg) | - | - | 848012 | 11.74 | - | 0.00950 |
| Compound 26 | i.v. (2.5 mg/kg) | 0.119 | 0.114 | 266055 | 12.8 | - | - |
| | i.g. (10 mg/kg) | - | - | 831553 | 8.68 | 78.1 | 0.01785 |
| Compound 44 | i.v. (2.5 mg/kg) | 0.335 | 0.135 | 125977 | 7.5 | - | - |
| | i.g. (10 mg/kg) | - | - | 442783 | 14.2 | 87.9 | 0.0162 |

| | | | | | | | |
|---|---|---|---|---|---|---|---|
| Note: Control compound 1 was compound Example 1 in the document WO 2021116055 A1; -: not applicable. | | | | | | | |

Conclusion: The compounds of the present invention, such as compounds in the examples, have good pharmacokinetic characteristics in mice and have better brain-penetrating properties than the control compounds.

### 4. Beagle Dog Pharmacokinetic Assay

**Experimental animals:** male beagle dogs, about 8-11 kg, 6 beagle dogs/compound, purchased from Beijing Marshall Biotechnology Co., Ltd.

**Experimental method:** On the day of the experiment, the beagle dogs were randomly grouped according to their body weights. The animals were fasted with water available for 12 to 14 h one day before the administration and were fed 4 h after the administration. The animals were administered according to Table 5.

**Table 5. Administration information**

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | Control compound 1 | 1 | 1 | 1 | Plasma | Intravenous administration |
| G2 | 3 | Control compound 1 | 3 | 0.6 | 5 | Plasma | Intragastric administration |
| G3 | 3 | Test compound | 0.5 | 0.25 | 2 | Plasma | Intravenous administration |
| G4 | 3 | Test compound | 3 | 0.6 | 5 | Plasma | Intragastric administration |

| | | | | | | | |
|---|---|---|---|---|---|---|---|
| Note: Vehicle for intravenous administration: 5% DMA+5% Solutol+90% Saline; vehicle for intragastric administration: 0.5% MC (DMA: dimethylacetamide; Solutol: polyethylene glycol-15-hydroxystearate; Saline: physiological saline; MC: Methylcellulose solution) | | | | | | | |

Before and after the administration, 1 ml of blood was taken from the jugular veins or limb veins, and placed in an EDTAK2 centrifuge tube. Centrifugation was performed at 5000 rpm at 4°C for 10 min, and plasma was collected. The blood collection time points for the intravenous group and intragastric group were both 0 min, 5 min, 15 min, 30 min, 1 h, 2 h, 4 h, 6 h, 8 h, 10 h, 12 h, 24 h, 48 h, and 72 h. Before analysis and detection, all samples were stored at -80°C. The samples were analysed quantitatively by LC-MS/MS.

**Table 6. Pharmacokinetic parameters of test compounds in plasma of beagle dogs**

| Test compound | Mode of administration | CL (mL/min/kg) | Vdₛₛ (L/kg) | AUC₀₋ₜ (hr×ng/mL) | F (%) |
|---|---|---|---|---|---|
| Control compound 1 | i.v. (1 mg/kg) | 0.686 | 0.189 | 24350 | - |
| Control compound 1 | i.g. (3 mg/kg) | - | - | 53143 | 72.7 |
| Compound 1 | i.v. (0.5 mg/kg) | 0.492 | 0.156 | 17680 | - |
| Compound 1 | i.g. (3 mg/kg) | - | - | 116724 | ≥ 98 |
| Compound 10 | i.v. (0.5 mg/kg) | 0.425 | 0.155 | 40879 | - |
| Compound 10 | i.g. (3 mg/kg) | - | - | 97760 | 79.7 |
| Compound 39 | i.v. (0.5 mg/kg) | 0.541 | 0.08 | 31095 | - |
| Compound 39 | i.g. (3 mg/kg) | - | - | 84281 | 90.3 |

| | | | | | |
|---|---|---|---|---|---|
| Note: Control compound 1 was compound Example 1 in the document WO 2021116055 A1; -: not applicable. | | | | | |

**Conclusion:** The compounds of the present invention, such as the compounds in the examples, have good pharmacokinetic characteristics in beagle dogs.

### 5. Rat Pharmacokinetic Assay

5.1 Experimental animals: Male SD rats, about 220 g, 6-8 weeks old, 6 rats/compound, purchased from Chengdu Dossy Experimental Animals Co., Ltd.

5.2 Experimental design: On the day of the experiment, the SD rats were randomly grouped according to their body weights. The animals were fasted with water available for 12 to 14 h one day before the administration and were fed 4 h after the administration.

**Table 7 Administration information**

| Group | Number | Administration information | | | | | |
|---|---|---|---|---|---|---|---|
| | Male | Test compound | Administrati on dosage (mg/kg) | Administr ation concentrat ion (mg/mL) | Administrati on volume (mL/kg) | Collected samples | Mode of administrat ion |
| G1 | 3 | Test compound | 2.5 | 0.5 | 5 | Plasma | Intravenous administrat ion |
| G2 | 3 | | 10 | 1 | 10 | Plasma | Intragastric administrat ion |

| | | | | | | | |
|---|---|---|---|---|---|---|---|
| Vehicle for administration: 0.5% MC | | | | | | | |

Before and after the administration, 0.1 ml of blood was taken from the orbit of the animals under isoflurane anaesthesia and placed in an EDTAK2 centrifuge tube. The blood was centrifuged at 5000 rpm and 4°C for 10 min to collect plasma. Blood sampling time points for the intravenous administration group: 0, 5 min, 15 min, 30 min, 1 h, 2 h, 4 h, 6 h, 8 h, and 24 h; blood sampling time points for the intragastric administration group: 0, 5 min, 15 min, 30 min, 1 h, 2 h, 4 h, 6 h, 8 h and 24 h. Before analysis and detection, all samples were stored at -80°C.

**Table 8 Pharmacokinetic parameters of test compounds in plasma of rats**

| Test compound | Mode of administration | CL (mL/min/kg) | Vdₛₛ (L/kg) | AUC₀₋ₜ (hr×ng/mL) | T_{1/2} (h) | F (%) |
|---|---|---|---|---|---|---|
| Control compound 1 | Intravenous administration (2.5 mg/kg) | 0.171 | 0.142 | 199410 | 10.5 | - |
| Control compound 1 | Intragastric administration (10 mg/kg) | - | - | 634644 | 9.73 | 79.6 |
| Compound 47 | Intravenous administration (2.5 mg/kg) | 0.036 | 0.156 | 1156844 | 50.9 | |
| Compound 47 | Intragastric administration (10 mg/kg) | - | - | 4256852 | 60.6 | 92 |

| | | | | | | |
|---|---|---|---|---|---|---|
| Note: Control compound 1 was compound Example 1 in the document WO 2021116055 A1; -: not applicable. | | | | | | |

Conclusion: The compounds of the present invention, such as the compounds in the examples, have good bioavailability and pharmacokinetic characteristics in rats.

### 6. Preparation stability investigation

### 6.1 High temperature (60°C) investigation test

Taking a bare sample of preparation 1-4, placing same under high temperature of 60°C for investigation, and taking samples for testing at day 5 and day 10, respectively. The test results are shown in the following table.

**Table 9 Results of high temperature investigation test**

| Detection item | | Day 0 | Day 5 | Day 10 |
|---|---|---|---|---|
| Character | This sample being an off-white to white circular tablet | Off-white circular tablet | Off-white circular tablet | Off-white circular tablet |
| Related Substances (%) | Impurity with RRT of about 0.85 | 0.25 | 0.27 | 0.29 |
| | Other maximum single impurity | 0.05 | 0.06 | 0.07 |
| | Total impurity | 0.63 | 0.65 | 0.66 |
| Dissolution rate (%) | | 93 | 90 | 91 |
| Content determination (%) | | 99.8 | 99.6 | 99.4 |

| | | | | |
|---|---|---|---|---|
| Note: RRT: Relative Retention Time | | | | |

### 6.2 High humidity (RH 92.5%) investigation test

Taking a bare sample of preparation 1-4, placing same under high humidity of RH 92.5% for investigation, and taking samples for testing at day 5 and day 10, respectively. The test results are shown in the following table.

**Table 10 Results of High humidity investigation test**

| Detection item | | 0 day | Day 5 | Day 10 |
|---|---|---|---|---|
| Character | This sample being an off-white to white circular tablet | Off-white circular tablet | Off-white circular tablet | Off-white circular tablet |
| Related Substances (%) | Impurity with RRT of about 0.85 | 0.25 | 0.24 | 0.25 |
| | Other maximum single impurity | 0.05 | 0.05 | 0.05 |
| | Total impurity | 0.63 | 0.62 | 0.64 |
| Dissolution rate (%) | | 93 | 92 | 93 |
| Content determination (%) | | 99.8 | 99.5 | 99.9 |

### 6.3 Accelerated test

Taking a sample of preparation 1-4 to simulate the commercially available packaging and placing same at 40°C ± 2°C and RH 75% ± 5% for investigation, and taking a sample for testing. The test results are shown in the following table.

**Table 11 Results of accelerated test**

| Detection item | | 0 day | Day 28 |
|---|---|---|---|
| Character | This sample being an off-white to white circular tablet | Off-white circular tablet | Off-white circular tablet |
| Related Substances (%) | Impurity with RRT of about 0.85 | 0.25 | 0.30 |
| | Other maximum single impurity | 0.05 | 0.08 |
| | Total impurity | 0.63 | 0.69 |
| Dissolution rate (%) | | 93 | 91 |
| Content determination (%) | | 99.8 | 98.9 |

Conclusion: The compound preparations of the present invention all had good stability under high temperature, high humidity, and accelerated conditions.

## Claims

1. A pharmaceutical composition or a pharmaceutical preparation, **characterised in that** the pharmaceutical composition or the pharmaceutical preparation comprises an active ingredient M and a pharmaceutically acceptable excipient, and the active ingredient M is selected from a compound as shown in general formula I, or a stereoisomer, tautomer, deuterated product, solvate, prodrug, metabolite, pharmaceutically acceptable salt or eutectic crystal thereof, 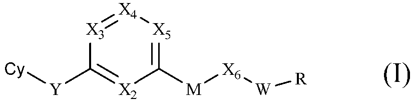
wherein, Cy is selected from P1, P2, P3, P4, P5, P6, P7 or P8; 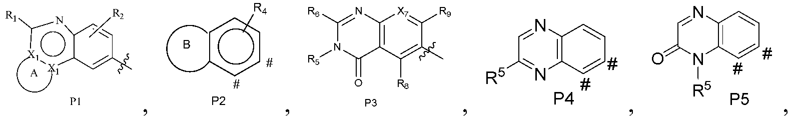 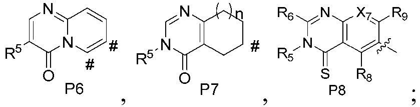
ring A is 5-membered or 6-membered heteroaryl containing 1 to 3 heteroatoms selected from N, S, and O, and the heteroaryl is optionally substituted with 1 or 2 groups selected from halogen, C₁₋₄ alkoxy, -NHC₁₋₄ alkyl, -N(C₁₋₄ alkyl)₂, CN, C₁₋₄ alkyl, haloC₁₋₄ alkoxy and haloC₁₋₄ alkyl;
ring B is 5-membered heterocycle containing 1 to 3 heteroatoms selected from N, S, and O, and the heterocycle is optionally substituted with 1 to 3 groups selected from =O, halogen, C₁₋₄ alkyl, haloC₁₋₄ alkyl, C₁₋₄ alkoxy, OH and haloC₁₋₄ alkoxy;
# means that one point is selected to connect to Y;
n is selected from 0 or 1;
each X₁ is independently N or C;
X₂ is N or CR₃;
X₃ is N or CR₃₁;
X₄ is N or CR₃₂;
X₅ is N or CR₃₃;
X₆ is C(O), S(O) or S(O)₂;
X₇ is CR₇ or N;
R₁, R₂ and R₄ are independently H, halogen, OH, -NHC₁₋₄ alkyl, -N(C₁₋₄ alkyl)₂, CN, C₁₋₄ alkyl, C₁₋₄ alkoxy, haloC₁₋₄ alkoxy, haloC₁₋₄ alkyl, C₂₋₄ alkenyl or C₂₋₄ alkynyl;
R₃, R₃₁, R₃₂, and R₃₃ are independently H, halogen, C₁₋₄ alkyl, haloC₁₋₄ alkyl, C₂₋₄ alkenyl, C₂₋₄ alkynyl, C₁₋₄ alkoxy, haloC₁₋₄ alkoxy, -NHC₁₋₄ alkyl, -N(C₁₋₄ alkyl)₂, CN or C₃₋₆ cycloalkyl, alternatively, R₃₁ and R₃₂, or R₃₂ and R₃₃ together with the atoms to which they are attached form C₃₋₆ carbocycle or 5-membered or 6-membered heterocycle containing 1 to 3 heteroatoms selected from N, S, and O, and the carbocycle or the heterocycle is optionally substituted with 1 to 3 groups selected from halogen, C₁₋₄ alkyl, haloC₁₋₄ alkyl, C₁₋₄ alkoxy, OH, NH₂ and CN;
R₅ is C₁₋₄ alkyl, haloC₁₋₄ alkyl, C₃₋₆ cycloalkyl, 4-membered to 7-membered heterocycloalkyl containing 1-3 heteroatoms selected from N, S, and O, C₁₋₄ alkoxy, haloC₁₋₄ alkoxy or C₃₋₆ cycloalkyloxy, and is optionally substituted with 1 to 3 groups selected from halogen, -NHC₁₋₄ alkyl, -N(C₁₋₄ alkyl)₂, C₁₋₄ alkoxy, haloC₁₋₄ alkoxy, haloC₁₋₄ alkyl, CN, C₁₋₄ alkyl or =O;
R₆ is H, halogen, C₁₋₄ alkyl or haloC₁₋₄ alkyl;
alternatively, R₅ and R₆ together with the atoms to which they are attached form 5-membered or 6-membered heterocycle containing 1 to 3 heteroatoms selected from N, S, and O, and the heterocycle is optionally substituted with 1 to 3 groups selected from halogen, OH, NH₂, -NHC₁₋₄ alkyl, -N(C₁₋₄ alkyl)₂, CN and C₁₋₄ alkyl;
R₇, R₈, and R₉ are independently H, halogen, -NHC₁₋₄ alkyl, -N(C₁₋₄ alkyl)₂, CN, C₁₋₄ alkyl, C₂₋₄ alkenyl, C₂₋₄ alkynyl, haloC₁₋₄ alkyl, C₁₋₄ alkoxy or haloC₁₋₄ alkoxy;
Y is C₁₋₂ alkylene, O or NR_{y};
R_{y} is H or C₁₋₄ alkyl;
M is C₁₋₂ alkylene, O or NRₘ;
W is a bond, O or NR_{w};
Rₘ and R_{w} are independently H or C₁₋₄ alkyl;
R is selected from C₁₋₄ alkyl, C₃₋₈ cycloalkyl, 4-membered to 10-membered heterocycle containing 1 to 3 heteroatoms selected from N, S, and O, or -O-C₃₋₆ cycloalkyl, and the alkyl, cycloalkyl or heterocycle is optionally substituted with 1 to 3 groups selected from halogen, C₁₋₄ alkyl, C₁₋₄ alkoxy, haloC₁₋₄ alkoxy, OH, NH₂, -NHC₁₋₄ alkyl, -N(C₁₋₄ alkyl)₂ and haloC₁₋₄ alkyl;
when Cy is P2, R is 4-membered to 10-membered saturated heterocycloalkyl containing 1 to 3 heteroatoms selected from N, S, and O, and is optionally substituted with 1 to 3 groups selected from halogen, C₁₋₄ alkyl, C₁₋₄ alkoxy, haloC₁₋₄ alkoxy, OH, NH₂, -NHC₁₋₄ alkyl, -N(C₁₋₄ alkyl)₂ and haloC₁₋₄ alkyl;
the pharmaceutical composition or the pharmaceutical preparation comprises 1-1000 mg of the active ingredient M and the excipient includes one or more of a filler, a binder, a glidant, a lubricant, and a disintegrant.

2. The pharmaceutical composition or the pharmaceutical preparation according to claim 1, **characterised in that** the pharmaceutical composition or the pharmaceutical preparation comprises 5-800 mg, 5-600 mg or 5-400 mg of the active ingredient M.

3. The pharmaceutical composition or the pharmaceutical preparation according to claim 2, **characterised in that** the pharmaceutical composition or the pharmaceutical preparation comprises 5 mg, 10 mg, 20 mg, 50 mg, 100 mg, 200 mg, 400 mg, 600 mg or 800 mg of the active ingredient M.

4. The pharmaceutical composition or the pharmaceutical preparation according to claim 1, **characterised in that** the pharmaceutical composition or the pharmaceutical preparation comprises an active ingredient M and a pharmaceutically acceptable excipient, and the active ingredient M is selected from a compound as shown in general formula I or a stereoisomer, tautomer, deuterated product, solvate, prodrug, metabolite, pharmaceutically acceptable salt or eutectic crystal thereof, having a structure of formula II: 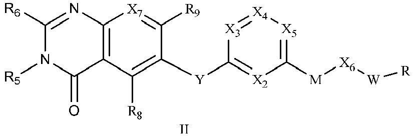 wherein
R₆ and R₉ are H; Y is O; M is NH;
X₂ is CR₃; X₃ is CR₃₁; X₄ is CR₃₂; X₅ is CH; X₆ is SO₂; X₇ is CH;
R₃ is C₁₋₂ alkyl, haloC₁₋₂ alkyl, C₁₋₂ alkoxy, haloC₁₋₂ alkoxy, C₂₋₄ alkenyl, C₂₋₄ alkynyl, -NHC₁₋₂ alkyl, -N(C₁₋₂ alkyl)₂ or CN;
each of R₃₁ and R₃₂ is independently H, F, Cl, C₁₋₂ alkyl, haloC₁₋₂ alkyl, C₂₋₄ alkenyl, C₂₋₄ alkynyl, C₁₋₂ alkoxy, haloC₁₋₂ alkoxy, -NHC₁₋₂ alkyl, -N(C₁₋₂ alkyl)₂, CN or C₃₋₆ cycloalkyl;
R₅ is C₁₋₂ alkyl, CN or =O substituted C₁₋₂ alkyl, haloC₁₋₂ alkyl, C₃₋₄ cycloalkyl, 4-membered to 7-membered heterocycloalkyl containing 1-3 heteroatoms selected from N, S, and O, C₁₋₂ alkoxy, haloC₁₋₂ alkoxy or C₃₋₄ cycloalkyloxy;
R₈ is selected from H, F, Cl, CN, C₁₋₂ alkyl, C₂₋₄ alkenyl, C₂₋₄ alkynyl, haloC₁₋₂ alkyl, C₁₋₂ alkoxy or haloC₁₋₂ alkoxy;
R is 4-membered or 5-membered monocyclic heterocycloalkyl containing 1 to 3 heteroatoms selected from N, S, and O, 6-membered to 8-membered monocyclic heterocycloalkyl, 5-membered or 6-membered monocyclic heteroaryl, 5-membered to 10-membered fused heterocycloalkyl, 5-membered to 10-membered bridged heterocycloalkyl, 6-membered to 10-membered spirocyclic heterocycloalkyl or -O-C₃₋₆ cycloalkyl, and the 4-membered monocyclic heterocycloalkyl is substituted with 1 to 3 groups selected from F, Cl, C₁₋₂ alkyl, C₁₋₂ alkoxy, OH, NH₂, -NHC₁₋₂ alkyl, -N(C₁₋₂ alkyl)₂ and haloC₁₋₂ alkyl, the 6-membered to 8-membered monocyclic heterocycloalkyl, the 5-membered or 6-membered monocyclic heteroaryl, the 5-membered to 10-membered fused heterocycloalkyl, the 5-membered to 10-membered bridged heterocycloalkyl, the 6-membered to 10-membered spirocyclic heterocycloalkyl or the C₃₋₆ cycloalkyl is optionally substituted with 1 to 3 groups selected from F, Cl, C₁₋₂ alkyl, C₁₋₂ alkoxy, OH, NH₂, -NHC₁₋₂ alkyl, -N(C₁₋₂ alkyl)₂ and haloC₁₋₂ alkyl.

5. The pharmaceutical composition or the pharmaceutical preparation according to claim 1, **characterised in that** the pharmaceutical composition or the pharmaceutical preparation comprises an active ingredient M and a pharmaceutically acceptable excipient, and the active ingredient M is selected from a compound as shown in general formula I or a stereoisomer, tautomer, deuterated product, solvate, prodrug, metabolite, pharmaceutically acceptable salt or eutectic crystal thereof, wherein
R₃ is CN;
each of R₃₁ and R₃₂ is independently H, F, Cl or C₃₋₆ cycloalkyl;
R₅ is selected from C₁₋₂ alkyl, haloC₁₋₂ alkyl, C₁₋₂ alkoxy, or CN or =O substituted C₁₋₂ alkyl;
R₈ is H;
R is 6-membered to 10-membered spirocyclic heterocycloalkyl containing 1 to 3 heteroatoms selected from N, S, and O, 4-membered or 5-membered monocyclic heterocycloalkyl containing 1 to 3 heteroatoms selected from N, S, and O, or -O-C₃₋₆ cycloalkyl, and the 6-membered to 10-membered spirocyclic heterocycloalkyl, the 4-membered or 5-membered monocyclic heterocycloalkyl or the C₃₋₆ cycloalkyl is optionally substituted with 1 to 3 groups selected from F, Cl, C₁₋₂ alkyl or C₁₋₂ alkoxy.

6. The pharmaceutical composition or the pharmaceutical preparation according to claim 1, **characterised in that** the active ingredient M is selected from one of the structures in Table 1 or Table 2.

7. A pharmaceutical composition or a pharmaceutical preparation, **characterised by** comprising the active ingredient M and the pharmaceutically acceptable excipient according to any one of claims 1-6, wherein the pharmaceutically acceptable excipient comprises a filler, a binder, a glidant, a lubricant, and a disintegrant, and preferably further comprises one or more of a solubilizer, a pH regulator, and a surfactant.

8. The pharmaceutical composition or the pharmaceutical preparation according to claim 7, **characterised in that** the active ingredient M is present with a content of 0.5%-99%, preferably 5%-60%.

9. The pharmaceutical composition or the pharmaceutical preparation according to claim 7, **characterised in that** the filler is one or more of microcrystalline cellulose, lactose, mannitol, pregelatineized starch, silicified microcrystalline cellulose, sucrose, sorbitol, dextran, calcium dihydrogen phosphate, or starch, preferably one or more of microcrystalline cellulose, silicified microcrystalline cellulose, or sorbitol, and preferably the content of the filler is 10% to 90%, preferably 10% to 80%, more preferably 10% to 50%, and/or
wherein the binder is one or more of povidone, hydroxypropyl cellulose, hypromellose, methylcellulose, sodium carboxymethyl cellulose, or sodium carboxymethyl cellulose, preferably one or more of hypromellose, methylcellulose, or sodium carboxymethyl cellulose, and preferably the content of the binder is 1% to 10%, preferably 1% to 5%, more preferably 1% to 2.5%, and/or
wherein the glidant is one or more of talc, silicon dioxide, micronized silica gel, polyethylene glycol, or magnesium lauryl sulphate, preferably one or more of silicon dioxide or micronized silica gel, and preferably the content of the glidant is 0.5% to 10%, preferably 0.5% to 5%, more preferably 0.5% to 3%, and/or
wherein the lubricant is one or more of magnesium stearate, calcium stearate, stearic acid, or sodium stearyl fumarate, preferably sodium stearyl fumarate, and preferably the content of the lubricant is 0.1% to 10%, preferably 0.1% to 5%, more preferably 0.5% to 2.0%, and/or
wherein the disintegrant is one or more of sodium carboxymethyl starch, low-substituted hydroxypropyl cellulose, crospovidone, croscarmellose sodium, or calcium carboxymethylcellulose, preferably one or more of crospovidone, croscarmellose sodium, or calcium carboxymethylcellulose, and preferably the content of the disintegrant is 0.5% to 10%, preferably 2% to 10%.

10. The pharmaceutical composition or the pharmaceutical preparation according to claim 7, **characterised in that** the solubilizer is one or more of polyoxyethylene castor oil, polyoxyethylene hydrogenated castor oil, polysorbate, polyethylene glycol 15-hydroxystearate, poloxamer, cyclodextrin, hydroxypropyl betacyclodextrin, docusate sodium, or vitamin E polyethylene glycol succinate, preferably one or more of cyclodextrin or hydroxypropyl betacyclodextrin, and preferably the content of the solubilizer is 5% to 50%, preferably 10% to 50%.

11. The pharmaceutical composition or the pharmaceutical preparation according to claim 7, **characterised in that** the pharmaceutical composition or the pharmaceutical preparation comprises:
(i) the active ingredient M with a content of 0.5%-99%, preferably 5%-60%;
(ii) a filler, which is one or more of microcrystalline cellulose, silicified microcrystalline cellulose or sorbitol with a content of 10%-90%, preferably 10%-70%, more preferably 10%-50%;
(iii) a binder, which is one or more of hypromellose, methyl cellulose or sodium carboxymethyl cellulose with a content of 1%-10%, preferably 1%-5%, and more preferably 1%-2.5%;
(iv) a glidant, which is one or more of silicon dioxide or micronized silica gel with a content of 0.5%-10%, preferably 0.5%-5%, more preferably 0.5%-3%;
(v) a lubricant, which is sodium stearyl fumarate with a content of 0.1%-10%, preferably 0.1%-5%, more preferably 0.5%-2.0%;
(vi) a disintegrant, which is one or more of crospovidone, croscarmellose sodium, or calcium carboxymethylcellulose with a content of 0.5% to 10%, preferably 2% to 10%.

12. The pharmaceutical composition or the pharmaceutical preparation according to claim 11, **characterised in that** the pharmaceutical composition or the pharmaceutical preparation further comprises one or more of a solubilizer, a surfactant, or a pH regulator;the solubilizer is one or more of cyclodextrin or hydroxypropyl betacyclodextrin with a content of 5%-50%, preferably 10%-50%;the surfactant is sodium dodecyl sulphate with a content of 1%-40%, preferably 10%-20%; the pH regulator is magnesium oxide with a content of 1-50%, preferably 1-5%.

13. Use of the pharmaceutical composition or the pharmaceutical preparation according to any one of claims 1-12 in preparation of a related drug for treating cancers.

14. A method for the treatment of a disease in a mammal, **characterised by** comprising administering to a subject a therapeutically effective amount of the active ingredient M, preferably 1-1000 mg, wherein preferably, the disease is cancer.
